# EUROPÄISCHE PATENTANMELDUNG

(11) **EP 2 161 259 A1**
(43) Veröffentlichungstag der Anmeldung: **10.03.2010**
(21) Anmeldenummer: 08163552.6
(22) Anmeldetag: 03.09.2008
(51) Int. Cl.: C07D 239/42, C07D 239/48, C07D 413/12, A01N 43/54

(54) **4-Halogenalkylsubstituierte Diaminopyrimidine als Fungizide**

(71) Anmelder: Bayer CropScience AG, 40789 Monheim (DE)
(72) Erfinder: Die Erfindernennung liegt noch nicht vor

(57) **Zusammenfassung**

4-Haloalkylsubstituierte-Diaminopyrimidine der Formel (I) 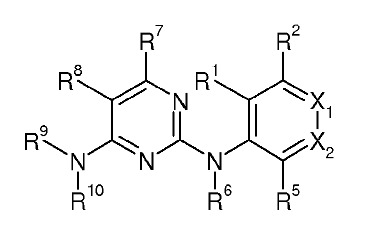 in welcher R¹ bis R¹⁴, sowie X¹ und X² die in der Beschreibung angegebenen Bedeutungen haben, sowie agrochemisch wirksame Salze davon, deren Verwendung sowie Verfahren und Mittel zur Bekämpfung von pflanzcnpathogcncn Schadpilzen in und/oder auf Pflanzen oder in und/oder auf Saatgut von Pflanzen, Verfahren zur Herstellung solcher Mittel und behandeltes Saatgut sowie deren Verwendung zur Bekämpfung von pflanzenpathogenen Schadpilzen in der Land-, Garten- und Forstwirtschaft, im Materialschutz sowie im Bereich Haushalt und Hygiene. Die vorliegende Erfindung betrifft weiterhin ein Verfahren zur Herstellung von 4-Haloalkylsubstituierten-Diaminopyrimidinen.

## Beschreibung

Die Erfindung betrifft 4-Halogenalkyl-substituierte Diaminopyrimidine sowie deren agrochemisch wirksame Salze, deren Verwendung sowie Verfahren und Mittel zur Bekämpfung von pflanzenpathogenen Schadpilzen in und/oder auf Pflanzen oder in und/oder auf Saatgut von Pflanzen, Verfahren zur Herstellung solcher Mittel und behandeltes Saatgut sowie deren Verwendung zur Bekämpfung von pflanzenpathogenen Schadpilzen in der Land-, Garten- und Forstwirtschaft, im Materialschutz sowie im Bereich Haushalt und Hygiene. Die vorliegende Erfindung betrifft weiterhin ein Verfahren zur Herstellung von 4-Halogenalkyl-substituierten Diaminopyrimidinen.

Es ist bereits bekannt, dass bestimmte alkinyl-substituierte Diaminopyrimidine als fungizide Pflanzenschutzmittel benutzt werden können (siehe DE 4029650 A1). Die fungizide Wirksamkeit dieser Verbindungen ist jedoch gerade bei niedrigeren Aufwandmengen nicht immer ausreichend.

Da sich die ökologischen und ökonomischen Anforderungen an moderne Pflanzenschutzmittel laufend erhöhen, beispielsweise was Wirkspektrum, Toxizität, Selektivität, Aufwandmenge, Rückstandsbildung und günstige Herstellbarkeit angeht, und außerdem z.B. Probleme mit Resistenzen auftreten können, besteht die ständige Aufgabe, neue Pflanzenschutzmittel, insbesondere Fungizide zu entwickeln, die zumindest in Teilbereichen Vorteile gegenüber den bekannten aufweisen.

Überraschenderweise wurde nun gefunden, dass die vorliegenden 4-Haloalkyl-substituierten Diaminopyrimidine die genannten Aufgaben zumindest in Teilaspekten lösen und sich als Pflanzenschutzmittel, insbesondere als Fungizide eignen.

Einige 4-Halogenalkyl-substituierte Diaminopyrimidine sind bereits als pharmazeutisch wirksame Verbindungen beschrieben (siehe z.B. WO 06/087230, WO 02/096888 oder WO 02/004429), jedoch nicht deren überraschende fungizide Wirksamkeit.

Gegenstand der Erfindung sind Verbindungen der Formel (**I**), 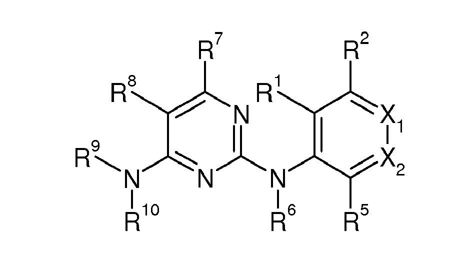 in welcher die Symbole folgende Bedeutungen haben:
- X¹: ist Stickstoff oder CR³,
- X²: ist Stickstoff oder CR⁴,
wobei X¹ und X² nicht gleichzeitig für Stickstoff stehen,
- R¹ und R⁵: sind unabhängig voneinander Wasserstoff, C₁-C₄-Alkyl, C₁-C₄-Alkoxy, OH, C₁-C₄-Halogenalkyl oder Halogen,
- R²: ist Wasserstoff, Halogen, Nitro, Cyano, C₁-C₄-Alkyl, C₁-C₂-Haloalkyl, Hydroxy, OR¹², OCON(R¹²)₂, C₁-C₃-Haloalkoxy, COR¹², CR¹²=NOR¹², SF₅, SR¹², SOR¹², SO₂R¹², CO₂R¹², N(R¹²)_{2,} NR¹²COR¹² oder NR¹²CO₂R¹³,
- R³ und R⁴: sind unabhängig voneinander Wasserstoff, Halogen, Cyano, Nitro, ein 3- bis 8-gliedriger, unsubstituierter oder substituierter, gesättigter oder ungesättigter Zyklus, der keine oder bis zu vier Heteroatome, ausgewählt aus N, O und S enthalten kann, wobei zwei Sauerstoffatome nicht benachbart sind, OR¹², OSO₂N(R¹²)₂, OCON(R¹²)₂, OCOR¹³, SF₅, SR¹², SOR¹², SO₂R¹², SON(R¹²)₂, SO₂N(R¹³)₂, SO₂NHR¹⁴, SO₂NHCOR¹², C=OR¹², CH=NOR¹², CR¹³=NOR¹², COCl, CON(R¹²)₂, COOR¹², COO(CH₂)ₘOR¹², CO(CH₂)ₘCN, CO(CH₂)ₘN(R¹²)₂, NR¹²COR¹², NR¹²COOR¹³, NR¹²(C=S)R¹³, NR¹²(C=S)OR¹³, N(R¹²)₂, NR¹²(CH₂)ₘN(R¹²)₂, NR¹²SO₂R¹³, NR¹²SOR¹³, [C(R¹²)₂]ₘCN, (CH₂)ₘSON(R¹²)₂, (CH₂)ₘSO₂N(R¹²)₂, (CH₂)ₘCOOR¹², (CH₂)ₘCOR¹², [C(R¹²)₂]ₘCOR¹², [C(R¹²)₂]ₘCON(R¹²)₂, (CH₂)ₘN(R¹²)₂, (CH₂)ₘNR¹²COR¹², (CH₂)ₘNR¹²COOR¹³, [C(R¹²)₂]ₘNR¹²COR¹², [C(R¹²)₂]ₘNR¹²COOR¹³, unsubstituiertes oder substituiertes C₁-C₈-Alkyl, unsubstituiertes oder substituiertes C₂-C₆-Alkenyl, unsubstituiertes oder substituiertes C₁-C₈-Haloalkyl, unsubstituiertes oder substituiertes C₂-C₆-Alkinyl; mit m = 1 - 4,
wobei zusätzlich oder unabhängig davon R³ und R⁴, ggf. über R¹² oder R¹³, gemeinsam einen 3- bis 7-gliedrigen, unsubstituierten oder substituierten, gesättigten oder ungesättigten Zyklus bilden können, der kein oder bis zu vier Heteroatome, ausgewählt aus N, O und S enthalten kann, wobei zwei Sauerstoffatome nicht benachbart sind,
wobei die Substituenten unabhängig voneinander ausgewählt sind aus Wasserstoff, Fluor, Chlor, oder Brom, C₁-C₄-Alkyl, C₁-C₄-Alkoxy, Hydroxy, Oxo, C₁-C₄-Halogenalkyl, (C₁-C₄-Alkyl)carbonyl oder Cyano,
- R⁶: ist Wasserstoff, C₁-C₄-Alkyl, C₁-C₄-Alkoxy(C₁-C₄)alkyl, Formyl, (C₁-C₄-Alkyl)carbonyl, (C₁-C₄-Alkoxy-C₁-C₄-alkyl)carbonyl, (C₃-C₆-Alkenyl-oxy)carbonyl, (C₃-C₆-Cycloalkyl)- carbonyl, (Halogen-C₁-C₄-alkoxy-C₁-C₄-alkyl)carbonyl, (C₁-C₄-Haloalkyl)carbonyl, (C₁-C₄-Alkoxy)carbonyl, (C₁-C₄-Haloalkoxy)carbonyl, Benzyloxycarbonyl, unsubstituiertes oder substituiertes Benzyl, unsubstituiertes oder substituiertes Benzoyl, unsubstituiertes oder substituiertes C₂-C₆-Alkenyl, unsubstituiertes oder substituiertes C₂-C₆-Alkinyl, C₁-C₂-Alkylsulfinyl oder C₁-C₂-Alkylsulfonyl,
wobei die Substituenten unabhängig voneinander ausgewählt sind aus Wasserstoff, Fluor, Chlor oder Brom, C₁-C₄-Alkyl, C₁-C₄-Alkoxy, Hydroxy, C₁-C₄-Halogenalkyl, oder Cyano,
- R⁷: ist Wasserstoff, C₁-C₃-Alkyl, Cyano oder C₁-C₃-Haloalkyl,
- R⁸: ist Fluor, Chlor, Brom, Iod, Cyano, Methyl, SMe, SOMe, SO₂Me, CF₃, CCl₃, CFH₂ oder CF₂H,
- R⁹: ist Wasserstoff, C₁-C₃-Alkyl, C₂-C₃-Haloalkyl, C₁-C₄-Alkoxy(C₁-C₄)alkyl, (C₁-C₄-Alkyl)carbonyl, (C₁-C₄-Haloalkyl)carbonyl, (C₁-C₄-Alkoxy)carbonyl, unsubstituiertes oder substituiertes Benzyl, unsubstituiertes oder substituiertes C₂-C₄-Alkenyl, unsubstituiertes oder substituiertes C₂-C₄-Alkinyl, C₁-C₆-Alkylsulfinyl, C₁-C₆-Alkylsulfonyl, C₁-C₆-Haloalkylsulfinyl oder C₁-C₆-Haloalkylsulfonyl,
wobei die Substituenten unabhängig voneinander ausgewählt sind aus Fluor-, Chlor- und/oder Bromatomen, Cyano, Hydroxy, Methyl, Methoxy, CF₃
- R¹⁰: ist eine Gruppe der Formel E1 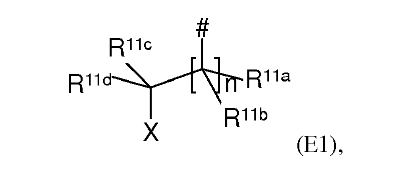
in welcher die Symbole die folgenden Bedeutungen haben und # für die Anknüpfungsstelle an das Stickstoffatom steht:
- R^{11a}: ist Wasserstoff oder C₁-C₄-Alkyl,
- R^{11b}: ist Wasserstoff, C₁-C₄-Alkyl, C₁-C₃-Halogenalkyl, (CH₂)ₘOH, (CH₂)ₘO(C₁-C₂-Alkyl), COOR¹², CON(R¹²)₂, (CH₂)ₘCOOR¹², COR¹², unsubstituiertes oder substituiertes Benzyl, unsubstituiertes oder substituiertes C₂-C₆-Alkenyl oder unsubstituiertes oder substituiertes C₂-C₆-Alkinyl,
wobei die Substituenten unabhängig voneinander ausgewählt sind aus Wasserstoff, Fluor, Chlor oder Brom, C₁-C₄-Alkyl, C₁-C₄-Alkoxy, Hydroxy, C₁-C₄-Halogenalkyl oder Cyano,
- R^{11c}: ist Wasserstoff, Halogen oder C₁-C₄-Alkyl,
- R^{11d}: ist Wasserstoff, Halogen, C₁-C₆-Alkyl, OR¹², COOR¹², (CH₂)ₘCOOR¹² oder COR¹²,
- X: ist Halogen oder C₁-C₆-Halogenalkyl,
mit m = 1 - 4 und n = 1 - 4
- R¹²: ist unabhängig voneinander gleich oder verschieden Wasserstoff, C₁-C₆-Alkyl, C₁-C₆-Haloalkyl, unsubstituiertes oder substituiertes C₃-C₆-Cycloalkyl, C₁-C₄-Trialkyl-Silyl, unsubstituiertes oder substituiertes C₂-C₄-Alkenyl, unsubstituiertes oder substituiertes C₂-C₄-Alkinyl, unsubstituiertes oder substituiertes Aryl, C₁-C₄-Alkoxy(C₁-C₄)alkyl, unsubstituiertes oder substituiertes Benzyl oder ein 3- bis 7-gliedriger, unsubstituierter oder substituierter, gesättigter oder ungesättigter Zyklus, der keine oder bis zu vier Heteroatome, ausgewählt aus N, O und S enthalten kann, wobei zwei Sauerstoffatome nicht benachbart sind
oder
für den Fall, dass zwei Reste R¹² an ein Stickstoffatom gebunden sind, können zwei Reste R¹² einen 3 bis 7-gliedrigen, unsubstituierten oder substituierten, gesättigten oder ungesättigten Zyklus bilden, der bis zu vier weitere Heteroatome, ausgewählt aus N, O und S enthalten kann, wobei zwei Sauerstoffatome nicht benachbart sind, oder
für den Fall, dass zwei Reste R¹² benachbart in der Gruppierung NR¹²COR¹² NR¹²SOR¹² NR¹²SO₂R¹² NR¹²SONR¹² NR¹²SO₂NR¹² vorliegen, können zwei Reste R¹² einen 3 bis 7-gliedrigen, unsubstituierten oder substituierten, gesättigten oder ungesättigten Zyklus, der bis zu vier weitere Heteroatome, ausgewählt aus N, O und S enthalten kann, wobei zwei Sauerstoffatome nicht benachbart sind, bilden,
- R¹³: ist unabhängig voneinander gleich oder verschieden C₁-C₈-Alkyl, C₁-C₈-Haloalkyl, C₁-C₄-Trialkyl-Silyl, unsubstituiertes oder substituiertes C₂-C₆-Alkenyl, unsubstituiertes oder substituiertes C₂-C₆-Alkinyl, unsubstituiertes oder substituiertes C₃-C₆-Cycloalkyl, unsubstituiertes oder substituiertes Aryl, C₁-C₄-Alkoxy(C₁-C₄)alkyl, unsubstituiertes oder substituiertes Benzyl oder ein 3- bis 7-gliedriger, unsubstituierter oder substituierter, gesättigter oder ungesättigter Zyklus, der keine oder bis zu vier Heteroatome, ausgewählt aus N, O und S enthalten kann, wobei zwei Sauerstoffatome nicht benachbart sind,
oder
für den Fall, dass zwei Reste R¹³ an ein Stickstoffatom gebunden sind, können zwei Reste R¹³ einen einen 3 bis 7-gliedrigen, unsubstituierten oder substituierten, gesättigten oder ungesättigten Zyklus, der bis zu vier weitere Heteroatome, ausgewählt aus N, O und S enthalten kann, wobei zwei Sauerstoffatome nicht benachbart sind, bilden können,
- R¹⁴: ist gleich oder verschieden C₁-C₆-Alkyl, C₁-C₈-Haloalkyl, C₁-C₄-Trialkyl-Silyl, unsubstituiertes oder substituiertes C₂-C₆-Alkenyl, unsubstituiertes oder substituiertes C₂-C₆-Alkinyl, unsubstituiertes oder substituiertes C₃-C₆-Cycloalkyl, unsubstituiertes oder substituiertes Aryl, unsubstituiertes oder substituiertes Benzyl oder ein 3- bis 7-gliedriger, unsubstituierter oder substituierter, gesättigter oder ungesättigter Zyklus, der keine oder bis zu vier Heteroatome, ausgewählt aus N, O und S enthalten kann, wobei zwei Sauerstoffatome oder zwei Stickstoffatome nicht benachbart sind,
und wobei mögliche Substituenten ausgewählt sind aus folgender Liste:
Fluor Chlor, Brom, Iod, Cyano, Nitro, CF₃, CFH₂, CF₂H, C₂F₅, CCl₃, Hydroxy, OMe, OEt, OPr, O*iso*Pr, OBu, O*sec*Bu, O*iso*Bu, O*tert*Bu, O(CH₂)₂OCH₃, O(CH₂)₃OCH₃, O-cylopentyl, O-Phenyl, OCF₃, OCF₂H, OCF₂CF₃, OCF₂CF₂H, SH, SMe, SEt, SCF₃, SCF₂H, SPh, SCF₅, SO₂Me, SO₂CF₃, SOMe, SOEt, CO₂H, CO₂CH₃, CO₂Et, CO₂Pr, CO₂*iso*Pr, CO₂*tert*Bu, COMe, COCF₃, NH₂, NHMe, NMe₂, NHEt, NEt₂, NHPr, NH*iso*Pr, NH*n*Bu, NH*tert*Bu, NH*iso*Bu, NH*sec*Bu, cyclopropylamino, Formyl, CH₂CN, CHMeCN, CH₂COCH₃, CH₂OMe, (CH₂)₂OMe, (CH₂)₃OMe, CH₂OH, CH₂SMe, (CH₂)₂SMe, Methyl, Ethyl, Propyl, 1-Methylethyl, Butyl, 1-Methyl-propyl, 2-Methylpropyl, 1,1-Dimethylethyl, Cyclopropyl, 1-methoxycyclopropyl, 1-chlorcyclopropyl, Cyclobutyl, 3-dimethylbutyl, Cyclopentyl, Cyclohexyl, Cyclohexylmethyl, Neopentyl, prop-2-en-1-yl, 1-methylprop-2-en-1-yl, but-3-en-1-yl, trimethylsilyl)methyl, Cyclopropyl, cyclobutyl, cyclopentyl, cyclohexyl, Phenyl, Benzyl,-CH₂CH=CH₂, -CH(CH₃)CH=CH₂, -CH₂C≡CH,
sowie die agrochemisch wirksamen Salze davon.

Ein weiterer Gegenstand ist die Verwendung der Verbindungen der Formel (I) als Fungizide.

Die vorgenannten erfindungsgemäßen Verbindungen zeigen vor allem eine starke fungizide Wirksamkeit und lassen sich sowohl im Pflanzenschutz, im Bereich Haushalt und Hygiene als auch im Materialschutz verwenden.

Die Verbindungen der Formel (**I**) können sowohl in reiner Form als auch als Mischungen verschiedener möglicher isomerer Formen, insbesondere von Stereoisomeren, wie E- und Z-, threo- und erythro-, sowie optischen Isomeren, wie R- und S-Isomeren oder Atropisomeren, gegebenenfalls aber auch von Tautomeren vorliegen. Es werden sowohl die E- als auch die Z-Isomeren, wie auch die threo- und erythro-, sowie die optischen Isomeren, beliebige Mischungen dieser Isomeren, sowie die möglichen tautomeren Formen beansprucht.

Bevorzugt werden Verbindungen der Formel (**I**), in denen ein oder mehrere der Symbole eine der folgenden Bedeutungen haben:
- X¹: ist Stickstoff oder CR³,
- X²: ist Stickstoff oder CR⁴,
wobei X¹ und X² nicht gleichzeitig für Stickstoff stehen
- R¹ und R⁵: sind unabhängig voneinander H, C₁-C₂-Alkyl, C₁-C₂-Alkoxy, OH, CF₃, F, Cl oder Br
- R²: ist Wasserstoff, Halogen, Nitro, Cyano, C₁-C₄-Alkyl, C₁-C₂-Haloalkyl, Hydroxy, C₁-C₄-Alkoxy, O(C₂-C₄-Alkenyl), O(C₂-C₄-Alkinyl), OCONH(C₁-C₄-Alkyl), OCON(C₁-C₄-Alkyl)₂, C₁-C₃-Haloalkoxy, COH, CO(C₁-C₄-Alkyl), C(=NOH)Me, C(=NO(C₁-C₄-Alkyl)Me, CH(=NO(C₁-C₄-Alkyl), SF₅, S(C₁-C₃)-Alkyl, S(C₁-C₂)-Haloalkyl, SO(C₁-C₄-Alkyl), SO₂(C₁-C₄-Alkyl), CO₂(C₁-C₃-Alkyl), NH₂, NH(C₁-C₄-Alkyl), N(C₁-C₄-Alkyl)₂, NHCO(C₁-C₄-Alkyl), NHCOH, NMeCO(C₁-C₄-Alkyl), NHCO₂(C₁-C₄-Alkyl),
- R³ und R⁴: sind unabhängig voneinander Wasserstoff, Halogen, Cyano, Nitro, Hydroxy, O-C₁-C₄-Alkyl, O-(C₁-C₃-Haloalkyl), O-(C₃-C₆-Cycloalkyl), O-C₂-C₄-Alkenyl, O-C₂-C₄-Alkinyl, OPh, OSO₂N(C₁-C₄-Alkyl)₂, OCONH(C₁-C₄-Alkyl), OCON(C₁-C₄-Alkyl)₂, OCO(C₁-C₄-Alkyl), SF₅, SH, S-C₁-C₄-Alkyl, S-C₁-C₃-Haloalkyl, SPh, SO(C₁-C₄-Alkyl), SO₂(C₁-C₄-Alkyl), SO₂(C₁-C₃-Haloalkyl), SO₂(C₂-C₄-Alkenyl), SO₂(C₂-C₄-Alkinyl), SONH(C₁-C₄-Alkyl), SON(C₁-C₄-Alkyl)₂, SO₂N(C₁-C₄-Alkyl)₂, SO₂ NHCO(C₁-C₄-Alkyl), SO₂NHPh, SO₂NH(C₂-C₄-Alkenyl), Formyl, (C₁-C₄-Alkyl)carbonyl, (C₁-C₃-Haloalkyl)carbonyl, CH=NO(C₁-C₄-Alkyl), C(C₁-C₄-Alkyl)=NO(C₁-C₄-Alkyl), CO(CH₂)ₘCN, CONH(C₁-C₄-Alkyl), CON(C₁-C₄-Alkyl)₂, CONH(C₁-C₃-Haloalkyl), CONH(C₃-C₄-Alkenyl), CONH(C₃-C₄-Alkinyl), CONH(C₃-C₆-Cycloalkyl), CONHCH(CH₃)CH₂O(C₁-C₄-Alkyl), CONH(CH₂)ₘO(C₁-C₄-Alkyl), CONHPh, COCH₂N(C₁-C₄-Alkyl)₂, Piperidin-1-ylcarbonyl, Morpholin-4-ylcarbonyl, (4-Methylpiperazin-1-yl)carbonyl, Pyrrolidin-1-ylcarbonyl, Azetidin-1-yl-carbonyl, Aziridin-1-yl-carbonyl, Hexamethylenimin-1-yl-carbonyl, Morpholin-1-ylcarbonyl, Thiomorpholin-1-ylcarbonyl, COOH, (C₁-C₄-Alkoxy)carbonyl, CO₂(CH₂)ₘO(C₁-C₄-Alkyl), NHCO(C₁-C₄-Alkyl), NHCO(C₁-C₄-Haloalkyl), N(C₁-C₂-Alkyl)CO(C₁-C₄-Alkyl), NHCO(C₂-C₄-Alkenyl), NHCOPh, NHCOC((C₁-C₄-Alkyl)₂CH₂Hal, NHCO(C=CH₂)CH₃, NHCO(CH₂)ₘO(C₁-C₄-Alkyl), NHCHO, N(C₁-C₄-Alkyl)CHO, NHCO₂(C₁-C₄-Alkyl), NHCO₂Ph, NHCO₂CH₂CH₂Hal, N(C₁-C₄-Alkyl)CO₂(C₁-C₄-Alkyl), NH(C=S)O(C₁-C₄-Alkyl), NH₂, NH(C₁-C₄-Alkyl), N(C₁-C₄-Alkyl)₂, cyclopropylamino, NHCH(C₁-C₄-Alkyl)CH₂O(C₁-C₄-Alkyl), acetyl(cyclopropyl)amino, [(1-methylcyclopropyl)carbonyl]-amino, morpholin-1-yl, morpholin-4-yl-methyl, NHSO₂(C₁-C₄-Alkyl), NHSO₂(C₁-C₃-Haloalkyl), CH₂CN, CH(C₁-C₄-Alkyl)CN, (CH₂)ₘSO₂NH(C₁-C₄-Alkyl), (CH₂)ₘSO₂N(C₁-C₄-Alkyl)₂, (CH₂)ₘCO(C₁-C₄-Alkyl), CH(C₁-C₄-Alkyl)CO(C₁-C₄-Alkyl), (CH₂)ₘCOcyclopropyl, (CH₂)ₘCO₂(C₁-C₄-Alkyl), (CH₂)ₘCONH(C₁-C₄-Alkyl), (CH₂)ₘCON(C₁-C₃-Alkyl)₂, (CH₂)ₘNHCOO(C₁-C₄-Alkyl), CH₂NHCOOBn, (CH₂)ₘNHCO(C₁-C₄-Alkyl), (CH₂)ₘNHCO(C₁-C₃-Haloalkyl), C₁-C₅-Alkyl, C₃-C₆-Cycloalkyl, 1-methoxycyclopropyl, 1-chlorcyclopropyl, Cyclopenten(1)yl, 2-Oxocyclopentyl C₂-C₆-Alkenyl, C₁-C₃-Haloalkyl, morpholin-4-ylsulfonyl, [(4,6-dimethylpyrimidin-2-yl)amino]sulfonyl, 1H-tetrazol-5-yl, (cyclo-propyl-carbonyl)amino, (2-furoyl-amino), (3-methyl-2,5-dioxoimidazolidin-1-yl), (piperidin-1-ylethyl)amino, cyclopropyl(trifluor-acetyl)amino, (1-methylcyclo-propyl)carbonylamino, 4,4-dimethyl-2,5-dioxoimidazolidin-1-yl, 2,3-dimethyl-5-oxo-2,5-dihydro-1H-pyrazol-1-yl, 5-thioxo-4,5-dihydro-1H-tetrazol-1-yl, 3-methyl-2-oxoimidazol-idin-1-yl, 3-(1-methylethyl)-2-oxoimidazolidin-1-yl, 3-(2-methylpropyl)-2-oxoimidazol-idin-1-yl, 2-oxo-3-prop-2-en-1-ylimidazolidin-1-yl, 3-tert-butyl-2-oxoimidazolidin-1-yl, pyrrolidin-1-ylsulfonyl, 2,5-dioxoimidazolidin-4-yl, piperidin-1-ylsulfonyl, 1,3-thiazol-2-yl, 1,3-thiazol-4-yl, (morpholin-4-ylsulfonyl)methyl, (Dimethylsulfamoyl)methyl, (piperidin-1-ylsulfonyl)methyl, [(4-methyl-phenyl)amino]sulfonyl, (pyrrolidin-1-ylsulfonyl)methyl, 2-oxoimidazolidin-1-yl, 3-methyl-5-oxo-4,5-dihydro-1H-pyrazol-1-yl, (3,4-dimethyl-5-oxo-4,5-dihydro-1H-pyrazol-1-yl, (1-methylcyclopentyl), pyrrolidin-1-yl, piperidin-1-yl, 2-oxo-2,5-dihydro-1H-pyrrol-1-yl, 3,3-dimethyl-2-oxocyclopentyl, 3-Oxo-4,5-dimethyl-2,4-dihydro-pyrazol-2-yl-, 3-Oxo-4-ethyl-5-methyl-2,4-dihydro-pyrazol-2-yl-, 3-Oxo-5-trifluormethyl-2,4-dihydro-pyrazol-2-yl-, 3-Oxo-5-isopropyl-2,4-dihydro-pyrazol-2-yl-, 3,5-Dioxo-4,4-dimethyl-pyrazolidin-1-yl, 3,5-Dioxo-4-ethyl-pyrazolidin-1-yl, 3-Oxo-4,4-dimethyl-pyrazolidin-1-yl, 2,5-dioxo-2,5-dihydro-1H-pyrrol-1-yl, 3,5-Dimethylpiperidin-1-yl, 4-(tert-butoxy-carbonyl)piperazin-1-yl, 5-Ethoxy-3,4-dimethyl-1H-pyrazol-1-yl, Acetyl(cyclo-hexyl)amino, 2-Furoylamino, 2,2,2-trifluor-ethyl)carbamoyl, 5-Ethoxy-3-(trifluormethyl)-1H-pyrazol-1-yl 3-(2-Chlorethyl)-2-oxoimidazolidin-1-yl, 3-oxobutyl, 1,1-dioxidoisothiazolidin-2-yl, 1,1-dioxido-tetrahydrothiophen-2-yl, 5-methyl-1,1-dioxido- 1,2,5-thiadiazolidin-2-yl, 4-methoxy-2-oxo-2,5-dihydro-1H-pyrrol-1-yl, 2-oxo-2,5-dihydro-1H-pyrrol-1-yl, 5-oxo-4,5-dihydro-1H-imidazol-1-yl, 4-methyl-5-oxo-4,5-dihydro-1H-1,2,4-triazol-1-yl, 3-methyl-5-oxo-2,5-dihydro-1H-pyrazol-1-yl, 2-(methoxymethyl)pyrrolidin-1-yl, 2-oxocyclopentyl, 2-oxotetrahydrofuran-3-yl, 1-methyl-3-oxo-2,3-dihydro-1H-pyrazol-4-yl, 1-methyl-3-oxopyrazolidin-4-yl, tetrahydro-furan-2-yl, furan-2-yl, 1,1-dioxido-1,2-thiazinan-2-yl, 5-methyl-1,1-dioxido-1,2,5-thiadiazoli-din-2-yl, 6-methyl-1,1-dioxido-1,2,6-thiadiazinan-2-yl,
mit m= 1 - 3
und für den Fall, dass R³ und R⁴, ggf. über R¹² oder R¹³ einen Zyklus bilden, kann folgende Untereinheit aus der allgemeinen Formel (**I**): 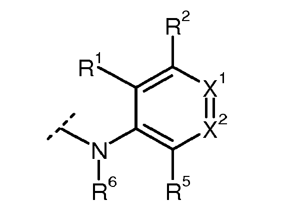 (2-oxo-2,3-dihydro-1H-indol-5-yl)amino, 1H-indol-6-ylamino, 1H-indol-5-ylamino, 2-(trifluormethyl)-1H-benzimidazol-6-yl]amino, (3-methyl-1,1-dioxido-2H-1,2,4-benzo-thiadiazin-7-yl)amino, (1,1-dioxido-2H-1,2,4-benzothiadiazin-6-yl)amino, (4-methyl-3-oxo-3,4-dihydro-2H-1,4-benzoxazin-6-yl)amino, (4-methyl-3-oxo-3,4-dihydro-2H-1,4-benzoxazin-7-yl)amino, (1-acetyl-2,3-dihydro-1H-indol-6-yl)amino, (4H-1,3-benzodioxin-7-ylamino, (2-oxo-2,3,4,5-tetra-hydro-1H-1-benzazepin-8-yl)amino, (2,2-dioxido-1,3-dihydro-2-benzothien-5-yl)amino, (1-oxo-2,3-dihydro-1H-inden-5-yl)amino, [2-(ethyl-sulfonyl)-2,3-dihydro-1,3-benzothiazol-6-yl]ami-no, (2,2,3,3-tetrafluor-2,3-dihydro-1,4-benzodioxin-6-yl)amino, 1,3-benzodioxol-5-ylamino, (1,3-dioxo-2,3-dihydro-1H-isoindol-5-yl)amino, 2-methyl-1,3-benzothiazol-6-yl)amino, (2-oxo-2,3-dihydro-1H-benzimidazol-5-yl)amino, (2-oxo-1,3-benzoxathiol-5-yl)amino, 2-oxo-2,3-dihydro-1,3-benzoxazol-5-yl)amino, (2-ethyl-1,3-benzoxazol-5-yl)amino, 2-oxo-1,2,3,4-tetrahy-droquinolin-6-yl)amino, (3-oxo-3,4-dihydro-2H-1,4-benzoxazin-6-yl)amino, (2-oxo-2,3-dihydro-1,3-benzoxazol-6-yl)amino, 3-oxo-1,3-dihydro-2-benzofuran-5-yl)amino, [2-(ethylsulfonyl)-1,3-benzothiazol-6-yl]amino, (2-methyl-1,3-benzothiazol-5-yl)amino, (1-acetyl-2,3-dihydro-1H-indol-5-yl)amino, (2,2,3,3-tetrafluoro-2,3-dihydro-1,4-benzodioxin-6-yl)amino, (2,2-dioxido-1,3-dihydro-2-benzothiophen-5-yl)amino, (2-oxo-2,3-dihydro-1H-indol-6-yl)amino, (2-oxo-1,2,3,4-tetrahydroquinolin-7-yl)amino, 1H-indazol-6-ylamino sein,
- R⁶: ist Wasserstoff, C₁-C₃-Alkyl, C₁-C₂-Alkoxy(C₁-C₂)alkyl, Formyl, (C₁-C₄-Alkyl)carbonyl, (C₁-C₄-Haloalkyl)carbonyl, (C₁-C₄-Alkoxy)carbonyl, Benzyloxycarbonyl, Benzyl, 4-Methoxybenzyl, 2,4-Dimethoxybenzyl, 2-Hydroxybenzyl, unsubstituiertes oder substituiertes Benzoyl, unsubstituiertes oder substituiertes C₂-C₄-Alkenyl, unsubstituiertes oder substituiertes C₂-C₄-Alkinyl, C₁-C₂-Alkylsulfinyl oder C₁-C₂-Alkylsulfonyl,
wobei die Substituenten unabhängig voneinander ausgewählt sind aus Wasserstoff, Fluor, Chlor oder Brom, C₁-C₂-Alkyl, C₁-C₂-Halogenalkyl oder Cyano,
- R⁷: ist Wasserstoff, Methyl, Cyano, CF₃, CFH₂ oder CF₂H,
- R⁸: ist Fluor, Chlor, Brom, Iod, Cyano, Methyl, SMe, SOMe, SO₂Me, CF₃, CFH₂ oder CF₂H,
- R⁹: ist Wasserstoff, C₁-C₃-Alkyl, C₂-C₃-Haloalkyl, C₁-C₂-Alkoxy(C₁-C₂)alkyl, COMe, COCF₃, COOMe, COOEt, COO*tert*Bu, Benzyl, 4-Methoxybenzyl, CH₂CH=CH₂ oder CH₂C≡CH,
- R¹⁰: ist eine Gruppe der Formel E1 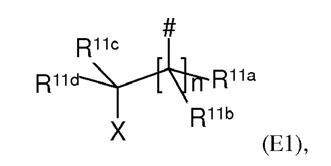
in welcher die Symbole die folgenden Bedeutungen haben und # für die Anknüpfungsstelle an das Stickstoffatom steht:
- R^{11a}: ist Wasserstoff oder Methyl,
- R^{11b}: ist Wasserstoff, C₁-C₄-Alkyl, C₁-Haloalkyl, (CH₂)ₘOH, (CH₂)ₘO(C₁-C₂-Alkyl), COO(C₁-C₂-Alkyl), CH₂COO(C₁-C₂-Alkyl) CO(C₁-C₄-Alkyl), Benzyl oder C₂-C₄-Alkenyl,
mit m = 1 - 2
- R^{11c}: ist Wasserstoff, Halogen oder Methyl,
- R^{11d}: ist Wasserstoff, Halogen, Methyl, Ethyl, Propyl, Hydroxy, OCH₃, OCH₂CH₃, OCF₃, COOMe oder COOEt,
- X: ist Halogen oder C₁-C₄-Halogenalkyl,
mit n = 1
oder für R^{11a} und R^{11b} = Wasserstoff kann n = 1-4 sein;
sowie die agrochemisch wirksamen Salze davon.

Besonders bevorzugt werden Verbindungen der Formel (**I**), in denen ein oder mehrere der Symbole eine der folgenden Bedeutungen haben:
- X¹: ist Stickstoff oder CR³,
- X²: ist Stickstoff oder CR⁴,
wobei X¹ und X² nicht gleichzeitig für Stickstoff stehen,
- R¹ und R⁵: sind unabhängig voneinander H, C₁-C₂-Alkyl, C₁-C₂-Alkoxy, F, Cl oder Br,
- R²: ist Wasserstoff, Fluor, Chlor, Brom, Iod, Nitro, Cyano, C₁-C₃-Alkyl, C₁-C₂-Haloalkyl, Hydroxy, C₁-C₃-Alkoxy, OCH₂CH=CH₂, OCH₂C≡CH, OCONHMe, C₁-C₂-Haloalkoxy, COMe, COH, COEt, CMe(=NOH), CMe(=NOMe), SF₅, S(C₁-C₃)-Alkyl, SCF₃, SCF₂H, SOMe, SO₂Me, CO₂(C₁-C₃-Alkyl), NH₂, NH(C₁-C₂-Alkyl), N(C₁-C₂-Alkyl)₂, NHCO(C₁-C₃-Alkyl), NHCOH, NMeCO(C₁-C₃-Alkyl) oder NHCO₂(C₁-C₃-Alkyl),
- R³ und R⁴: sind unabhängig voneinander Wasserstoff, Halogen, Cyano, Nitro, Hydroxy, O-C₁-C₄-Alkyl, O-cylopentyl, OCF₃, OCF₂H, OCF₂CF₃, OCF₂CF₂H, OCONH(C₁-C₃-Alkyl), OCON(C₁-C₃-Alkyl)₂, OCO(C₁-C₄-Alkyl), OSO₂N(CH₃)₂, SH, SF₅, S-C₁-C₃-Alkyl, SCF₃, SCF₂H, SPh, SOMe, SONHMe, SONMe₂, SO₂Me, SO₂CF₃, SO₂CH₂CH=CH₂, SO₂CH₂C≡CH, SO₂N(C₁-C₄-Alkyl)₂, SO₂NHAc, SO₂NHPh, CO(C₁-C₄-Alkyl), COCHF₂, COCF₃, COCH₂CN, CONH(C₁-C₄-Alkyl), CON(C₁-C₄-Alkyl)₂, CONHCH₂CF₃, CONHCH₂CH=CH₂, CONHCH₂C≡CH, CONHCH₂C(=CH₂)CH₃, CONHCH(CH₃)CH₂OCH₃, CONH(CH₂)₂OCH₃, CONHPh, COCH₂NMe₂, CONHcyclopropyl, Piperidin-1-ylcarbonyl, Morpholin-4-ylcarbonyl, (4-Methylpiperazin-1-yl)carbonyl, Formyl, COOH, (C₁-C₃-Alkoxy)carbonyl, CO₂(CH₂)₂OCH₃, NHCO(C₁-C₄-Alkyl), NHCOCH=CH₂, NHCOPh, NHCOCF₃, NHCOC(CH₃)₂CH₂F, NHCOC(CH₃)₂CH₂Cl, NHCO(C=CH₂)CH₃, NHCOCH₂OCH₃, NHCO(CH₂)₂OCH₃, N(C₁-C₂-Alkyl)CO(C₁-C₄-Alkyl), NHCHO, NMeCHO, NHCO₂(C₁-C₄-Alkyl), NHCO₂Ph, NHCO₂CH₂CH₂Cl, N(C₁-C₂-Alkyl)CO₂(C₁-C₂-Alkyl), NH(C=S)OMe, NH₂, NH(C₁-C₄-Alkyl), N(C₁-C₂-Alkyl)₂, cyclopropylamino, acetyl(cyclopropyl)amino, [(1-methylcyclopropyl)carbonyl]amino, morpholin-1-yl, morpholin-4-yl-methyl, NHSO₂Me, NHSO₂CF₃, CH₂CN, CHMeCN, CH₂SO₂NH(C₁-C₄-Alkyl), CH₂SO₂H(C₁-C₄-Alkyl)₂, CH₂COCH₃, CH₂CO*tert*Bu, CH(CH₃)COCH₃, CH₂COCH(CH₃)₂, CH₂COcyclopropyl, CH₂CONH*tert*Bu, CH₂NHCOO(C₁-C₄-Alkyl), CH₂NHCOOBn, CH=NOMe, C(CH₃)=NOMe, CH=NOEt, C(CH₃)=NOEt, CH₂NHAc, CH₂NHCOCF₃, CH₂COOCH₃, CH₂COOEt, C₁-C₄-Alkyl, C₃-C₆-Cycloalkyl, 1-methoxycyclopropyl, 1-chlorcyclopropyl, 3-dimethylbutyl, C₂-C₆-Alkenyl, C₁-C₂-Haloalkyl, morpholin-4-ylsulfonyl, [(4,6-dimethylpyrimidin-2-yl)amino]sulfonyl, 1H-tetrazol-5-yl, (cyclo-propyl-carbonyl)amino, (2-furoyl-amino), (3-methyl-2,5-dioxoimidazolidin-1-yl), (piperidin-1-ylethyl)amino, cyclopropyl(trifluor-acetyl)amino, (1-methylcyclo-propyl)carbonylamino, 4,4-dimethyl-2,5-dioxoimidazolidin-1-yl, 2,3-dimethyl-5-oxo-2,5-dihydro-1H-pyrazol-1-yl, 5-thioxo-4,5-dihydro-1H-tetrazol-1-yl, 3-methyl-2-oxoimida-zolidin-1-yl, 3-(1-methylethyl)-2-oxoimidazolidin-1-yl, 3-(2-methylpropyl)-2-oxoimidazo-lidin-1-yl, 2-oxo-3-prop-2-en-1-ylimidazolidin-1-yl, 3-tert-butyl-2-oxoimidazolidin-1-yl, pyrrolidin-1-ylsulfonyl, 2,5-dioxoimidazolidin-4-yl, piperidin-1-ylsulfonyl, 1,3-thiazol-2-yl, 1,3-thiazol-4-yl, (Dimethylsulfamoyl)methyl, (morpholin-4-ylsulfonyl)methyl, (piperidin-1-ylsulfonyl)methyl, [(4-methylphenyl)amino]sulfonyl, (pyrrolidin-1-ylsulfonyl)methyl, 2-oxoimidazolidin-1-yl, 3-methyl-5-oxo-4,5-dihydro-1H-pyrazol-1-yl, (3,4-dimethyl-5-oxo-4,5-dihydro-1H-pyrazol-1-yl, (1-methylcyclopentyl), pyrrolidin-1-yl, piperidin-1-yl, 2-oxo-2,5-dihydro-1H-pyrrol-1-yl, 3,3-dimethyl-2-oxocyclopentyl, 3-Oxo-4,5-dimethyl-2,4-dihydro-pyrazol-2-yl-, 3-Oxo-4-ethyl-5-methyl-2,4-dihydro-pyrazol-2-yl-, 3-Oxo-5-trifluormethyl-2,4-dihydro-pyrazol-2-yl-, 3-Oxo-5-isopropyl-2,4-dihydro-pyrazol-2-yl-, 3,5-Dioxo-4,4-dimethyl-pyrazolidin-1-yl, 3,5-Dioxo-4-ethyl-pyrazolidin-1-yl, , 3-Oxo-4,4-dimethyl-pyrazolidin-1-yl, 2,5-dioxo-2,5-dihydro-1H-pyrrol-1-yl, 3,5-Dimethylpiperidin-1-yl, 4-(tert-butoxycarbonyl)piperazin-1-yl, 5-Ethoxy-3,4-dimethyl-1H-pyrazol-1-yl, Acetyl(cyclo-hexyl)amino, 2-Furoylamino, 2,2,2-trifluorethyl)carbamoyl, 5-Ethoxy-3-(trifluormethyl)-1H-pyrazol-1-yl, 3-(2-Chlorethyl)-2-oxoimidazolidin-1-yl, 3-oxobutyl, 1,1-dioxido-isothiazolidin-2-yl, 1,1-dioxidotetrahydrothiophen-2-yl, 5-methyl-1,1-dioxido-1,2,5-thiadiazolidin-2-yl, 4-methoxy-2-oxo-2,5-dihydro-1H-pyrrol-1-yl, 2-oxo-2,5-dihydro-1H-pyrrol-1-yl, 5-oxo-4,5-dihydro-1H-imidazol-1-yl, 4-methyl-5-oxo-4,5-dihydro-1H-1,2,4-triazol-1-yl, 3-methyl-5-oxo-2,5-dihydro-1H-pyrazol-1-yl, 2-(methoxymethyl-)pyrrolidin-1-yl, 2-oxocyclopentyl, 2-oxo-tetrahydrofuran-3-yl, 1-methyl-3-oxo-2,3-dihydro-1H-pyrazol-4-yl, 1-methyl-3-oxo-pyrazolidin-4-yl, tetrahydrofuran-2-yl, furan-2-yl, 1,1-dioxido-1,2-thiazinan-2-yl, 5-methyl-1,1-dioxido-1,2,5-thiadiazol-idin-2-yl, 6-methyl-1,1-dioxido-1,2,6-thiadiazinan-2-yl.

Und für den Fall, dass je R³ und R⁴, ggf. über R¹² oder R¹³ einen Zyklus bilden, kann folgende Untereinheit aus der allgemeinen Formel (I): 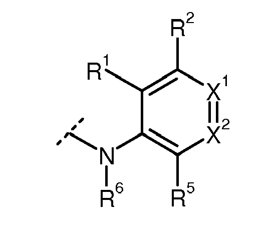 (2-oxo-2,3-dihydro-1H-indol-5-yl)amino, 1H-indol-6-ylamino, 1H-indol-5-ylamino, 2-(trifluormethyl)-1H-benzimidazol-6-yl]amino, (3-methyl-1,1-dioxido-2H-1,2,4-benzo-thiadiazin-7-yl)amino, (1,1-dioxido-2H-1,2,4-benzothiadiazin-6-yl)amino, (4-methyl-3-oxo-3,4-dihydro-2H-1,4-benzoxazin-6-yl)amino, (4-methyl-3-oxo-3,4-dihydro-2H-1,4-benzoxazin-7-yl)amino, (1-acetyl-2,3-dihydro-1H-indol-6-yl)amino, (4H-1,3-benzodioxin-7-ylamino, (2-oxo-2,3,4,5-tetrahydro-1H-1-benzazepin-8-yl)amino, (2,2-dioxido-1,3-dihydro-2-benzothien-5-yl)amino, (1-oxo-2,3-dihydro-1H-inden-5-yl)amino, [2-(ethyl-sulfonyl)-2,3-dihydro-1,3-benzothiazol-6-yl]ami-no, (2,2,3,3-tetrafluor-2,3-dihydro-1,4-benzodioxin-6-yl)amino, 1,3-benzodioxol-5-ylamino, (1,3-dioxo-2,3-dihydro-1H-isoindol-5-yl)amino, 2-methyl-1,3-benzothiazol-6-yl)amino, (2-oxo-2,3-dihydro-1H-benzimidazol-5-yl)amino, (2-oxo-1,3-benzoxathiol-5-yl)amino, 2-oxo-2,3-dihydro-1,3-benzoxazol-5-yl)amino, (2-ethyl-1,3-benzoxazol-5-yl)amino, 2-oxo-1,2,3,4-tetrahydro-quinolin-6-yl)amino, (3-oxo-3,4-dihydro-2H-1,4-benzoxazin-6-yl)amino, (2-oxo-2,3-dihydro-1,3-benzoxazol-6-yl)amino, 3-oxo-1,3-dihydro-2-benzofuran-5-yl)amino, [2-(ethylsulfonyl)-1,3-benzothiazol-6-yl]amino, (2-methyl-1,3-benzothiazol-5-yl)amino, (1-acetyl-2,3-dihydro-1H-indol-5-yl)amino, (2,2,3,3-tetrafluoro-2,3-dihydro-1,4-benzodioxin-6-yl)amino, (2,2-dioxido-1,3-dihydro-2-benzothiophen-5-yl)amino, (2-oxo-2,3-dihydro-1H-indol-6-yl)amino, (2-oxo-1,2,3,4-tetrahydroquinolin-7-yl)amino, 1H-indazol-6-ylamino sein,
- R⁶: ist Wasserstoff, C₁-C₃-Alkyl, CH₂OCH₃, CH₂CH₂OCH₃, Formyl, (C₁-C₄-Alkyl)carbonyl, (C₁-C₄-Haloalkyl)carbonyl, (C₁-C₄-Alkoxy)carbonyl, Benzyloxycarbonyl, Benzyl, 4-Methoxybenzyl, 2,4-Dimethoxybenzyl, 2-Hydroxybenzyl, unsubstituiertes oder substituiertes Benzoyl, unsubstituiertes oder substituiertes C₂-C₄-Alkenyl, unsubstituiertes oder substituiertes C₂-C₄-Alkinyl, C₁-C₂-Alkylsulfinyl oder C₁-C₂-Alkylsulfonyl,
wobei die Substituenten unabhängig voneinander ausgewählt sind aus Wasserstoff, Fluor, Chlor oder Brom, C₁-C₂-Alkyl, C₁-C₂-Halogenalkyl oder Cyano,
- R⁷: ist Wasserstoff, Methyl, Cyano, CF₃ oder CFH₂,
- R⁸: ist Fluor, Chlor, Brom, Iod, Cyano, Methyl, SMe, SOMe, SO₂Me, CF₃, CFH₂ oder CF₂H,
- R⁹: ist Wasserstoff, C₁-C₃-Alkyl, CH₂CH₂OCH₃, CH₂OCH₃, COMe, COCF₃, COOMe, COOEt, Benzyl, 4-Methoxybenzyl, CH₂CH=CH₂ oder CH₂C≡CH,
- R¹⁰: ist eine Gruppe der Formel E1 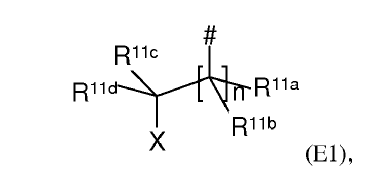
in welcher die Symbole die folgenden Bedeutungen haben und # für die Anknüpfungsstelle an das Stickstoffatom steht:
- R^{11a}: ist Wasserstoff oder Methyl,
- R^{11b}: ist Wasserstoff, Methyl, Ethyl, Propyl, 1-Methylethyl, Butyl, 1-Methylpropyl, 2-Methylpropyl, CH₂F, CH₂OH, CH₂CH₂OH, CH₂OEt, COOMe, COOEt, CH₂COOMe, CH₂COOEt, COMe, Benzyl oder Allyl,
- R^{11c}: ist Wasserstoff, Halogen oder Methyl,
- R^{11d}: ist Wasserstoff, Halogen, Methyl, Hydroxy oder COOMe,
- X: ist Halogen oder C₁-C₃-Halogenalkyl,
mit n = 1
oder für R^{11a} und R^{11b} = Wasserstoff kann n = 1-3 sein;
sowie die agrochemisch wirksamen Salze davon.

Ganz besonders bevorzugt werden Verbindungen der Formel (**I**), in denen ein oder mehrere der Symbole eine der folgenden Bedeutungen haben:
- X¹: ist CR³,
- X²: ist Stickstoff oder CR⁴
- R¹ und R⁵: sind unabhängig voneinander Wasserstoff, Methyl, Methoxy oder F,
- R²: ist Wasserstoff, Fluor, Chlor, Brom, Iod, Nitro, Cyano, Methyl, Ethyl, Propanyl, Propan-2-yl, CF₃, CF₂H, CFH₂, CF₂CF₃, Hydroxy, OMe, OEt, OPr, O*iso*Pr, OCH₂CH=CH₂, OCH₂C≡CH, OCONHMe, OCF₃, OCF₂CF₃, OCF₂CF₂H, COMe, COH, COEt, C(=NOH)Me, C(=NOMe)Me, SF₅, SMe, SEt, SPr, SiPr, SCF₃, SCF₂H, SOMe, SO₂Me, CO₂CH₃, CO₂Et, NHMe, NMe₂, NHEt, NEt₂ NHCOMe NHCOH, NMeCOMe, NHCO₂Me oder NHCO₂Et,
- R³ und R⁴: sind unabhängig voneinander Wasserstoff, Fluor, Chlor, Brom, Iod, Cyano, Nitro,
Hydroxy, O-C₁-C₄-Alkyl, O-cylopentyl, OCF₃, OCF₂H, OCF₂CF₃, OCF₂CF₂H, OCONH(C₁-C₃-Alkyl), OCON(C₁-C₃-Alkyl)₂, OCO(C₁-C₄-Alkyl), SH, SF₅, S-C₁-C₃-Alkyl, SCF₃, SCF₂H, SPh, SOMe, SONHMe, SONMe₂, SO₂Me, SO₂CF₃, SO₂CH₂CH=CH₂, SO₂CH₂C≡CH, SO₂N(C₁-C₄-Alkyl)₂, SO₂NHAc, SO₂NHPh, Formyl, CO(C₁-C₄-Alkyl), COCHF₂, COCF₃, COCH₂CN, CONH(C₁-C₄-Alkyl), CON(C₁-C₄-Alkyl)₂, CONHCH₂CF₃, CONHCH₂CH=CH₂, CONHCH₂C≡CH, CONHCH₂C(=CH₂)CH₃, CONHCH(CH₃)CH₂OCH₃, CONH(CH₂)₂OCH₃, CONHPh, COCH₂NMe₂, CONHcyclopropyl, Piperidin-1-ylcarbonyl, Morpholin-4-ylcarbonyl, (4-Methylpiperazin-1-yl)carbonyl, COOH, (C₁-C₃-Alkoxy)carbonyl, CO₂(CH₂)₂OCH₃, NHCO(C₁-C₄-Alkyl), NHCOCH=CH_{2,} NHCOPh, NHCOCF₃, NHCOC(CH₃)₂CH₂F, NHCOC(CH₃)₂CH₂Cl, NHCO(C=CH₂)CH_{3,} NHCOCH₂OCH₃, NHCO(CH₂)₂OCH₃, N(CH₃)COCH₃, N(C₂H₅)COCH₃, N(CH₃)COC(CH₃)₃, NHCHO, NMeCHO, NHCO₂(C₁-C₄-Alkyl), NHCO₂Ph, NHCO₂CH₂CH₂Cl, NEtCO₂Me, NMeCO₂Me, NH(C=S)OMe, NH₂, NH(C₁-C₄-Alkyl), N(C₁-C₂-Alkyl)₂, cyclopropylamino, acetyl(cyclopropyl)amino, [(1-methylcyclopropyl)carbonyl]amino, morpholin-1-yl, morpholin-4-yl-methyl, NHSO₂Me, NHSO₂CF₃, CH₂CN, CHMeCN, CH₂SO₂NH(C₁-C₄-Alkyl), CH₂SO₂N(C₁-C₄-Alkyl)₂, CH₂COCH₃, CH₂CO*tert*Bu, CH(CH₃)COCH₃, CH₂COCH(CH₃)₂, CH₂COcyclopropyl, CH₂CONH*tert*Bu, CH₂NHCOO(C₁-C₄-Alkyl), CH₂NHCOOBn, CH=NOMe, C(CH₃)=NOMe, CH=NOEt, C(CH₃)=NOEt, CH₂NHAc, CH₂NHCOCF₃ CH₂COOCH₃, CH₂COOEt, C₁-C₄-Alkyl, C₃-C₆-Cycloalkyl, 1-methoxycyclopropyl, 1-chlorcyclopropyl, 3,3-dimethylbutyl, C₂-C₆-Alkenyl, CF₃, CF₂H, CCl₃, C₂F₅, morpholin-4-ylsulfonyl, [(4,6-dimethylpyrimidin-2-yl)amino]sulfonyl, 1H-tetrazol-5-yl, (cyclo-propyl-carbonyl)amino, (2-furoyl-amino), (3-methyl-2,5-dioxo-imidazolidin-1-yl), cyclo-propyl(trifluoracetyl)amino, (1-methylcyclo-propyl)carbonylamino, 4,4-dimethyl-2,5-dioxoimidazolidin-1-yl, 2,3-dimethyl-5-oxo-2,5-dihydro-1H-pyrazol-1-yl, 5-thioxo-4,5-dihydro-1H-tetrazol-1-yl, 3-methyl-2-oxoimidazolidin-1-yl, 3-(1-methylethyl)-2-oxoimidazolidin-1-yl, 3-(2-methylpropyl)-2-oxoimidazolidin-1-yl, 2-oxo-3-prop-2-en-1-ylimidazolidin-1-yl, 3-tert-butyl-2-oxoimidazolidin-1-yl, pyrrolidin-1-ylsulfonyl, 2,5-dioxoimidazolidin-4-yl, piperidin-1-ylsulfonyl, (Dimethylsulfamoyl)methyl, 1,3-thiazol-2-yl, 1,3-thiazol-4-yl, (morpholin-4-ylsulfonyl)methyl, (piperidin-1-ylsulfonyl)methyl, [(4-methylphenyl)amino]sulfonyl, (pyrrolidin-1-ylsulfonyl)methyl, 2-oxoimidazolidin-1-yl, 3-methyl-5-oxo-4,5-dihydro-1H-pyrazol-1-yl, (3,4-dimethyl-5-oxo-4,5-dihydro-1H-pyrazol-1-yl, pyrrolidin-1-yl, piperidin-1-yl, 2-oxo-2,5-dihydro-1H-pyrrol-1-yl, 3,3-dimethyl-2-oxocyclopentyl, 3-Oxo-4,5-dimethyl-2,4-dihydro-pyrazol-2-yl-, 3-Oxo-4-ethyl-5-methyl-2,4-dihydro-pyrazol-2-yl-, 3-Oxo-5-trifluormethyl-2,4-dihydro-pyrazol-2-yl-, 3-Oxo-5-isopropyl-2,4-dihydro-pyrazol-2-yl-, 3,5-Dioxo-4,4-dimethyl-pyrazolidin-1-yl, 3,5-Dioxo-4-ethyl-pyrazolidin-1-yl, 3-Oxo-4,4-dimethyl-pyr-azolidin-1-yl, 2,5-dioxo-2,5-dihydro-1H-pyrrol-1-yl, 3,5-Dimethyl-piperidin-1-yl, 4-(tert-butoxycarbonyl)piperazin-1-yl, 5-Ethoxy-3,4-dimethyl-1H-pyrazol-1-yl, Acetyl(cyclo-hexyl)amino, 2-Furoyl-amino, 2,2,2-tri-fluorethyl)carbamoyl, 5-Ethoxy-3-(trifluormethyl)-1H-pyrazol-1-yl, 3-(2-Chlorethyl)-2-oxoimidazolidin-1-yl, 3-oxobutyl, 1,1-dioxido-isothiazolidin-2-yl, 1,1-dioxidotetra-hydrothiophen-2-yl, 5-methyl-1,1-dioxido-1,2,5-thiadiazolidin-2-yl, 4-methoxy-2-oxo-2,5-dihydro-1H-pyrrol-1-yl, 2-oxo-2,5-dihydro-1H-pyrrol-1-yl, 5-oxo-4,5-dihydro-1H-imidazol-1-yl, 4-methyl-5-oxo-4,5-dihydro-1H-1,2,4-triazol-1-yl, 3-methyl-5-oxo-2,5-dihydro-1H-pyrazol-1-yl, 2-(methoxymethyl)pyrrolidin-1-yl, 2-oxo-cyclopentyl, 2-oxotetrahydrofuran-3-yl, 1-methyl-3-oxo-2,3-dihydro-1H-pyrazol-4-yl, 1-methyl-3-oxopyrazolidin-4-yl, tetrahydrofuran-2-yl, furan-2-yl., 1,1-dioxido-1,2-thia-zinan-2-yl, 5-methyl-1,1-dioxido-1,2,5-thiadiazolidin-2-yl, 6-methyl-1,1-dioxido-1,2,6-thiadiazinan-2-yl,
und für den Fall, dass R³ und R⁴, ggf. über R¹² oder R¹³ einen Zyklus bilden, kann folgende Untereinheit aus der allgemeinen Formel (I): 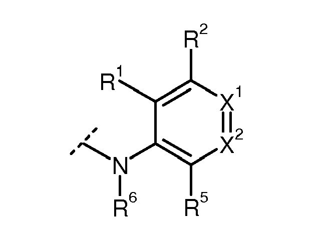 (2-oxo-2,3-dihydro-1H-indol-5-yl)amino, 1H-indol-6-ylamino, 1H-indol-5-ylamino, (4-methyl-3-oxo-3,4-dihydro-2H-1,4-benzoxazin-6-yl)amino, (4-methyl-3-oxo-3,4-dihydro-2H-1,4-benzoxazin-7-yl)amino, (1-acetyl-2,3-dihydro-1H-indol-6-yl)amino, (2-oxo-2,3,4,5-tetrahydro-1H-1-benzaze-pin-8-yl)amino, 1,3-benzodioxol-5-ylamino, (1,3-dioxo-2,3-dihydro-1H-isoindol-5-yl)amino, (2-oxo-1,3-benzoxathiol-5-yl)amino, 2-oxo-2,3-dihydro-1,3-benzoxazol-5-yl)amino, (2-ethyl-1,3-benz oxazol-5-yl)amino, 2-oxo-1,2,3,4-tetrahydroquinolin-6-yl)amino, (3-oxo-3,4-dihydro-2H-1,4-benz-oxazin-6-yl)amino, (2-oxo-2,3-dihydro-1,3-benzoxazol-6-yl)amino, [2-(ethylsulfonyl)-1,3-benzo-thiazol-6-yl]amino, (2-methyl-1,3-benzothiazol-5-yl)amino, (1-acetyl-2,3-dihydro-1H-indol-5-yl)amino, (2,2,3,3-tetrafluoro-2,3-dihydro-1,4-benzodioxin-6-yl)amino, (2,2-dioxido-1,3-dihydro-2-benzothiophen-5-yl)amino sein,
- R⁶: ist Wasserstoff, Methyl, Ethyl, Propanyl, CH₂OCH₃, Formyl, COMe, COCF₃, COOMe, COOEt, COO*tert*Bu, Benzyloxycarbonyl, Benzyl, Benzoyl oder 2-Methylbenzoyl,
- R⁷: ist Wasserstoff, Methyl oder CF₃,
- R⁸: ist Fluor, Chlor, Brom, Iod, Cyano, Methyl, SMe, SOMe, SO₂Me, CF₃, CFH₂ oder CF₂H,
- R⁹: ist Wasserstoff, Methyl, Ethyl, Propanyl, Benzyl, 4-Methoxybenzyl, CH₂CH=CH₂ oder CH₂C≡CH,
- R¹⁰: ist 2,2-Difluorethyl, 2,2-Dichlorethyl, 1,1,1-Trifluorpropan-2-yl, 2,2,2-Trifluorethyl, 2,2,2-Trichlorethyl, 4,4,4-Trifluorbutan-2-yl, 2-Fluorethyl, 2-Chlorethyl, 2,2,3,3-Tetrafluorpropan-1-yl, 3,3,3-Trifluorpropan-1yl, 2,2-Difluorpropan-1-yl, 1,1,1-Trifluor-4-methyl-pentan-2-yl, 1,1,1-Trifluorpentan-2-yl, 1,1,1-Trifluorbutan-2-yl, 1-Fluorpropan-2-yl, 2-Fluorpropan-1-yl, 2,3,3,3-Tetrafluorpropan-1-yl, 2,2,3,3,4,4,5,5,5-Nonafluorpentan-1-yl, 2,2,3,3,4,4,5,5-Octafluorpentan-1-yl, 3-Fluorpropan-1-yl, 3,3-Difluorpropan-1-yl, 3,3,3-Trifluorpropan-1-yl, 3-Chlorpropan-1-yl, 3,3-Dichlorpropan-1-yl, 3,3,3-Trichlorpropan-1-yl, 2-Brom-2,2-difluorethyl, 2-Chlor-2,2-difluorethyl, 3-Brom-3,3-difluorpropan-1 yl, 2,2,3,3,3-Pentafluorpropan-1-yl, 2,2,3,3,4,4,4-Heptafluorbutan-1-yl, 3,3,4,4,4-Pentafluorbutan-1-yl, 3,3,4,4,5,5-Heptafluorpentan-1-yl, 4,4,4-Trifluorbutan-1-yl, 4,4-Difluorbutan-1-yl, 1,3-Difluorpropan-2-yl, 1-Fluor-2-methylpropan-2-yl, 1,3-Difluor-2-methylpropan-2-yl, 1,1,1-Trifluor-2-methylpropan-2-yl, 1,1-Difluorpropan-2-yl, 1,1,1-Trifluor-3-hydroxypropan-2-yl, 1,1,1 -Trifluor-3-ethoxypropan-2-yl, 1,1,1-Trifluor-4-hydroxybutan-2-yl, 1,1,1-Trifluorpent-4-en-2-yl, 3-Chlor-2-methylpropyl, 2-Hydroxy-3,3,3-trifluorpropan-1-yl, 2-Methyl-3,3,3-Trifluorpropan-1-yl, 3-Methyl-4,4,4-trifluorbutan-1-yl, 4,4,4-Trifluor-3-(trifluormethyl)butan-1-yl, 6,6,6-Trifluor-5-methylhexan-3-yl, 6,6,6-Trifluorhexan-3-yl, 5,5,5-Trifluor-4-methylpentan-2-yl, 5,5,5-Trifluorpentan-2-yl, 2-Hydroxy-4,4,4-trifluorbutan-1-yl, 3-Methyl-1,1,1-trifluorbutan-2-yl, 2-Methyl-4,4,4-trifluorbutan-2-yl, 1,1,1-Trifluor-3-phenylpropan-2-yl, 1,1,1-Trifluor-4-methoxy-4-oxobutan-2-yl, 4-Ethoxy-1,1,1-trifluor-4-oxobutan-2-yl, 1,1,1-Trifluor-3-methoxy-3-oxopropan-2-yl, 3,3,3-Trifluor-2-(methoxycarbonyl)propan-1-yl, 3-Brombutan-1-yl, 1-Brombutan-2-yl, 2-Brompropan-1-yl, 2-Chlorpropan-1-yl, 4-Chlorbutan-2-yl, 4,4,4-Trichlorbutan-2-yl, 1-Ethoxy-4-fluor-3-hydroxy-1-oxobutan-2-yl, 3,3,3-Trifluor-2-hydroxy-2-methylpropan-1-yl, 4,4,4-Trifluor-3-hydroxybutan-2-yl, 2,3-Dichlorpropan-1-yl, 2,3-Dibrompropan-1-yl, 1,1,1-Trifluor-3-oxobutan-2-yl oder 2,5-Dichlorpentan-1-yl,
sowie die agrochemisch wirksamen Salze davon.

Insbesondere bevorzugt werden Verbindungen der Formel **(I),** in denen ein oder mehrere der Symbole eine der folgenden Bedeutungen haben:
- X¹: CR³,
- X²: CR⁴,
- R¹ und R⁵: sind unabhängig voneinander Wasserstoff, Methyl, Methoxy oder F,
- R²: ist Wasserstoff, Fluor, Chlor, Brom, Cyano, Methyl, Ethyl, Propanyl, Propan-2-yl, CF₃, Hydroxy, OMe, OEt, OCF₃, COMe, COEt, SMe, SEt, CO₂CH₃, CO₂Et, NHMe, NMe₂, NHEt, NEt₂ oder NHCOMe,
- R³ und R⁴: sind unabhängig voneinander Wasserstoff, Halogen, Cyano, Nitro, Hydroxy, O-C₁-C₄-Alkyl, OCF₃, OCF₂H, OCF₂CF₃, OCF₂CF₂H, OCONH(C₁-C₃-Alkyl), OCO(C₁-C₄-Alkyl), SH, SF₅, S-C₁-C₃-Alkyl, SCF₃, SCF₂H, SPh, SOMe, SO₂Me, SO₂CF₃, SO₂CH₂CH=CH_{2,} SO₂CH₂C≡CH, SO₂N(C₁-C₄-Alkyl)₂, SO₂NHAc, SO₂NHPh, Formyl, CO(C₁-C₄-Alkyl), COCHF₂, COCF₃, COCH₂CN, CONH(C₁-C₄-Alkyl), CON(C₁-C₄-Alkyl)₂, CONHCH₂CF₃, CONHCH₂CH=CH₂, CONHCH₂C≡CH, CONHCH₂C(=CH₂)CH₃, CONHPh, CONHcyclopropyl, piperidin-1-ylcarbonyl, morpholin-4-ylcarbonyl, (4-methylpiperazin-1-yl)carbonyl, (C₁-C₃-Alkoxy)carbonyl, CO₂(CH₂)₂OCH₃, NHCO(C₁-C₄-Alkyl), NHCOCF_{3,} NHCOCH=CH_{2,} NHCOPh, NHCOC(CH₃)₂CH₂F, NHCO(C=CH₂)CH_{3,} NHCOCH₂OCH₃, NHCO(CH₂)₂OCH₃, N(CH₃)COCH₃, N(C₂H₅)COCH₃, N(CH₃)COC(CH₃)₃, NHCHO, NMeCHO, NHCO₂(C₁-C₄-Alkyl), NHCO₂Ph, NEtCO₂Me, NMeCO₂Me, NH(C=S)OMe, NH(C₁-C₄-Alkyl), N(C₁-C₂-Alkyl)₂, cyclopropylamino, acetyl(cyclopropyl)amino, [(1-methylcyclopropyl)carbonyl]amino, morpholin-1-yl, morpholin-4-yl-methyl, NHSO₂Me, NHSO₂CF₃, CH₂CN, CHMeCN, CH₂SO₂NH(C₁-C₄-Alkyl), CH₂SO₂N(C₁-C₄-Alkyl)₂, CH₂COCH₃, CH₂CO*tert*Bu, CH(CH₃)COCH₃, CH₂COCH(CH₃)₂, CH₂COcyclopropyl, CH₂CONH*tert*Bu, CH₂NHCOO(C₁-C₄-Alkyl), CH=NOMe, C(CH₃)=NOMe, CH=NOEt, C(CH₃)=NOEt, CH₂NHCOCF₃, CH₂COOCH₃, CH₂COOEt, C₁-C₄-Alkyl, C₃-C₆-Cycloalkyl, prop-2-en-1-yl, 1-methylprop-2-en-1-yl, but-3-en-1-yl, CF₃, CF₂H, C₂F₅, morpholin-4-ylsulfonyl, [(4,6-dimethylpyrimidin-2-yl)amino]sulfonyl, 1H-tetrazol-5-yl, (cyclo-propyl-carbonyl)amino, (2-furoyl-amino), (3-methyl-2,5-dioxoimidazolidin-1-yl), cyclopropyl(trifluor-acetyl)amino, (1-methylcyclo-propyl)carbonylamino, 4,4-dimethyl-2,5-dioxoimidazolidin-1-yl, 2,3-dimethyl-5-oxo-2,5-dihydro-1H-pyrazol-1-yl, 3-methyl-2-oxoimidazolidin-1-yl, 3-(1-methylethyl)-2-oxoimidazolidin-1-yl, 3-(2-methylpropyl)-2-oxoimidazolidin-1-yl, 3-tert-butyl-2-oxoimidazolidin-1-yl, pyrrolidin-1-ylsulfonyl, 2,5-dioxoimidazolidin-4-yl, piperidin-1-ylsulfonyl, (Dimethylsulfamoyl)methyl, 1,3-thiazol-2-yl, 1,3-thiazol-4-yl, (morpholin-4-ylsulfonyl)methyl, (piperidin-1-ylsulfonyl)methyl, [(4-methylphenyl)ami-no]sulfonyl,-(pyrrolidin-1-ylsulfonyl)methyl, 2-oxoimidazolidin-1-yl, 3-methyl-5-oxo-4,5-dihydro-1H-pyrazol-1-yl, (3,4-dimethyl-5-oxo-4,5-dihydro-1H-pyrazol-1-yl, pyrrolidin-1-yl, piperidin-1-yl, 3-Oxo-4,5-dimethyl-2,4-dihy-dropyrazol-2-yl-, 3-Oxo-4-ethyl-5-methyl-2,4-dihydro-pyrazol-2-yl-, 3-Oxo-5-trifluor-methyl-2,4-dihydro-pyrazol-2-yl-, 3-Oxo-5-isopropyl-2,4-dihydro-pyrazol-2-yl-, 3,5-Dioxo-4,4-dimethyl-pyrazolidin-1-yl, 3,5-Dioxo-4-ethyl-pyrazolidin-1-yl, 3-Oxo-4,4-dimethyl-pyrazolidin-1-yl, 2,5-dioxo-2,5-dihydro-1H-pyrrol-1-yl, 3,5-Dimethylpiperidin-1-yl, 4-(tert-butoxycarbonyl)piperazin-1-yl, Acetyl-(cyclohexyl)amino, 2-Furoylamino, 3-(2-Chlorethyl)-2-oxoimidazolidin-1-yl, 1,1-dioxidoisothiazolidin-2-yl, 1,1-dioxidotetrahydrothiophen-2-yl, 5-methyl-1,1-dioxido-1,2,5-thiadiazolidin-2-yl, 4-methoxy-2-oxo-2,5-dihydro-1H-pyrrol-1-yl, 5-oxo-4,5-dihydro-1H-imidazol-1-yl, 3-methyl-5-oxo-2,5-dihydro-1H-pyrazol-1-yl, 2-(methoxymethyl)pyrrolidin-1-yl, 2-oxotetrahydrofuran-3-yl, 1-me-thyl-3-oxo-2,3-dihydro-1H-pyrazol-4-yl, tetrahydro-fura-n-2-yl, furan-2-yl., 1,1-dioxido-1,2-thiazinan-2-yl, 5-methyl-1,1-dioxido-1,2,5-thiadiazolidin-2-yl, 6-methyl-1,1-dioxido-1,2,6-thiadiazinan-2-yl.
und für den Fall, dass R³ und R⁴, ggf. über R¹² oder R¹³ einen Zyklus bilden, kann folgende Untereinheit aus der allgemeinen Formel (**I**): 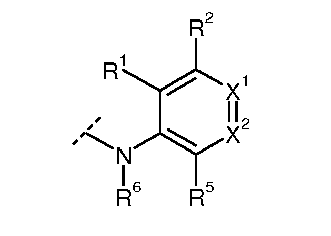 (2-oxo-2,3-dihydro-1H-indol-5-yl)amino, 1H-indol-6-ylamino, 1H-indol-5-ylamino, (4-methyl-3-oxo-3,4-dihydro-2H-1,4-benzoxazin-6-yl)amino, (4-methyl-3-oxo-3,4-dihydro-2H-1,4-benzoxazin-7-yl)amino, (1-acetyl-2,3-dihydro-1H-indol-6-yl)amino, (2-oxo-2,3,4,5-tetrahydro-1H-1-benzazepin-8-yl)amino, 1,3-benzodioxol-5-ylamino, (1,3-dioxo-2,3-dihydro-1H-isoindol-5-yl)amino, (2-oxo-1,3-benzoxathiol-5-yl)amino, 2-oxo-2,3-dihydro-1,3-benzoxazol-5-yl)amino, (2-ethyl-1,3-benz oxazol-5-yl)amino, 2-oxo-1,2,3,4-tetrahydroquinolin-6-yl)amino, (3-oxo-3,4-dihydro-2H-1,4-benzoxazin-6-yl)amino, (2-oxo-2,3-dihydro-1,3-benzoxazol-6-yl)amino, [2-(ethylsulfonyl)-1,3-benzothiazol-6-yl]amino, (2-methyl-1,3-benzothiazol-5-yl)amino, (1-acetyl-2,3-dihydro-1H-indol-5-yl)amino, (2,2,3,3-tetrafluoro-2,3-dihydro-1,4-benzodioxin-6-yl)amino, (2,2-dioxido-1,3-dihydro-2-benzothiophen-5-yl)amino sein,
- R⁶: ist Wasserstoff, Methyl, Formyl oder COMe,
- R⁷: ist Wasserstoff,
- R⁸: ist Fluor, Chlor, Brom, Iod, Cyano, Methyl, SMe, SOMe, SO₂Me oder CF₃,
- R⁹: ist Wasserstoff" Methyl, Ethyl, Propanyl, CH₂CH=CH₂ oder CH₂C≡CH,
- R¹⁰: ist 2,2-Difluorethyl, 2,2-Dichlorethyl, 1,1,1-Trifluorpropan-2-yl, 2,2,2-Trifluorethyl, 2,2,2-Trichlorethyl, 4,4,4-Trifluorbutan-2-yl, 2-Fluorethyl, 2-Chlorethyl, 2,2,3,3-Tetrafluorpropan-1-yl, 3,3,3-Trifluorpropan-1yl, 2,2-Difluorpropan-1-yl, 1,1,1-Trifluor-4-methyl-pentan-2-yl, 1,1,1-Trifluorpentan-2-yl, 1,1,1-Trifluorbutan-2-yl, 1-Fluorpropan-2-yl, 2-Fluorpropan-1-yl, 2,3,3,3-Tetrafluorpropan-1-yl, 2,2,3,3,4,4,5,5,5-Nonafluorpentan-1-yl, 2,2,3,3,4,4,5,5-Octafluorpentan-1-yl, 3-Fluorpropan-1-yl, 3,3-Difluorpropan-1-yl, 3,3,3-Trifluorpropan-1-yl, 3-Chlorpropan-1-yl, 3,3-Dichlorpropan-1-yl, 3,3,3-Trichlorpropan-1-yl, 2-Brom-2,2-difluorethyl, 2-Chlor-2,2-difluorethyl, 3-Brom-3,3-difluorpropan-1 yl, 2,2,3,3,3-Pentafluorpropan-1-yl, 2,2,3,3,4,4,4-Heptafluorbutan-1-yl, 3,3,4,4,4-Pentafluorbutan-1-yl, 3,3,4,4,5,5-Heptafluorpentan-1-yl, 4,4,4-Trifluorbutan-1-yl, 4,4-Difluorbutan-1-yl, 1,3-Difluorpropan-2-yl, 1,1-Difluorpropan-2-yl, 1,1,1-Trifluor-3-hydroxypropan-2-yl, 1,1,1-Trifluor-3-ethoxypropan-2-yl, 1,1,1-Trifluor-4-hydroxybutan-2-yl, 1,1,1-Trifluorpent-4-en-2-yl, 3-Chlor-2-methylpropyl, 2-Hydroxy-3,3,3-trifluorpropan-1-yl oder 2,5-Dichlorpentan-1-yl,
sowie die agrochemisch wirksamen Salze davon.

Weiterhin insbesondere bevorzugt werden Verbindungen der Formel (**I**), in denen ein oder mehrere der Symbole eine der folgenden Bedeutungen haben:
- X¹: ist CR³,
- X²: ist CR⁴,
- R¹ und R⁵: sind unabhängig voneinander Wasserstoff,
- R²: ist Wasserstoff, Fluor, Chlor, Brom, Cyano, Methyl, CF₃, Hydroxy, OMe, OEt, OCF₃, COMe, SMe, CO₂CH₃, CO₂Et, NHMe, NMe₂, NHEt, NEt₂ oder NHCOMe,
- R³ und R⁴: sind unabhängig voneinander Wasserstoff, Fluor, Chlor, Brom, Cyano, Hydroxy, OMe, OEt, OPr, O*iso*Pr, OCF₃, OCONHMe, OCONHEt, OCONMe₂, SF₅, SMe, SEt, S*n*Pr, SCF₃, SCF₂H, SO₂Me, SO₂CF₃, SO₂NMe₂, SO₂NHPh, Formyl, COMe, COCF₃, COCH₂CN, CONHMe, CONMe₂, CONHEt, CONEt₂, CONHPr, CONH*iso*Pr, CONH*tert*Bu, CONHCH₂CH=CH₂, CONHCH₂C≡CH, CONHPh, CONHcyclopropyl, Piperidin-1-ylcarbonyl, Morpholin-4-ylcarbonyl, CO₂CH₃, CO₂Et, CO₂Pr, CO₂*iso*Pr, NHCOMe, NHCOEt, NHCOPr, NHCO*iso*Pr, NHCOCF₃, NHCO*tert*Bu, N(C₂H₅)COMe, NHCOCH=CH₂, NHCOPh, NHCOC(CH₃)₂CH₂F, NHCO(C=CH₂)CH₃, N(CH₃)COCH₃, NHCHO, NMeCHO, NHCO₂Me, NHCO₂Et, NHCO₂Ph, NHMe, NMe₂, NHEt, NEt₂, NHPr, NH*iso*Pr, Cyclopropylamino, Acetyl(cyclopropyl)amino, [(1-methylcyclopropyl)carbonyl]amino, morpholin-1-yl, morpholin-4-yl-methyl, CH₂CN, CHMeCN, CH₂SO₂NHMe, CH₂SO₂NHPr, CH₂COCH₃, CH₂CO*tert*Bu, CH₂CO₂Et, Methyl, Ethyl, Propyl, 1-Methylethyl, Butyl, 1-Methyl-propyl, 2-Methylpropyl, 1,1-Dimethylethyl, prop-2-en-1-yl, 1-methylprop-2-en-1-yl, but-3-en-1-yl, CF₃, CF₂H, morpholin-4-ylsulfonyl, (3-methyl-2,5-dioxoimidazolidin-1-yl), 3-methyl-2-oxoimidazolidin-1-yl, 2,5-dioxoimidazolidin-4-yl, piperidin-1-ylsulfonyl, (Dimethylsulfamoyl)methyl, 1,3-thiazol-2-yl, 1,3-thiazol-4-yl, [(4-methylphenyl)amino]sulfonyl, 2,5-dioxo-2,5-dihydro-1H- pyrrol-1-yl, 3,5-Dimethylpiperidin-1-yl, 4-(tert-butoxycarbonyl)piperazin-1-yl, Acetyl(cyclo-hexyl-)amino, 2-Furoylamino, 3-(2-Chlorethyl)-2-oxoimidazolidin-1-yl, 5-methyl-1,1-dioxido-1,2,5-thiadiazolidin-2-yl, oder 3-me-thyl-5-oxo-2,5-dihydro-1H-pyrazol-1-yl,
und für den Fall, dass je R³ und R⁴, ggf. über R¹² oder R¹³ einen Zyklus bilden, kann folgende Untereinheit aus der allgemeinen Formel (**I**): 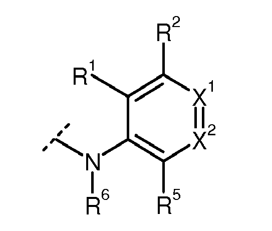 1H-indol-6-ylamino, 1H-indol-5-ylamino, (4-methyl-3-oxo-3,4-dihydro-2H-1,4-benzoxazin-6-yl)amino, (1-acetyl-2,3-dihydro-1H-indol-6-yl)amino, (2-oxo-2,3,4,5-tetrahydro-1H-1-benzaze-pin-8-yl)amino, 1,3-benzodioxol-5-ylamino, (2-oxo-1,3-benzoxathiol-5-yl)amino, 2-oxo-2,3-dihydro-1,3-benzoxazol-5-yl)amino, (2-ethyl-1,3-benz oxazol-5-yl)amino, 2-oxo-1,2,3,4-tetrahydroquinolin-6-yl)amino, (2-oxo-2,3-dihydro-1,3-benzoxazol-6-yl)amino, (2-methyl-1,3-benzothiazol-5-yl)amino, (1-acetyl-2,3-dihydro-1H-indol-5-yl)amino, (2,2,3,3-tetrafluoro-2,3-dihydro-1,4-benzodioxin-6-yl)amino, sein,
- R⁶: ist Wasserstoff,
- R⁷: ist Wasserstoff,
- R⁸: ist Chlor, Brom oder CF₃,
- R⁹: ist Wasserstoff, Methyl oder Ethyl
- R¹⁰: ist 2,2-Difluorethyl, 1,1,1-Trifluorpropan-2-yl, 2,2,2-Trifluorethyl, 4,4,4-Trifluorbutan-2-yl, 2-Fluorethyl, 2-Chlorethyl, 2,2,3,3-Tetrafluorpropan-1-yl, 3,3,3-Trifluorpropan-1yl, 2,2-Difluorpropan-1-yl, 1,1,1-Trifluor-4-methyl-pentan-2-yl, 1-Fluorpropan-2-yl, 2-Fluorpropan-1-yl, 2,3,3,3-Tetrafluorpropan-1-yl, 2,2,3,3,4,4,5,5,5-Nonafluorpentan-1-yl, 2,2,3,3,4,4,5,5-Octafluorpentan-1-yl, 3-Fluorpropan-1-yl, 3,3,3-Trifluorpropan-1-yl, 2-Brom-2,2-difluorethyl, 3-Brom-3,3-difluorpropan-1 yl, 2,2,3,3,3-Pentafluorpropan-1-yl, 2,2,3,3,4,4,4-Heptafluorbutan-1-yl, 3,3,4,4,4-Pentafluorbutan-1-yl, 3,3,4,4,5,5-Heptafluorpentan-1-yl, 4,4,4-Trifluorbutan-1-yl oder 2,5-Dichlorpentan-1-yl,
wobei die übrigen Substituenten eine oder mehrere der oben genannten Bedeutungen haben,
sowie die agrochemisch wirksamen Salze davon.

Weiterhin insbesondere bevorzugt werden Verbindungen der Formel (**I**), in denen ein oder mehrere der Symbole eine der folgenden Bedeutungen haben:
- X¹: ist CR³,
- X²: ist CR⁴,
- R¹ und R⁵: sind unabhängig voneinander Wasserstoff,
- R²: ist Wasserstoff,
- R³: steht für Wasserstoff, Cyano, OMe, SMe, SO₂Me, SO₂NMe₂, Formyl, COMe, CONHMe, CONMe₂, CONHCH₂CH=CH₂, NHCOMe, NHCOCF₃, N(C₂H₅)COMe, N(CH₃)COCH₃, NHCO₂Me, NHCO₂Et, [(1-Methylcyclopropyl)carbonyl]amino, CH₂CO*tert*Bu, Methyl oder 2,5-Dioxoimidazolidin-4-yl,
- R⁴: steht für Wasserstoff, Chlor, Cyano, OMe, OCF₃, OCONHMe, SMe, Formyl, COMe, CONHMe, NHCOMe, NHCO*tert*Bu, NHCOC(CH₃)₂CH₂F, NHCO(C=CH₂)CH₃, NHCO₂Me, NHCO₂Et, CH₂CN, Methyl oder Piperidin-1-ylsulfonyl,
und für den Fall, dass R³ und R⁴ einen Zyklus bilden, kann folgende Untereinheit aus der allgemeinen Formel (**I**): 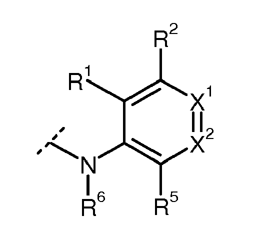 (2-Oxo-2,3-dihydro-1,3-benzoxazol-6-yl)amino, sein,
- R⁶: ist Wasserstoff,
- R⁷: ist Wasserstoff,
- R⁸: ist Chlor oder Brom,
- R⁹: ist Wasserstoff,
- R¹⁰: ist 2,2-Difluorethyl, 1,1,1-Trifluorpropan-2-yl, 2,2,2-Trifluorethyl oder 4,4,4-Trifluorbutan-2-yl,
wobei die übrigen Substituenten eine oder mehrere der oben genannten Bedeutungen haben,
sowie die agrochemisch wirksamen Salze davon.

Weiterhin bevorzugt werden Verbindungen der Formel (I), in denen ein oder mehrere der Symbole eine der folgenden Bedeutungen haben:
- X¹: ist CR³,
- X²: ist CR⁴,
- R³: ist Wasserstoff, Cyano, OMe, SMe, SO₂Me, SO₂NMe₂, Formyl, COMe, CONHMe, CONMe₂, CONHCH₂CH=CH_{2,} NHCOMe, NHCOCF_{3,} N(C₂H₅)COMe, N(CH₃)COCH₃, NHCO₂Me, NHCO₂Et, [(1-Methylcyclopropyl)carbonyl]amino, CH₂CO*tert*Bu, Methyl oder 2,5-Dioxoimidazolidin-4-yl,
wobei die übrigen Substituenten eine oder mehrere der oben genannten Bedeutungen haben,
sowie die agrochemisch wirksamen Salze davon.

Weiterhin bevorzugt werden Verbindungen der Formel (I), in denen ein oder mehrere der Symbole eine der folgenden Bedeutungen haben:
- X¹: ist CR³ ,
- X²: ist CR⁴,
- R⁴: ist Wasserstoff, Chlor, Cyano, OMe, OCF₃, OCONHMe, SMe, Formyl, COMe, CONHMe, NHCOMe, NHCO*tert*Bu, NHCOC(CH₃)₂CH₂F, NHCO(C=CH₂)CH₃, NHCO₂Me, NHCO₂Et, CH₂CN, Methyl oder Piperidin-1-ylsulfonyl,
wobei die übrigen Substituenten eine oder mehrere der oben genannten Bedeutungen haben,
sowie die agrochemisch wirksamen Salze davon.

Weiterhin bevorzugt werden Verbindungen der Formel (I), in denen ein oder mehrere der Symbole eine der folgenden Bedeutungen haben:
- R⁶: ist Wasserstoff, CHO oder COCH₃,
- R⁷: ist Wasserstoff,
- R⁹: ist Wasserstoff, Methyl oder Ethyl,
wobei die übrigen Substituenten eine oder mehrere der oben genannten Bedeutungen haben,
sowie die agrochemisch wirksamen Salze davon.

Weiterhin bevorzugt werden Verbindungen der Formel (I), in denen ein oder mehrere der Symbole eine der folgenden Bedeutungen haben:
- R¹: ist Wasserstoff,
- R⁵: ist Wasserstoff,
wobei die übrigen Substituenten eine oder mehrere der oben genannten Bedeutungen haben,
sowie die agrochemisch wirksamen Salze davon.

Weiterhin bevorzugt werden Verbindungen der Formel (I), in denen
- R⁶: Wasserstoff ist,
wobei die übrigen Substituenten eine oder mehrere der oben genannten Bedeutungen haben,
sowie die agrochemisch wirksamen Salze davon.

Weiterhin bevorzugt werden Verbindungen der Formel (I), in denen ein oder mehrere der Symbole eine der folgenden Bedeutungen haben:
- R¹: ist Wasserstoff"
- R⁵: ist Wasserstoff,
- X¹: ist CR³,
- X²: ist CR⁴,
wobei die übrigen Substituenten eine oder mehrere der oben genannten Bedeutungen haben,
sowie die agrochemisch wirksamen Salze davon.

Weiterhin bevorzugt werden Verbindungen der Formel (I), in denen
- R⁷: Wasserstoff ist,
wobei die übrigen Substituenten eine oder mehrere der oben genannten Bedeutungen haben,
sowie die agrochemisch wirksamen Salze davon.

Weiterhin bevorzugt werden Verbindungen der Formel (I), in denen ein oder mehrere der Symbole eine der folgenden Bedeutungen haben:
- R⁸: ist Chlor, Brom oder CF₃,
wobei die übrigen Substituenten eine oder mehrere der oben genannten Bedeutungen haben,
sowie die agrochemisch wirksamen Salze davon.

Weiterhin bevorzugt werden Verbindungen der Formel (I), in denen
- R⁹: ist Wasserstoff oder Methyl,
wobei die übrigen Substituenten eine oder mehrere der oben genannten Bedeutungen haben,
sowie die agrochemisch wirksamen Salze davon.

Weiterhin bevorzugt werden Verbindungen der Formel (I), in denen
- R¹⁰: für 2,2-Difluorethyl, 1,1,1-Trifluorpropan-2-yl, 2,2,2-Trifluorethyl, 4,4,4-Trifluorbutan-2-yl, 2-Fluorethyl, 2,2,3,3-Tetrafluorpropan-1-yl, 3,3,3-Trifluorpropan-1yl, 2,2-Difluorpropan-1-yl oder 1,1,1-Trifluor-4-methyl-pentan-2-yl steht,
wobei die übrigen Substituenten eine oder mehrere der oben genannten Bedeutungen haben,
sowie die agrochemisch wirksamen Salze davon.

Weiterhin bevorzugt werden Verbindungen der Formel (I), in denen
- R¹, R⁵, R⁶ und R⁷: für Wasserstoff stehen,
wobei die übrigen Substituenten eine oder mehrere der oben genannten Bedeutungen haben,
sowie die agrochemisch wirksamen Salze davon.

Die zuvor genannten Reste-Definitionen können untereinander in beliebiger Weise kombiniert werden. Außerdem können einzelne Definitionen entfallen.

Beispiele für anorganische Säuren sind Halogenwasserstoffsäuren wie Fluorwasserstoff, Chlorwasserstoff, Bromwasserstoff und Iodwasserstoff, Schwefelsäure, Phosphorsäure und Salpetersäure und saure Salze wie NaHSO₄ und KHSO₄. Als organische Säuren kommen beispielsweise Ameisensäure, Kohlensäure und Alkansäuren wie Essigsäure, Trifluoressigsäure, Trichloressigsäure und Propionsäure sowie Glycolsäure, Thiocyansäure, Milchsäure, Bernsteinsäure, Zitronensäure, Benzoesäure, Zimtsäure, Oxal-säure, Alkylsulfonsäuren (Sulfonsäuren mit geradkettigen oder verzweigten Alkylresten mit 1 bis 20 Kohlenstoffatomen), Arylsulfonsäuren oder -disulfonsäuren (aromatische Reste wie Phenyl und Naphthyl welche ein oder zwei Sulfonsäuregruppen tragen), Alkylphosphon-säuren (Phosphonsäuren mit geradkettigen oder verzweigten Alkylresten mit 1 bis 20 Kohlenstoffatomen), Arylphosphonsäuren oder - diphosphonsäuren (aromatische Reste wie Phenyl und Naphthyl welche ein oder zwei Phosphonsäurereste tragen), wobei die Alkyl- bzw. Arylreste weitere Substituenten tragen können, z.B. p-Toluolsulfonsäure, Salicylsäure, p-Aminosalicylsäure, 2-Phenoxybenzoesäure, 2-Acetoxybenzoesäure etc..

Als Metallionen kommen insbesondere die Ionen der Elemente der zweiten Hauptgruppe, insbesondere Calzium und Magnesium, der dritten und vierten Hauptgruppe, insbesondere Aluminium, Zinn und Blei, sowie der ersten bis achten Nebengruppe, insbesondere Chrom, Mangan, Eisen, Kobalt, Nickel, Kupfer, Zink und andere in Betracht. Besonders bevorzugt sind die Metallionen der Elemente der vierten Periode. Die Metalle können dabei in den verschiedenen ihnen zukommenden Wertigkeiten vorliegen.

Gegebenenfalls substituierte Gruppen können einfach oder mehrfach substituiert sein, wobei bei Mehrfachsubstitutionen die Substituenten gleich oder verschieden sein können.

Bei den in den vorstehenden Formeln angegebenen Definitionen der Symbole wurden Sammelbegriffe verwendet, die allgemein repräsentativ für die folgenden Substituenten stehen:
**Halogen:** Fluor, Chlor, Brom und Jod;
**Aryl:** unsubstituiertes oder gegebenenfalls substituiertes, 5 bis 15-gliedriges, teilweise oder vollständig ungesättigtes mono-, bi- oder tricyclisches Ringsystem, mit bis zu 3 Ringgliedern, ausgewählt aus den Gruppen C(=O), (C=S), wobei mindestens einer der Ringe des Ringsystems vollständig ungesättigt ist, wie beispielsweise (aber nicht beschränkt auf) Benzol, Naphthalin, Tetrahydronaphthalin, Anthracen, Indan, Phenanthren, Azulen.
**Alkyl:** gesättigte, geradkettige oder verzweigte Kohlenwasserstoffreste mit 1 bis 10 Kohlenstoffatomen, wie beispielsweise (aber nicht beschränkt auf) Methyl, Ethyl, Propyl, 1-Methylethyl, Butyl, 1-Methyl-propyl, 2-Methylpropyl, 1,1-Dimethylethyl, Pentyl, 1-Methylbutyl, 2-Methylbutyl, 3-Methylbutyl, 2,2-Di-methylpropyl, 1-Ethylpropyl, Hexyl, 1,1-Dimethylpropyl, 1,2-Dimethylpropyl,1-Methylpentyl, 2-Methylpentyl, 3-Methylpentyl, 4-Methylpentyl, 1,1-Dimethylbutyl, 1,2-Dimethylbutyl, 1,3-Dimethylbutyl, 2,2-Dimethylbutyl, 2,3-Dimethylbutyl, 3,3-Dimethylbutyl, 1-Ethylbutyl, 2-Ethylbutyl, 1,1,2-Trimethylpropyl, 1,2,2-Trimethylpropyl, 1-Ethyl-1-methylpropyl und 1-Ethyl-2-methylpropyl, Heptyl, 1-Methylhexyl, Octyl, 1,1-Dimethylhexyl, 2-Ethylhexyl,1-Ethylhexyl, Nonyl, 1,2,2-Trimethylhexyl, Decyl.
**Haloalkyl:** geradkettige oder verzweigte Alkylgruppen mit 1 bis 4 Kohlenstoffatomen (wie vorstehend genannt), wobei in diesen Gruppen teilweise oder vollständig die Wasserstoffatome durch Halogenatome wie vorstehend genannt ersetzt sein können, so z.B. (aber nicht beschränkt auf) C₁-C₂-Halogenalkyl wie Chlormethyl, Brommethyl, Dichlormethyl, Trichlormethyl, Fluormethyl, Difluormethyl, Trifluormethyl, Chlorfluormethyl, Dichlorfluormethyl, Chlordifluormethyl, 1-Chlorethyl, 1-Bromethyl, 1-Fluorethyl, 2-Fluorethyl, 2,2-Difluorethyl, 2,2,2-Trifluorethyl, 2-Chlor-2-fluorethyl, 2-Chlor,2-difluorethyl, 2,2-Dichlor-2-fluorethyl, 2,2,2-Trichlorethyl, Pentafluorethyl und 1,1,1-Trifluorprop-2-yl;
**Alkenyl:** ungesättigte, geradkettige oder verzweigte Kohlenwasserstoffreste mit 2 bis 16 Kohlenstoffatomen und mindestens einer Doppelbindung in einer beliebigen Position, wie beispielsweise (aber nicht beschränkt auf) C₂-C₆-Alkenyl wie Ethenyl, 1-Propenyl, 2-Propenyl, 1-Methylethenyl, 1-Butenyl, 2-Butenyl, 3-Butenyl, 1-Methyl-1-propenyl, 2-Methyl-1-propenyl, 1-Methyl-2-propenyl, 2-Methyl-2-propenyl, 1-Pentenyl, 2-Pentenyl, 3-Pentenyl, 4-Pentenyl, 1-Methyl-1-butenyl, 2-Methyl-1-butenyl, 3-Methyl-1-butenyl, 1-Methyl-2-butenyl, 2-Methyl-2-butenyl, 3-Methyl-2-butenyl, 1-Methyl-3-butenyl, 2-Methyl-3-butenyl, 3-Methyl-3-butenyl, 1,1-Dimethyl-2-propenyl, 1,2-Dimethyl-1-propenyl, 1,2-Dimethyl-2-propenyl, 1-Ethyl-1-propenyl, 1-Ethyl-2-propenyl, 1-Hexenyl, 2-Hexenyl, 3-Hexenyl, 4-Hexenyl, 5-Hexenyl, 1-Methyl-1-pentenyl, 2-Methyl-1-pentenyl, 3-Methyl-1-pentenyl, 4-Methyl-1-pentenyl, 1-Methyl-2-pentenyl, 2-Methyl-2-pentenyl, 3-Methyl-2-pentenyl, 4-Methyl-2-pentenyl, 1-Methyl-3-pentenyl, 2-Methyl-3-pentenyl, 3-Methyl-3-pentenyl, 4-Methyl-3-pentenyl, 1-Methyl-4-pentenyl, 2-Methyl-4-pentenyl, 3-Methyl-4-pentenyl, 4-Methyl-4-pentenyl, 1,1-Dimethyl-2-butenyl, 1,1,-Dimethyl-3-butenyl, 1,2-Dimethyl-1-butenyl, 1,2-Dimethyl-2-butenyl, 1,2-Dimethyl-3-butenyl, 1,3-Dimethyl-1-butenyl, 1,3-Dimethyl-2-butenyl, 1,3-Dimethyl-3-butenyl, 2,2-Dimethyl-3-butenyl, 2,3-Dimethyl-1-butenyl, 2,3-Dimethyl-2-butenyl, 2,3-Dimethyl-3-butenyl, 3,3-Dimethyl-1-butenyl, 3,3-Dimethyl-2-butenyl, 1-Ethyl-1-butenyl, 1-Ethyl-2-butenyl, 1-Ethyl-3-butenyl, 2-Ethyl-1-butenyl, 2-Ethyl-2-butenyl, 2-Ethyl-3-butenyl, 1,1,2-Trimethyl-2-propenyl, 1-Ethyl-1-methyl-2-propenyl, 1-Ethyl-2-methyl-1-propenyl und 1-Ethyl-2-methyl-2-propenyl;
**Alkinyl:** geradkettige oder verzweigte Kohlenwasserstoffgruppen mit 2 bis 16 Kohlenstoffatomen und mindestens einer Dreifachbindung in einer beliebigen Position, wie beispielsweise (aber nicht beschränkt auf) C₂-C₆-Alkinyl wie Ethinyl, 1-Propinyl, 2-Propinyl, 1-Butinyl, 2-Butinyl, 3-Butinyl, 1-Methyl-2-propinyl, 1-Pentinyl, 2-Pentinyl, 3-Pentinyl, 4-Pentinyl, 1-Methyl-2-butinyl, 1-Methyl-3-butinyl, 2-Methyl-3-butinyl, 3-Methyl-1-butinyl, 1,1-Dimethyl-2-propinyl, 1-Ethyl-2-propinyl, 1-Hexinyl, 2-Hexinyl, 3-Hexinyl, 4-Hexinyl, 5-Hexinyl, 1-Methyl-2-pentinyl, 1-Methyl-3-pentinyl, 1-Methyl-4-pentinyl, 2-Methyl-3-pentinyl, 2-Methyl-4-pentinyl, 3-Methyl-1-pentinyl, 3-Methyl-4-pentinyl, 4-Methyl-1-pentinyl, 4-Methyl-2-pentinyl, 1,1-Dimethyl-2-butinyl, 1,1-Dimethyl-3-butinyl, 1,2-Dimethyl-3-butinyl, 2,2-Dimethyl-3-butinyl, 3,3-Dimethyl-1-butinyl, 1-Ethyl-2-butinyl, 1-Ethyl-3-butinyl, 2-Ethyl-3-butinyl und 1-Ethyl-1-methyl-2-propinyl;
**Alkoxy:** gesättigte, geradkettige oder verzweigte Alkoxyreste mit 1 bis 4 Kohlenstoffatomen, wie beispielsweise (aber nicht beschränkt auf) C₁-C₄-Alkoxy wie Methoxy, Ethoxy, Propoxy, 1-Methylethoxy, Butoxy, 1-Methyl-propoxy, 2-Methylpropoxy, 1,1-Dimethylethoxy;
**Haloalkoxy:** geradkettige oder verzweigte Alkoxygruppen mit 1 bis 4 Kohlenstoffatomen (wie vorstehend genannt), wobei in diesen Gruppen teilweise oder vollständig die Wasserstoffatome durch Halogenatome wie vorstehend genannt ersetzt sein können, wie beispielsweise (aber nicht beschränkt auf) C₁-C₂-Halogenalkoxy wie Chlormethoxy, Brommethoxy, Dichlormethoxy, Trichlormethoxy, Fluormethoxy, Difluormethoxy, Trifluormethoxy, Chlorfluormethoxy, Dichlorfluormethoxy, Chlordifluormethoxy, 1-Chlorethoxy, 1-Bromethoxy, 1-Fluorethoxy, 2-Fluorethoxy, 2,2-Difluorethoxy, 2,2,2-Trifluorethoxy, 2-Chlor-2-fluorethoxy, 2-Chlor,2-difluorethoxy, 2,2-Dichlor-2-fluorethoxy, 2,2,2-Trichlorethoxy, Pentafluor-ethoxy und 1,1,1-Trifluorprop-2-oxy;
**Thioalkyl:** gesättigte, geradkettige oder verzweigte Alkylthioreste mit 1 bis 6 Kohlenstoffatomen, wie beispielsweise (aber nicht beschränkt auf) C₁-C₆-Alkylthio wie Methylthio, Ethylthio, Propylthio, 1-Methylethylthio, Butylthio, 1-Methyl-propylthio, 2-Methylpropylthio, 1,1-Dimethylethylthio, Pentylthio, 1-Methylbutylthio, 2-Methylbutylthio, 3-Methylbutylthio, 2,2-Dimethylpropylthio, 1-Ethylpropylthio, Hexylthio, 1,1-Dimethylpropylthio, 1,2-Dimethylpropylthio,1-Methylpentylthio, 2-Methylpentylthio, 3-Methyl-pentylthio, 4-Methylpentylthio, 1,1-Dimethylbutylthio, 1,2-Dimethylbutylthio, 1,3-Dimethyl-butylthio, 2,2-Dimethylbutylthio, 2,3-Dimethylbutylthio, 3,3-Dimethylbutylthio, 1-Ethylbutylthio, 2-Ethylbutylthio, 1,1,2-Trimethylpropylthio, 1,2,2-Trimethylpropylthio, 1-Ethyl-1-methylpropyl-thio und 1-Ethyl-2-methylpropylthio;
**Thiohaloalkyl:** geradkettige oder verzweigte Alkylthiogruppen mit 1 bis 6 Kohlenstoffatomen (wie vorstehend genannt), wobei in diesen Gruppen teilweise oder vollständig die Wasserstoffatome durch Halogenatome wie vorstehend genannt ersetzt sein können, wie beispielsweise (aber nicht beschränkt auf) C₁-C₂-Halogenalkylthio wie Chlormethylthio, Brommethylthio, Dichlormethylthio, Trichlormethylthio, Fluormethylthio, Difluormethylthio, Trifluormethylthio, Chlorfluormethylthio, Dichlorfluor-methylthio, Chlordifluormethylthio, 1-Chlorethylthio, 1-Bromethylthio, 1-Fluorethylthio, 2-Fluorethylthio, 2,2-Difluorethylthio, 2,2,2-Trifluorethylthio, 2-Chlor-2-fluorethylthio, 2-Chlor,2-difluorethylthio, 2,2-Dichlor-2-fluorethylthio, 2,2,2-Trichlorethylthio, Pentafluorethylthio und 1,1,1-Trifluorprop-2-ylthio;
**Cycloalkyl:** mono-, bi- oder tricyclische, gesättigte Kohlenwasserstoffgruppen mit 3 bis 12 Kohlenstoffringgliedern, wie z.B. (aber nicht beschränkt auf) Cyclopropyl, Cyclobutyl, Cyclopentyl und Cyclohexyl, Bicyclo[1,0,1]butan, Decalinyl Norbornyl;
**Cylcoalkenyl:** mono-, bi- oder tricyclische, nicht aromatische Kohlenwasserstoffgruppen mit 5 bis 15 Kohlenstoffringgliedern mit mindestens einer Doppelbindung, wie beispielsweise (aber nicht beschränkt auf) Cyclopenten-1-yl, Cyclohexen-1-yl, Cyclohepta-1,3-dien-1-yl, Norbornen-1-yl;
**(Alkoxy)carbonyl:** eine Alkoxygruppe mit 1 bis 4 Kohlenstoffatomen (wie vorstehend genannt), welche über eine Carbonylgruppe (-CO-) an das Gerüst gebunden ist;
**Heterocyclyl: drei- bis fünfzehngliedriger gesättigter oder partiell ungesättigter Heterocyclus, enthaltend ein bis vier Heteroatome aus der Gruppe Sauerstoff, Stickstoff oder Schwefel:** mono-, bi- oder tricyclische Heterocyclen enthaltend neben Kohlenstoffringgliedern ein bis drei Stickstoffatome und/oder ein Sauerstoff- oder Schwefelatom oder ein oder zwei Sauerstoff- und/oder Schwefelatome; enthält der Ring mehrere Sauerstoffatome, so stehen diese nicht direkt benachbart; wie beispielsweise (aber nicht beschränkt auf) Oxiranyl, Aziridinyl, 2-Tetrahydrofuranyl, 3-Tetrahydrofuranyl, 2-Tetrahydrothienyl, 3-Tetrahydrothienyl, 2-Pyrrolidinyl, 3-Pyrrolidinyl, 3-Isoxazolidinyl, 4-Isoxazolidinyl, 5-Isoxazolidinyl, 3-Isothiazolidinyl, 4-Isothiazolidinyl, 5-Isothiazolidinyl, 3-Pyrazolidinyl, 4-Pyrazolidinyl, 5-Pyrazolidinyl, 2-Oxazolidinyl, 4-Oxazolidinyl, 5-Oxazolidinyl, 2-Thiazolidinyl, 4-thiazolidinyl, 5-Thiazolidinyl, 2-Imidazolidinyl, 4-Imidazolidinyl, 1,2,4-Oxadiazolidin-3-yl, 1,2,4-Oxadiazolidin-5-yl, 1,2,4-Thiadiazolidin-3-yl, 1,2,4-Thiadiazolidin-5-yl, 1,2,4-Triazolidin-3-yl, 1,3,4-Oxadiazolidin-2-yl, 1,3,4-Thiadiazolidin-2-yl, 1,3,4-Triazolidin-2-yl, 2,3-Dihydrofur-2-yl, 2,3-Dihydrofur-3-yl, 2,4-Dihydrofur-2-yl, 2,4-Dihydrofur-3-yl, 2,3-Dihydrothien-2-yl, 2,3-Dihydrothien-3-yl, 2,4-Dihydrothien-2-yl, 2,4-Dihydrothien-3-yl, 2-Pyrrolin-2-yl, 2-Pyrrolin-3-yl, 3-Pyrrolin-2-yl, 3-Pyrrolin-3-yl, 2-Isoxazolin-3-yl, 3-Isoxazolin-3-yl, 4-Isoxazolin-3-yl, 2-Isoxazolin-4-yl, 3-Isoxazolin-4-yl, 4-Isoxazolin-4-yl, 2-Isoxazolin-5-yl, 3-Isoxazolin-5-yl, 4-Isoxazolin-5-yl, 2-Isothiazolin-3-yl, 3-Isothiazolin-3-yl, 4-Isothiazolin-3-yl, 2-Isothiazolin-4-yl, 3-Isothiazolin-4-yl, 4-Isothiazolin-4-yl, 2-Isothiazolin-5-yl, 3-Isothiazolin-5-yl, 4-Isothiazolin-5-yl, 2,3-Dihydropyrazol-1-yl, 2,3-Dihydropyrazol-2-yl, 2,3-Dihydropyrazol-3-yl, 2,3-Dihydropyrazol-4-yl, 2,3-Dihydropyrazol-5-yl, 3,4-Dihydropyrazol-1-yl, 3,4-Dihydropyrazol-3-yl, 3,4-Dihydropyrazol-4-yl, 3,4-Dihydropyrazol-5-yl, 4,5-Dihydroopyrazol-1-yl, 4,5-Dihydropyrazol-3-yl, 4,5-Dihydropyrazol-4-yl, 4,5-Dihydropyrazol-5-yl, 2,3-Dihydrooxazol-2-yl, 2,3-Dihydrooxazol-3-yl, 2,3-Dihydrooxazol-4-yl, 2,3-Dihydrooxazol-5-yl, 3,4-Dihydrooxazol-2-yl, 3,4-Dihydrooxazol-3-yl, 3,4-Dihydrooxazol-4-yl, 3,4-Dihydrooxazol-5-yl, 3,4-Dihydrooxazol-2-yl, 3,4-Dihydrooxazol-3-yl, 3,4-Dihydrooxazol-4-yl, 2-Piperidinyl, 3-Piperidinyl, 4-Piperidinyl, 1,3-Dioxan-5-yl, 2-Tetrahydropyranyl, 4-Tetrahydropyranyl, 2-Tetrahydrothienyl, 3-Hexahydro-pyridazinyl, 4-Hexahydropyridazinyl, 2-Hexahydropyrimidinyl, 4-Hexahydropyrimidinyl, 5-Hexahydropyrimidinyl, 2-Piperazinyl, 1,3,5-Hexahydro-triazin-2-yl und 1,2,4-Hexahydrotriazin-3-yl;
**Hetaryl:** unsubstituiertes oder gegebenenfalls substituiertes, 5 bis 15-gliedriges, teilweise oder vollständig ungesättigtes mono-, bi- oder tricyclisches Ringsystem, wobei mindestens einer der Ringe des Ringsystems vollständig ungesättigt ist, **enthaltend ein bis vier Heteroatome aus der Gruppe Sauerstoff, Stickstoff oder Schwefel,** enthält der Ring mehrere Sauerstoffatome, so stehen diese nicht direkt benachbart;
wie beispielsweise (aber nicht beschränkt auf)
- **5-gliedriges Heteroaryl, enthaltend ein bis vier Stickstoffatome oder ein bis drei Stickstoffatome und ein Schwefel- oder Sauerstoffatom:** 5-Ring Heteroarylgruppen, welche neben Kohlenstoffatomen ein bis vier Stickstoffatome oder ein bis drei Stickstoffatome und ein Schwefel- oder Sauerstoffatom als Ringglieder enthalten können, z.B. 2-Furyl, 3-Furyl, 2-Thienyl, 3-Thienyl, 2-Pyrrolyl, 3-Pyrrolyl, 3-Isoxazolyl, 4-Isoxazolyl, 5-Isoxazolyl, 3-Isothiazolyl, 4-Isothiazolyl, 5-Isothiazolyl, 3-Pyrazolyl, 4-Pyrazolyl, 5-Pyrazolyl, 2-Oxazolyl, 4-Oxazolyl, 5- Oxazolyl, 2-Thiazolyl, 4-Thiazolyl, 5-Thiazolyl, 2-Imidazolyl, 4-Imidazolyl, 1,2,4-Oxadiazol-3-yl, 1,2,4-Oxadiazol-5-yl, 1,2,4-Thiadiazol-3-yl, 1,2,4-Thiadiazol-5-yl, 1,2,4-Triazol-3-yl, 1,3,4-Oxadiazol-2-yl, 1,3,4-Thiadiazol-2-yl und 1,3,4-Triazol-2-yl;
- **benzokondensiertes 5-gliedriges Heteroaryl, enthaltend ein bis drei Stickstoffatome oder ein Stickstoffatom und ein Sauerstoff- oder Schwefelatom:** 5-Ring Heteroarylgruppen, welche neben Kohlenstoffatomen ein bis vier Stickstoffatome oder ein bis drei Stickstoffatome und ein Schwefel- oder Sauerstoffatom als Ringglieder enthalten können, und in welchen zwei benachbarte Kohlenstoffringglieder oder ein Stickstoff- und ein benachbartes Kohlenstoffringglied durch eine Buta-1,3-dien-1,4-diylgruppe verbrückt sein können, in der ein oder zwei C-Atome durch N-Atome ersetzt sein können; z.B. Benzindolyl, Benzimidazolyl, Benzothiazolyl, Benzopyrazolyl, Benzofuryl,;
- **über Stickstoff gebundenes 5-gliedriges Heteroaryl, enthaltend ein bis vier Stickstoffatome, oder über Stickstoff gebundenes benzokondensiertes 5-gliederiges Heteroaryl, enthaltend ein bis drei Stickstoffatome:** 5-Ring Heteroarylgruppen, welche neben Kohlenstoffatomen ein bis vier Stickstoffatome bzw. ein bis drei Stickstoffatome als Ringglieder enthalten können, und in welchen zwei benachbarte Kohlenstoffringglieder oder ein Stickstoff- und ein benachbartes Kohlenstoffringglied durch eine Buta-1,3-dien-1,4-diylgruppe verbrückt sein können, in der ein oder zwei C-Atome durch N-Atome ersetzt sein können, in der ein oder zwei C-Atome durch N-Atome ersetzt sein können, wobei diese Ringe über eines der Stickstoffringglieder an das Gerüst gebunden sind, z.B. 1-Pyrrolyl, 1-Pyrazolyl, 1,2,4-Triazol-1-yl, 1-Imidazolyl, 1,2,3-Triazol-1-yl, 1,3,4-Triazol-1-yl;
- **6-gliedriges Heteroaryl, enthaltend ein bis drei bzw. ein bis vier Stickstoffatome:** 6-Ring Heteroarylgruppen, welche neben Kohlenstoffatomen ein bis drei bzw. ein bis vier Stickstoffatome als Ringglieder enthalten können, beispielsweise 2-Pyridinyl, 3-Pyridinyl, 4-Pyridinyl, 3-Pyridazinyl, 4-Pyridazinyl, 2-Pyrimidinyl, 4-Pyrimidinyl, 5-Pyrimidinyl, 2-Pyrazinyl, 1,3,5-Triazin-2-yl und 1,2,4-Triazin-3-yl;

Nicht umfasst sind solche Kombinationen, die den Naturgesetzen widersprechen und die der Fachmann daher aufgrund seines Fachwissens ausgeschlossen hätte. Beispielsweise sind Ringstrukturen mit drei oder mehreren benachbarten O-Atomen ausgeschlossen.

Ein weiterer Gegenstand der vorliegenden Erfindung betrifft ein Verfahren zum Herstellen der erfindungsgemäßen Diaminopyrimidine der Formeln (**I**) umfassend wenigstens einen der folgenden Schritte (a) bis (e):
(a) Umsetzung von 2,4-Dihalopyrimidinen der Formel **(III)** mit Halogen-substituierten Aminen der Formel **(II)** zu Verbindungen der Formel **(V)** in Gegenwart einer Base gegebenenfalls in Gegenwart eines Lösungsmittels, gegebenenfalls in Gegenwart eines Katalysators gemäß dem nachfolgenden Reaktionsschema (Schema 1): 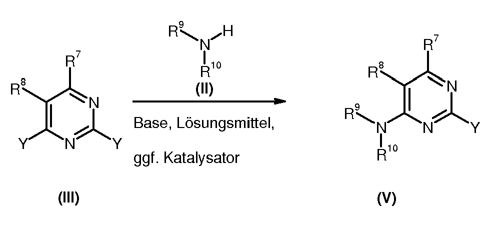 Mit Y = F, Cl, Br, I
(b) Umsetzung von Verbindungen der Formel **(V)** mit (het-)aromatischen Aminen der Formel **(IV)** gegebenenfalls in Gegenwart einer Säure, gegebenenfalls in Gegenwart eines Lösungsmittels gemäß dem nachfolgenden Reaktionsschema (Schema 2): 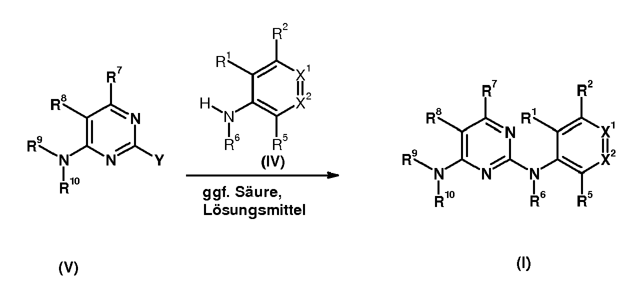 Mit Y = F, Cl, Br, I
(c) Umsetzung von Verbindungen der Formel **(VIb)** mit einem (het-)aromatischen Amin der Formel **(IV),** gegebenenfalls in Gegenwart einer Säure und in Gegenwart eines Lösungsmittels gemäß dem nachfolgenden Reaktionsschema (Schema 3): 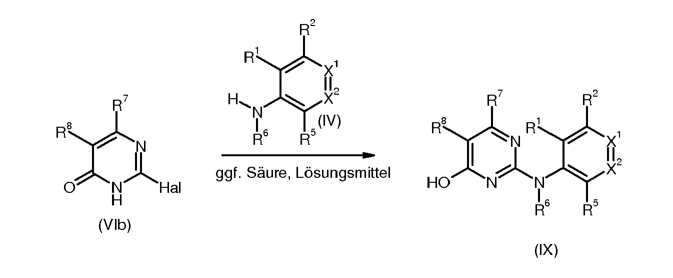 Mit Hal = F, Cl, Br, I
(d) Umsetzung von Verbindungen der Formel **(IX)** mit einem Halogenierungsmittel zu Verbindungen der Formel **(X),** gegebenenfalls in Gegenwart eines Lösungsmittels gemäß dem nachfolgenden Reaktionsschema (Schema 4): 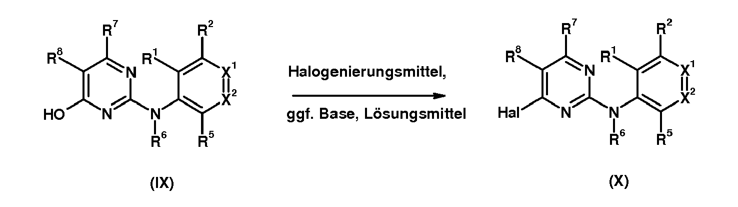
(e) Umsetzung von Verbindungen der Formel **(X)** mit Halogen-substituierten Aminen der Formel **(II)** zu Verbindungen der Formel **(I)** in Gegenwart einer Base gegebenenfalls in Gegenwart eines Lösungsmittels, gegebenenfalls in Gegenwart eines Katalysators gemäß dem nachfolgenden Reaktionsschema (Schema 5):

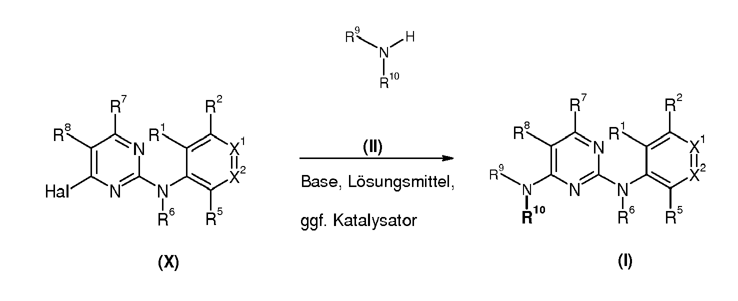

Wobei die Definitionen der Reste R¹ bis R¹⁰ und X¹ und X² in den obigen Schemata den oben angegebenen Definitionen entsprechen, und Y und Hal für F, Cl, Br, I steht.

Eine Möglichkeit zur Darstellung der Zwischenstufe **(V)** ist in Schema 1 gezeigt.

Die Halogen-substituierten Aminoverbindungen der Formel **(II)** sind entweder kommerziell verfügbar oder lassen sich nach Literaturvorschriften herstellen. Eine Methode zur Herstellung geeigneter Aminoverbindungen **(II)** ist beispielsweise die Reduktion entsprechender Carbonsäureamide (z.B. beschrieben in EP30092) bzw. entsprechender Oxime oder Azide (z.B. beschrieben in Chem. Ber. 1988, 119, 2233) oder Nitro-Verbindungen (z.B. beschrieben in J. Am. Chem Soc, 1953, 75, 5006) Eine weitere Möglichkeit besteht in der Behandlung entsprechender Aminocarbonsäuren mit SF₄ in HF (z.B. beschrieben in J. Org. Chem. 1962, 27, 1406). Die Ringöffnung substitiuerter Aziridine mittels HF ist in J. Org. Chem. 1981, 46, 4938 beschrieben. Weitere Methoden zur Herstellung von **(II)** beinhalten die Spaltung entsprechender Phthalimide nach Gabriel (z.B. beschrieben in DE 3429048), Amminolyse geeigneter Haloalkylhalogeniden (z.B. beschrieben in US2539406) oder den Abbau entsprechender Carbonsäureazide (z.B. beschrieben in DE3611195). Aminoaldehyde bzw. -ketone können mittels geeigneter Fluorierungsreagenzien (z.B. DAST) in die entsprechenden Difluoroalkylamine überführt werden (W02008008022), während Aminoalkohole die entsprechenden Monofluoralkylamine bilden (z.B. W02006029115). Analog können aus Aminoalkoholen mittels geeigneter Chlorierungs- und Bromierungsmittel Chlor- und Bromalkylamine gewonnen werden (.J. Org. Chem. 2005, 70, 7364, bzw. Org. Lett., 2004, 6, 1935).

Geeignete substituierte 2,4-Dihalopyrimidine **(III)** sind entweder kommerziell verfügbar oder lassen sich nach Literaturvorschriften, beispielsweise ausgehend von kommerziell verfügbaren substituierten Uracilen, herstellen (z.B. R⁸ = CN: J. Org. Chem. 1962, 27, 2264; J. Chem. Soc. 1955, 1834; Chem. Ber. 1909, 42, 734; R⁸ = CF₃: J. Fluorine Chem. 1996, 77, 93; siehe auch WO 2000/047539).

Zunächst wird unter Verwendung einer geeigneten Base bei einer Temperatur von -30°C bis +80°C in einem geeigneten Lösungmittel wie beispielsweise Dioxan, THF, Dimethylformamid oder Acetonitril ein Halogen-substituiertes Amin (**II**) mit einem 2,4-Dihalopyrimidin **(III)** über einen Zeitraum von 1-24 h zur Reaktion gebracht. Als Base können z.B. anorganische Salze wie NaHCO₃, Na₂CO₃ oder K₂CO₃, metallorganische Verbindungen wie LDA oder NaHMDS oder Aminbasen wie Ethyldiisopropylamin, DBU, DBN oder tri-n-Butylamin verwendet werden. Alternativ kann die Reaktion auch wie beispielsweise in Org. Lett. 2006, 8, 395 beschrieben, unter Zuhilfenahme eines geeigneten Übergangsmetallkatalysators wie zum Beispiel Palladium gemeinsam mit einem geeigneten Liganden wie beispielsweise Triphenylphosphin oder Xanthphos durchgeführt werden.

Die Verbindungen der Formel **(V)** sind teilweise neu und damit ebenfalls Gegenstand der vorliegenden Erfindung.

Neu sind Verbindungen der Formel **(Va),** in denen 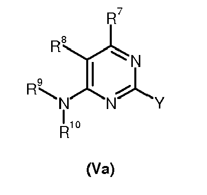
- R⁷: für Wasserstoff, C₂-C₃-Alkyl, Cyano oder C₁-C₃-Haloalkyl steht,
- R⁸: für Chlor, Brom, Iod, Cyano, SMe, SOMe, SO₂Me, CF₃, CCl₃, CFH₂ oder CF₂H steht,
Y = F, Cl, Br oder I ist
und
- R⁹, R¹⁰,: wie oben definiert die genannten allgemeinen, bevorzugten, besonders bevorzugten und ganz besonders bevorzugten Bedeutungen haben,
mit der Massgabe, dass wenn
- R⁸: = Brom ist,
- R¹⁰: nicht 2,2,3,3,3-Pentafluorpropan-1-yl oder 2,2,3,3,4,4,4-Heptafluorbutan-1-yl sein darf
oder
mit der Massgabe, dass wenn
- R⁸: = CN ist,
- R¹⁰: nicht 2,2,2 Trifluorethyl oder 2-Fluorethyl sein darf

Eine Möglichkeit zur Darstellung der Verbindung **(I)** ist in Schema 2 gezeigt.

Die substituierten (het-)aromatischen Amine **(IV)** sind entweder kommerziell verfügbar oder können durch literaturbekannte Methoden aus kommerziell verfügbaren Vorstufen hergestellt werden. (Het-)aromatische Amine, welche einen oder mehrere gleiche oder verschiedene Substituenten im (Het-)Aromatenteil tragen, lassen sich durch eine Vielzahl von Methoden herstellen, die in der einschlägigen Literatur beschrieben sind. Im Folgenden sind beispielhaft einige der Methoden genannt.

Die Darstellung von Sulfonamid- bzw. Sulfonester-substituierten Arylaminen gelingt beispielsweise durch literaturbekannte Umsetzung von kommerziell verfügbaren Aminosulfonsäuren mit Chlorierungsreagenzien (z.B. POCl₃) und anschliessender Umsetzung der gebildeten Sulfochloride mit O- bzw. N-Nukleophilen.

Zwei gängige Methoden zur Herstellung von N-monoacylierten Diaminoaromaten sind unten skizziert. So lassen sich beispielsweise Nitroaniline nach Standardmethoden mit Acylhalogeniden, Chlorkohlensäureestern oder Iso(thio)cyanaten zu den korrespondierenden N-Acylnitroaromaten umsetzen, welche sich dann nach literaturbekannten Vorschriften zu N-Acyl-Aminoaromaten reduzieren lassen. Eine weitere Methode beschreibt die Herstellung der genannten Verbindungen mittels übergangsmetallkatalysierter Kreuzkupplung von Aminohalogenaromaten und N-Acylverbindungen (siehe z.B. J. Am. Chem. Soc. 2001, 123, 7727).

### Synthese N-monoacylierter Diaminoaromaten

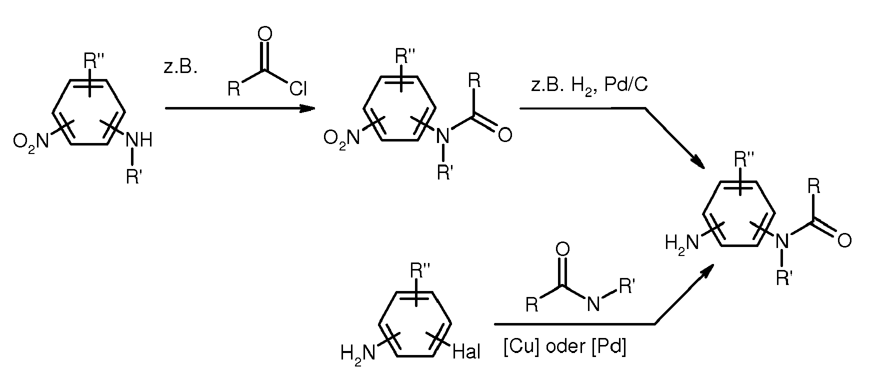

Cyclische, N-gebundene Reste R³ oder R⁴ lassen sich beispielsweise durch Kondensation von Nitroaminoaromaten mit Haloalkylcarbonylhalogeniden oder Diestern bzw. Diesteräquivalenten oder Lactonen darstellen; die anschliessende Reduktion der Nitrogruppe liefert das gewünschte aromatische Amin. Eine weitere Möglichkeit zur Synthese von N-gebundenen Resten R³ bis R⁴ ist die Kondensation von Nitroarylhydrazinen mit Diestern bzw. Diesteräquivalenten, Propargylsäureestern oder Ketoestern. Die Reduktion der Nitrogruppe ergibt das Anilin.

Die Darstellung substituierter 3- oder 4-Aminopyridine gelingt beispielsweise durch die Reduktion entsprechender substituierter Nitropyridine (z.B. W02006074147), mittels Hoffmann- oder Curtiusabbau entsprechender Pyridincarbonsäurederivate (z.B. z.B. Bioorg. Med. Chem. Lett. 2006, 16, 3484) oder Aminolyse substituierter Halogenpyridine (z.B. EP228846). Weitere Methoden bestehen in der literaturbekannten Umsetzung ensprechender Halogen-Aminopyridine mit O-, N- oder S-Nucleophilen (z.B. Bioorg. Med. Chem. Lett. 2002, 12, 1517) sowie C-Nucleophilen (z.B. Bioorg. Med. Chem. Lett. 2000, 10, 2383). Eine weitere Methode beschreibt die Herstellung der genannten Verbindungen mittels übergangsmetallkatalysierter Kreuzkupplung von Aminohalogenpyridinen (W02006025979).

Das Zwischenprodukt **(V)** wird in Gegenwart von Brönstedt-Säuren wie z.B. wasserfreie Salzsäure, Camphersulfonsäure oder p-Toluolsulfonsäure in einem geeigneten Lösungsmittel wie beispielsweise Dioxan, THF, DMSO, DME, 2-Methoxyethanol, n-Butanol oder Acetonitril bei einer Temperatur von 0°C-140°C über einen Zeitraum von 1-48 h mit einem (het-)aromatischen Amin **(IV)** zur Reaktion gebracht. Analog beschrieben z.B. in Bioorg. Med. Chem. Lett. 2006, 16, 2689; GB2002 A1-2369359, Org. Lett. 2005, 7, 4113).

Alternativ kann die Reaktion von **(V)** und **(IV)** zu **(I)** auch basenkatalysiert, also unter Verwendung von beispielsweise Carbonaten wie Kaliumcarbonat, Alkoholaten wie Kalium-*tert*.-Butylat oder Hydriden wie Natriumhydrid durchgeführt werden, wobei dabei auch die katalytische Verwendung eines Übergangsmetalls wie zum Beispiel Palladium gemeinsam mit einem geeigneten Liganden wie beispielsweise Xanthphos nützlich sein kann.

Schließlich besteht die Möglichkeit, die Reaktion von **(V)** und **(IV)** zu (**I**), in Abwesenheit von Lösungsmitteln und/oder Brönstedt-Säuren (beschrieben z.B. in Bioorg. Med. Chem. Lett. 2006, 16, 108; Bioorg. Med. Chem. Lett. 2005, 15, 3881) durchzuführen.

Eine Möglichkeit zur Darstellung von Verbindungen der Formel **(IX)** und auch **(IXa)** ist in Schema 3 gezeigt.

2-Halogen-substituierte Pyrimidin-4-one **(VIb)** sind aus 2,4-Dihalogen-substituierten Pyrimidinen durch regioselektive Hydrolyse zugänglich. Dies ist z.B. beschrieben in Russ. J. Org. Chem. 2006, 42, 580; J. Med. Chem.1965, 8, 253.

Zwischenprodukte der Formel **(VIb)** werden in Gegenwart von Brönstedt-Säuren wie z.B. wasserfreie Salzsäure, Camphersulfonsäure oder p-Toluolsulfonsäure in einem geeigneten Lösungsmittel wie beispielsweise Dioxan, THF, DMSO, DME, 2-Methoxyethanol, n-Butanol oder Acetonitril bei einer Temperatur von 0°C-140°C über einen Zeitraum von 1-48 h mit einem (het-) aromatischen Amin **(IV)** zur Reaktion gebracht.

Alternativ kann die Reaktion von **(VIb)** und **(IV)** zu **(IX)** auch basenkatalysiert, also unter Verwendung von beispielsweise Carbonaten wie Kaliumcarbonat, Alkoholaten wie Kalium-*tert*.Butylat oder Hydriden wie Natriumhydrid durchgeführt werden, wobei dabei auch die katalytische Verwendung eines Übergangsmetalls wie zum Beispiel Palladium gemeinsam mit einem geeigneten Liganden wie beispielsweise Xanthphos nützlich sein kann.

Schließlich besteht die Möglichkeit, die Reaktion von **(VIb)** und **(IV)** zu **(IX)** in Abwesenheit von Lösungsmitteln und/oder Brönstedt-Säuren (beschrieben z.B. in Bioorg. Med. Chem. Lett. 2006, 16, 108; Bioorg. Med. Chem. Lett. 2005, 15, 3881) durchzuführen.

Die Verbindungen der Formel **(IX)** sind teilweise neu und damit ebenfalls Gegenstand der vorliegenden Erfindung.

Neu sind Verbindungen der Formel **(IXa),** 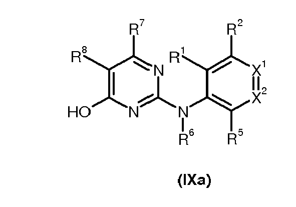 in welcher die Symbole folgende Bedeutungen haben:
- X¹, X², R¹ bis R⁶, R¹², R¹³ und R¹⁴: haben die oben angegebenen allgemeinen, bevorzugten, besonders bevorzugten, ganz besonders bevorzugten und insbesondere bevorzugten Bedeutungen,
- R⁷: ist Wasserstoff,
- R⁸: steht für Fluor, Chlor, Brom, Iod, CFH₂, CF₂H, CF₃, CCl₃, SMe, SOMe oder SO₂Me,
mit der Massgabe, dass wenn
- R¹ = R² = R⁵: = Wasserstoff sind und X² = CH ist,
- R³: nicht gleich Wasserstoff, CO₂H, (CH₂)₂OH, SMe, SOMe, oder Cyano sein darf
oder
mit der Massgabe, dass wenn
- R¹ = R⁵ =: Wasserstoff sind und X¹ = CH ist,
weder R² noch R⁴ = OH oder CONH₂ sein darf.

Eine Möglichkeit zur Darstellung von Verbindungen der Formel **(X)** und auch **(Xa)** ist in Schema 4 gezeigt.

Zwischenprodukte der Formel **(IX)** lassen sich durch Umsetzung mit geeigneten Halogenierungsmitteln wie beispielsweise Thionylchlorid, Phosphorpentoxid oder Phosphorylchlorid oder einer Mischung daraus gegebenenfalls in Anwesenheit eines geeigneten Lösungsmittels wie beispielsweise Toluol oder Ethanol und gegebenenfalls in Anwesenheit einer geeigneten Base wie beispielsweise Triethylamin in 2-Anilino-4-chlorpyrimidine der Formel **(X)** überführen. Analog beispielsweise beschrieben in J. Med. Chem. 1989, 32, 1667; J. Heterocycl. Chem. 1989, 26, 313.

Die Verbindungen der Formel **(X)** sind teilweise neu und damit ebenfalls Gegenstand der vorliegenden Erfindung.

Neu sind Verbindungen der Formel **(Xa),** 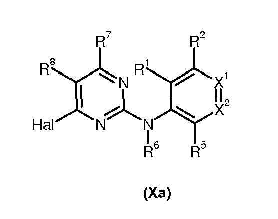 in welcher die Symbole folgende Bedeutungen haben:
- X¹, X², R² bis R⁴, R¹² R¹³ und R¹⁴: haben die oben angegebenen allgemeinen, bevorzugten, besonders bevorzugten, ganz besonders bevorzugten und insbesondere bevorzugten Bedeutungen und

Hal steht für Fluor, Chlor, Brom oder Iod,
- R⁸: steht für Fluor, Chlor, Brom, Iod, Me, CF₃, CFH₂, CF₂H, CCl₃ und Cyano,
- R¹, R⁵, R⁶ und R⁷: stehen für Wasserstoff
mit der Massgabe, dass wenn
- X²: = CH und X¹ = CR³ ist
- R³: nicht gleich CON(Me)-4-(N-Methyl-piperidinyl), N-Piperazinyl, CO-1-(4-Methyl-piperazinyl), N-Morpholinyl, SO₂Me, CONH₂, Me, OMe, COO-Benzyl, COOH, COCI, CN, NO₂, NMe₂ oder Cl ist,
oder
mit der Massgabe, dass wenn
- X¹ =: CH und X² = CR⁴ ist
- R² oder R⁴: nicht für CN, Cl oder 5-Oxazolyl steht,
oder
mit der Massgabe, dass wenn
- X¹: = CR³ und X² = CR⁴ ist
- R² und R³: bzw. R³ und R⁴ nicht Chlor sind,
oder
mit der Massgabe, dass wenn
- R⁸: = F oder CF₃ ist
- R³ und R⁴: nicht gemeinsam einen gesättigten oder teilweise ungesättigten Heterocyclus bilden.

Eine weitere Möglichkeit zur Darstellung der Verbindung **(I)** ist in Schema 5 gezeigt.

Zur Darstellung von Verbindungen der Formel **(I)** wird das Zwischenprodukt **(X)** in Gegenwart von Basen wie beispielsweise Carbonaten wie Kaliumcarbonat, Alkoholaten wie Kalium-*tert*.-Butylat oder Hydriden wie Natriumhydrid in einem geeigneten Lösungsmittel wie beispielsweise Dioxan, THF, DMSO, DME, 2-Methoxyethanol, n-Butanol oder Acetonitril bei einer Temperatur von 0°C-140°C über einen Zeitraum von 1-48 h mit mit Halogen-substituierten Aminen der Formel **(II)** zur Reaktion gebracht, wobei dabei auch die katalytische Verwendung eines Übergangsmetalls wie zum Beispiel Palladium gemeinsam mit einem geeigneten Liganden wie beispielsweise Triphenylphosphin oder Xanthphos nützlich sein kann.

Generell kann auch ein anderer Weg zur Herstellung der erfindungsgemäßen Verbindungen **(I)** gewählt werden, wie in Schema 6 gezeigt.

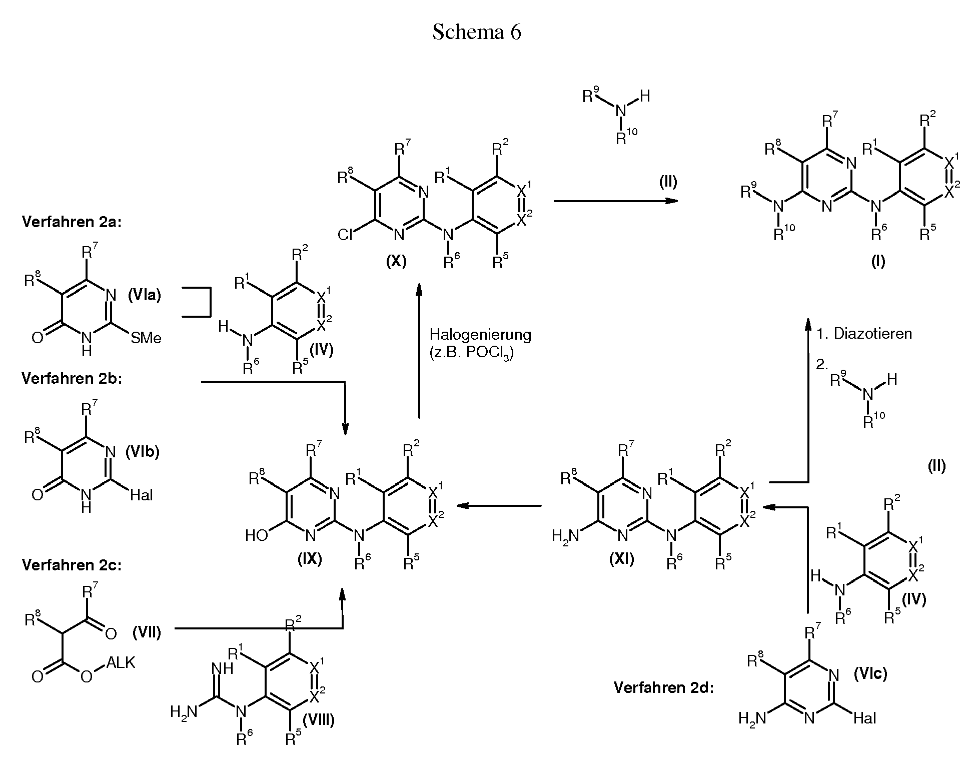

Eine weitere Methode zur Herstellung von Diaminopyrimidinen der Formel **(I)** ist in Schema 7 dargestellt:

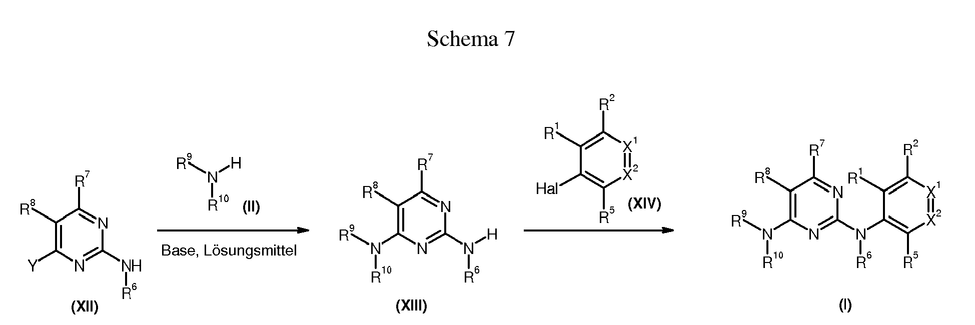

Ausgehend von 4-halogensubstituierten 2-Aminopyrimidinen **(XII),** die sich beispielsweise analog **(X)** aus Verbindungen des Typs **(VIa), (VIb)** oder **(VII)** durch Umsetzung mit R⁶-Aminen mit nachfolgender Chlorierung in 4-Position darstellen lassen, können nach Addition einer Aminoverbindung **(II)** bestimmte Diaminopyrimidine **(XIII)** erhalten werden. Diese können in einem übergangsmetall-katalysierten Folgeschritt mit einem Arylhalogenid **(XIV)** (wie beispielsweise in Org. Lett. 2002, 4, 3481 beschrieben) zur gewünschten Zielverbindung **(I)** umgesetzt werden.

Die erfindungsgemäßen Verfahren zur Herstellung der Verbindungen der Formel **(I)** werden vorzugsweise unter Verwendung eines oder mehrerer Reaktionshilfsmittel durchgeführt.

Als Reaktionshilfsmittel kommen gegebenenfalls die üblichen anorganischen oder organischen Basen oder Säureakzeptoren in Betracht. Hierzu gehören vorzugsweise Alkalimetall- oder Erdalkalimetall- -acetate, -amide, -carbonate, -hydrogencarbonate, -hydride, -hydroxide oder -alkanolate, wie beispielsweise Natrium-, Kalium- oder Calcium-acetat, Lithium-, Natrium-, Kalium- oder Calcium-amid, Natrium-, Kalium- oder Calcium-carbonat, Natrium-, Kalium- oder Calcium-hydrogencarbonat, Lithium-, Natrium-, Kalium- oder Calcium-hydrid, Lithium-, Natrium- - Kalium- oder Calcium-hydroxid, Natrium- oder Kalium- -methanolat, -ethanolat, -n- oder -i-propanolat, -n-, -i-, -s- oder -tButanolat; weiterhin auch basische organische Stickstoffverbindungen, wie beispielsweise Trimethylamin, Triethylamin, Tripropylamin, Tributylamin, Ethyldiisopropylamin, N,N-Dimethyl-cyclohexylamin, Dicyclohexylamin, Ethyl-dicyclohexylamin, N,N-Dimethyl-anilin, N,N-Dimethyl-benzylamin, Pyridin, 2-Methyl-, 3-Methyl-, 4-Methyl-, 2,4-Dimethyl-, 2,6-Dimethyl-, 3,4-Dimethyl-und 3,5-Dimethyl-pyridin, 5-Ethyl-2-methyl-pyridin, 4-Dimethylamino-pyridin, N-Methyl-piperidin, 1,4-Diazabicyclo[2,2,2]-octan (DABCO), 1,5-Diazabicyclo[4,3,0]-non-5-en (DBN), oder 1,8 Diazabicyclo[5,4,0]-undec-7-en (DBU).

Die erfindungsgemäßen Verfahren werden vorzugsweise unter Verwendung eines oder mehrerer Verdünnungsmittel durchgeführt. Als Verdünnungsmittel kommen praktisch alle inerten organischen Lösungsmittel in Frage. Hierzu gehören vorzugsweise aliphatische und aromatische, gegebenenfalls halogenierte Kohlenwasserstoffe wie Pentan, Hexan, Heptan, Cyclohexan, Petrolether, Benzin, Ligroin, Benzol, Toluol, Xylol, Methylenchlorid, Ethylenchlorid, Chloroform, Tetrachlorkohlenstoff, Chlorbenzol und o-Dichlorbenzol, Ether wie Diethyl-und Dibutylether, Glykoldimethylether und Diglykoldimethylether, Tetrahydrofuran und Dioxan, Ketone wie Aceton, Methylethyl-, Methyl-isopropyl-oder Methyl-isobutyl-keton, Ester wie Essigsäuremethylester oder - ethylester, Nitrile wie z.B. Acetonitril oder Propionitril, Amide wie z.B. Dimethylformamid, Dimethylacetamid und N-Methyl-pyrrolidon, sowie Dimethylsulfoxid, Tetramethylensulfon und Hexamethylphosphorsäuretriamid und DMPU.

Die Reaktionstemperaturen können bei den erfindungsgemäßen Verfahren in einem größeren Bereich variiert werden. Im allgemeinen arbeitet man bei Temperaturen zwischen 0°C und 250°C, vorzugsweise bei Temperaturen zwischen 10°C und 185°C.

Die erfindungsgemäßen Verfahren werden im allgemeinen unter Normaldruck durchgeführt. Es ist jedoch auch möglich, unter erhöhtem oder vermindertem Druck zu arbeiten.

Zur Durchführung der erfindungsgemäßen Verfahren werden die jeweils benötigten Ausgangsstoffe im allgemeinen in angenähert äquimolaren Mengen eingesetzt. Es ist jedoch auch möglich, eine der jeweils eingesetzten Komponenten in einem größeren Überschuss zu verwenden. Die Aufarbeitung erfolgt bei den erfindungsgemäßen Verfahren jeweils nach üblichen Methoden (vgl. die Herstellungsbeispiele).

Allgemein können Verbindungen der Formel **(I),** beispielsweise durch sequenzielle nukleophile Addition eines Halogen-substituierten, aliphatischen Amins **(II)** und eines (hetero-)aromatischen Amins (**IV**) an ein geeignetes substituiertes Pyrimidin **(III)** hergestellt werden, wie nachfolgend in Schema 8 skizziert ist:

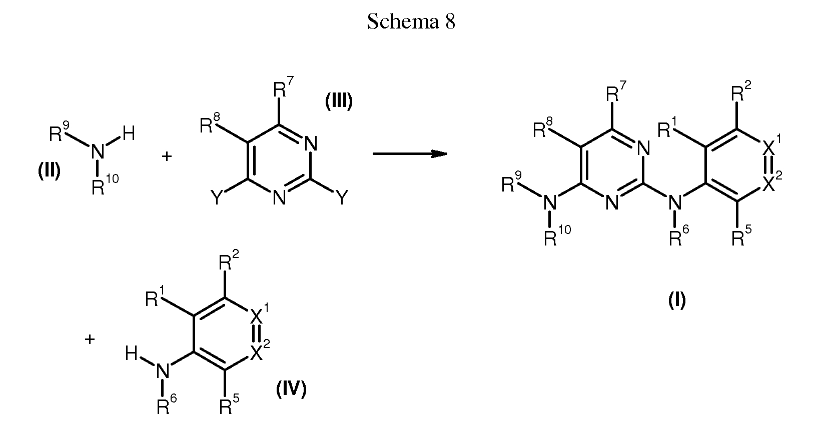

Dabei steht Y jeweils unabhängig voneinander stellvertretend für eine geeignete Fluchtgruppe, z.B. für ein Halogenatom (Hal = F, Cl, Br, I), SMe, SO₂Me, SOMe oder auch Triflat (CF₃SO₂O: bei Pyrimidinen bekannt aus WO05/095386).

Die Synthese von Diaminopyrimidinen der Formel **(I)** gemäß Schema 8 oder auch auf anderen Wegen ist in der Literatur vielfältig beschrieben (siehe dazu auch beispielsweise WO 06/021544, WO 07/072158, WO 07/003596, WO 05/016893, WO 05/013996, WO 04/056807, WO 04/014382, WO 03/030909)

Ein weiterer Gegenstand der Erfindung betrifft die nichtmedizinische Verwendung der erfindungsgemäßen Diaminopyrimidine zum Bekämpfen unerwünschter Mikroorganismen.

Ein weiterer Gegenstand der Erfindung betrifft ein Mittel zum Bekämpfen unerwünschter Mikroorganismen, umfassend wenigstens ein Diaminopyrimidin gemäß der vorliegenden Erfindung.

Außerdem betrifft die Erfindung ein Verfahren zum Bekämpfen unerwünschter Mikroorganismen, **dadurch gekennzeichnet, dass** die erfindungsgemäßen Diaminopyrimidine auf die Mikroorganismen und/oder in deren Lebensraum ausgebracht werden.

Die erfindungsgemäßen Stoffe weisen eine starke mikrobizide Wirkung auf und können zur Bekämpfung von unerwünschten Mikroorganismen, wie Fungi und Bakterien, im Pflanzenschutz und im Materialschutz eingesetzt werden.

Die erfindungsgemäßen Diaminopyrimidine der Formel **(I)** besitzen sehr gute fungizide Eigenschaften und lassen sich im Pflanzenschutz beispielsweise zur Bekämpfung von Plasmodiophoromyceten, Oomyceten, Chytridiomyceten, Zygomyceten, Ascomyceten, Basidiomyceten und Deuteromyceten einsetzen.

Bakterizide lassen sich im Pflanzenschutz beispielsweise zur Bekämpfung von Pseudomonadaceae, Rhizobiaceae, Enterobacteriaceae, Corynebacteriaceae und Streptomycetaceae einsetzen.

Die erfindungsgemäßen fungiziden Mittel können zur Bekämpfung von phytopathogenen Pilzen kurativ oder protektiv eingesetzt werden. Die Erfindung betrifft daher auch kurative und protektive Verfahren zum Bekämpfen von phytopathogenen Pilzen durch die Verwendung der erfindungsgemäßen Wirkstoffe oder Mittel, welche auf das Saatgut, die Pflanze oder Pflanzenteile, die Früchten oder den Boden, in welcher die Pflanzen wachsen, ausgebracht wird.

Die erfindungsgemäßen Mittel zum Bekämpfen von phytopathogenen Pilzen im Pflanzenschutz umfassen eine wirksame, aber nicht-phytotoxische Menge der erfindungsgemäßen Wirkstoffe. "Wirksame, aber nicht-phytotoxische Menge" bedeutet eine Menge des erfindungsgemäßen Mittels, die ausreichend ist, um die Pilzerkrankung der Pflanze ausreichend zu kontrollieren oder ganz abzutöten und die gleichzeitig keine nennenswerten Symptome von Phytotoxizität mit sich bringt. Diese Aufwandmenge kann im Allgemeinen in einem größeren Bereich variieren. Sie hängt von mehreren Faktoren ab, z.B. vom zu bekämpfenden Pilz, der Pflanze, den klimatischen Verhältnissen und den Inhaltsstoffen der erfindungsgemäßen Mittel.

Erfindungsgemäß können alle Pflanzen und Pflanzenteile behandelt werden. Unter Pflanzen werden hierbei alle Pflanzen und Pflanzenpopulationen verstanden, wie erwünschte und unerwünschte Wildpflanzen oder Kulturpflanzen (einschließlich natürlich vorkommender Kulturpflanzen). Kulturpflanzen können Pflanzen sein, die durch konventionelle Züchtungs- und Optimierungsmethoden oder durch biotechnologische und gentechnologische Methoden oder Kombinationen dieser Methoden erhalten werden können, einschließlich der transgenen Pflanzen und einschließlich der durch Sortenschutzrechte schützbaren oder nicht schützbaren Pflanzensorten. Unter Pflanzenteilen sollen alle oberirdischen und unterirdischen Teile und Organe der Pflanzen, wie Spross, Blatt, Blüte und Wurzel verstanden werden, wobei beispielhaft Blätter, Nadeln, Stängel, Stämme, Blüten, Fruchtkörper, Früchte und Samen sowie Wurzeln, Knollen und Rhizome aufgeführt werden. Zu den Pflanzenteilen gehört auch Erntegut sowie vegetatives und generatives Vermehrungsmaterial, beispielsweise Stecklinge, Knollen, Rhizome, Ableger und Samen.

Als Pflanzen, welche erfindungsgemäß behandelt werden können, seien folgende erwähnt: Baumwolle, Flachs, Weinrebe, Obst, Gemüse, wie *Rosaceae sp.* (beispielsweise Kernfrüchte wie Apfel und Birne, aber auch Steinfrüchte wie Aprikosen, Kirschen, Mandeln und Pfirsiche und Beerenfrüchte wie Erdbeeren), *Ribesioidae sp., Juglandaceae sp., Betulaceae sp., Anacardiaceae sp., Fagaceae sp., Moraceae sp*., *Oleaceae sp., Actinidaceae sp., Lauraceae sp*., *Musaceae sp.* (beispielsweise Bananenbäume und -plantagen), *Rubiaceae sp.* (beispielsweise Kaffee), *Theaceae sp., Sterculiceae sp., Rutaceae sp*. (beispielsweise Zitronen, Organen und Grapefruit); *Solanaceae sp*. (beispielsweise Tomaten), *Liliaceae sp., Asteraceae sp*. (beispielsweise Salat), *Umbelliferae sp., Cruciferae sp., Chenopodiaceae sp., Cucurbitaceae sp*. (beispielsweise Gurke), *Alliaceae sp.* (beispielsweise Lauch, Zwiebel), *Papilionaceae sp*. (beispielsweise Erbsen); Hauptnutzpflanzen, wie *Gramineae sp.* (beispielsweise Mais, Rasen, Getreide wie Weizen, Roggen, Reis, Gerste, Hafer, Hirse und Triticale), *Asteraceae sp*. (beispielsweise Sonnenblume), *Brassicaceae sp.* (beispielsweise Weißkohl, Rotkohl, Brokkoli, Blumenkohl, Rosenkohl, Pak Choi, Kohlrabi, Radieschen sowie Raps, Senf, Meerrettich und Kresse), *Fabacae sp*. (beispielsweise Bohne, Erdnüsse), *Papilionaceae sp*. (beispielsweise Sojabohne), *Solanaceae sp*. (beispielsweise Kartoffeln), *Chenopodiaceae sp*. (beispielsweise Zuckerrübe, Futterrübe, Mangold, Rote Rübe); Nutzpflanzen und Zierpflanzen in Garten und Wald; sowie jeweils genetisch modifizierte Arten dieser Pflanzen. Bevorzugt werden Getreidepflanzen erfindungsgemäß behandelt.

Beispielhaft, aber nicht begrenzend, seien einige Erreger von pilzlichen Erkrankungen, die erfindungsgemäß behandelt werden können, genannt:
Erkrankungen, hervorgerufen durch Erreger des Echten Mehltaus wie z.B. Blumeria-Arten, wie beispielsweise Blumeria graminis; Podosphaera-Arten, wie beispielsweise Podosphaera leucotricha; Sphaerotheca-Arten, wie beispielsweise Sphaerotheca fuliginea; Uncinula-Arten, wie beispielsweise Uncinula necator;
Erkrankungen, hervorgerufen durch Erreger von Rostkrankheiten wie z.B. Gymnosporangium-Arten, wie beispielsweise Gymnosporangium sabinae; Hemileia-Arten, wie beispielsweise Hemileia vastatrix; Phakopsora-Arten, wie beispielsweise Phakopsora pachyrhizi und Phakopsora meibomiae; Puccinia-Arten, wie beispielsweise Puccinia recondita oder Puccinia triticina; Uromyces-Arten, wie beispielsweise Uromyces appendiculatus;
Erkrankungen, hervorgerufen durch Erreger der Gruppe der Oomyceten wie z.B. Bremia-Arten, wie beispielsweise Bremia lactucae; Peronospora-Arten, wie beispielsweise Peronospora pisi oder P. brassicae; Phytophthora-Arten, wie beispielsweise Phytophthora infestans; Plasmopara-Arten, wie beispielsweise Plasmopara viticola; Pseudoperonospora-Arten, wie beispielsweise Pseudoperonospora humuli oder Pseudoperonospora cubensis; Pythium-Arten, wie beispielsweise Pythium ultimum;
Blattfleckenkrankheiten und Blattwelken, hervorgerufen durch z.B. Alternaria-Arten, wie beispielsweise Alternaria solani; Cercospora-Arten, wie beispielsweise Cercospora beticola; Cladiosporum-Arten, wie beispielsweise Cladiosporium cucumerinum; Cochliobolus-Arten, wie beispielsweise Cochliobolus sativus (Konidienform: Drechslera, Syn: Helminthosporium); Colletotrichum-Arten, wie beispielsweise Colletotrichum lindemuthanium; Cycloconium-Arten, wie beispielsweise Cycloconium oleaginum; Diaporthe-Arten, wie beispielsweise Diaporthe citri; Elsinoe-Arten, wie beispielsweise Elsinoe fawcettii; Gloeosporium-Arten, wie beispielsweise Gloeosporium laeticolor; Glomerella-Arten, wie beispielsweise Glomerella cingulata; Guignardia-Arten, wie beispielsweise Guignardia bidwelli; Leptosphaeria-Arten, wie beispielsweise Leptosphaeria maculans; Magnaporthe-Arten, wie beispielsweise Magnaporthe grisea; Microdochium-Arten, wie beispielsweise Microdochium nivale; Mycosphaerella-Arten, wie beispielsweise Mycosphaerella graminicola und M. fijiensis; Phaeosphaeria-Arten, wie beispielsweise Phaeosphaeria nodorum; Pyrenophora-Arten, wie beispielsweise Pyrenophora teres; Ramularia-Arten, wie beispielsweise Ramularia collo-cygni; Rhynchosporium-Arten, wie beispielsweise Rhynchosporium secalis; Septoria-Arten, wie beispielsweise Septoria apii; Typhula-Arten, wie beispielsweise Typhula incarnata; Venturia-Arten, wie beispielsweise Venturia inaequalis;
Wurzel- und Stängelkrankheiten, hervorgerufen durch z.B. Corticium-Arten, wie beispielsweise Corticium graminearum; Fusarium-Arten, wie beispielsweise Fusarium oxysporum; Gaeumannomyces-Arten, wie beispielsweise Gaeumannomyces graminis; Rhizoctonia-Arten, wie beispielsweise Rhizoctonia solani; Tapesia-Arten, wie beispielsweise Tapesia acuformis; Thielaviopsis-Arten, wie beispielsweise Thielaviopsis basicola;
Ähren- und Rispenerkrankungen (inklusive Maiskolben), hervorgerufen durch z.B. Alternaria-Arten, wie beispielsweise Alternaria spp.; Aspergillus-Arten, wie beispielsweise Aspergillus flavus; Cladosporium-Arten, wie beispielsweise Cladosporium cladosporioides; Claviceps-Arten, wie beispielsweise Claviceps purpurea; Fusarium-Arten, wie beispielsweise Fusarium culmorum; Gibberella-Arten, wie beispielsweise Gibberella zeae; Monographella-Arten, wie beispielsweise Monographella nivalis; Septoria-Arten, wie beispielsweise Septoria nodorum;
Erkrankungen, hervorgerufen durch Brandpilze wie z.B. Sphacelotheca-Arten, wie beispielsweise Sphacelotheca reiliana; Tilletia-Arten, wie beispielsweise Tilletia caries, T. controversa; Urocystis-Arten, wie beispielsweise Urocystis occulta; Ustilago-Arten, wie beispielsweise Ustilago nuda, U. nuda tritici;
Fruchtfäule hervorgerufen durch z.B. Aspergillus-Arten, wie beispielsweise Aspergillus flavus; Botrytis-Arten, wie beispielsweise Botrytis cinerea; Penicillium-Arten, wie beispielsweise Penicillium expansum und P. purpurogenum; Sclerotinia-Arten, wie beispielsweise Sclerotinia sclerotiorum;
Verticilium-Arten, wie beispielsweise Verticilium alboatrum;
Samen- und bodenbürtige Fäulen und Welken, sowie Sämlingserkrankungen, hervorgerufen durch z.B. Fusarium-Arten, wie beispielsweise Fusarium culmorum; Phytophthora Arten, wie beispielsweise Phytophthora cactorum; Pythium-Arten, wie beispielsweise Pythium ultimum; Rhizoctonia-Arten, wie beispielsweise Rhizoctonia solani; Sclerotium-Arten, wie beispielsweise Sclerotium rolfsii;
Krebserkrankungen, Gallen und Hexenbesen, hervorgerufen durch z.B. Nectria-Arten, wie beispielsweise Nectria galligena;
Welkeerkrankungen hervorgerufen durch z.B. Monilinia-Arten, wie beispielsweise Monilinia laxa;
Deformationen von Blättern, Blüten und Früchten, hervorgerufen durch z.B. Taphrina-Arten, wie beispielsweise Taphrina deformans;
Degenerationserkrankungen holziger Pflanzen, hervorgerufen durch z.B. Esca-Arten, wie beispielsweise Phaemoniella clamydospora und Phaeoacremonium aleophilum und Fomitiporia mediterranea;
Blüten- und Samenerkrankungen, hervorgerufen durch z.B. Botrytis-Arten, wie beispielsweise Botrytis cinerea;
Erkrankungen von Pflanzenknollen, hervorgerufen durch z.B. Rhizoctonia-Arten, wie beispielsweise Rhizoctonia solani; Helminthosporium-Arten, wie beispielsweise Helminthosporium solani;
Erkrankungen, hervorgerufen durch bakterielle Erreger wie z.B. Xanthomonas-Arten, wie beispielsweise Xanthomonas campestris pv. oryzae; Pseudomonas-Arten, wie beispielsweise Pseudomonas syringae pv. lachrymans; Erwinia-Arten, wie beispielsweise Erwinia amylovora;
Bevorzugt können die folgenden Krankheiten von Soja-Bohnen bekämpft werden:
Pilzkrankheiten an Blättern, Stängeln, Schoten und Samen verursacht durch z.B. Alternaria leaf spot (Alternaria spec. atrans tenuissima), Anthracnose (Colletotrichum gloeosporoides dematium var. truncatum), Brown spot (Septoria glycines), Cercospora leaf spot and blight (Cercospora kikuchii), Choanephora leaf blight (Choanephora infundibulifera trispora (Syn.)), Dactuliophora leaf spot (Dactuliophora glycines), Downy Mildew (Peronospora manshurica), Drechslera blight (Drechslera glycini), Frogeye Leaf spot (Cercospora sojina), Leptosphaerulina Leaf Spot (Leptosphaerulina trifolii), Phyllostica Leaf Spot (Phyllosticta sojaecola), Pod and Stem Blight (Phomopsis sojae), Powdery Mildew (Microsphaera diffusa), Pyrenochaeta Leaf Spot (Pyrenochaeta glycines), Rhizoctonia Aerial, Foliage, and Web Blight (Rhizoctonia solani), Rust (Phakopsora pachyrhizi, Phakopsora meibomiae), Scab (Sphaceloma glycines), Stemphylium Leaf Blight (Stemphylium botryosum), Target Spot (Corynespora cassiicola).

Pilzkrankheiten an Wurzeln und der Stängelbasis verursacht durch z.B. Black Root Rot (Calonectria crotalariae), Charcoal Rot (Macrophomina phaseolina), Fusarium Blight or Wilt, Root Rot, and Pod and Collar Rot (Fusarium oxysporum, Fusarium orthoceras, Fusarium semitectum, Fusarium equiseti), Mycoleptodiscus Root Rot (Mycoleptodiscus terrestris), Neocosmospora (Neocosmopspora vasinfecta), Pod and Stem Blight (Diaporthe phaseolorum), Stem Canker (Diaporthe phaseolorum var. caulivora), Phytophthora Rot (Phytophthora megasperma), Brown Stem Rot (Phialophora gregata), Pythium Rot (Pythium aphanidermatum, Pythium irregulare, Pythium debaryanum, Pythium myriotylum, Pythium ultimum), Rhizoctonia Root Rot, Stem Decay, and Damping-Off (Rhizoctonia solani), Sclerotinia Stem Decay (Sclerotinia sclerotiorum), Sclerotinia Southern Blight (Sclerotinia rolfsii), Thielaviopsis Root Rot (Thielaviopsis basicola).

Unter unerwünschten Mikroorganismen sind im vorliegenden Fall phytopathogene Pilze und Bakterien zu verstehen. Die erfindungsgemäßen Stoffe können also eingesetzt werden, um Pflanzen innerhalb eines gewissen Zeitraumes nach der Behandlung gegen den Befall durch die genannten Schaderreger zu schützen. Der Zeitraum, innerhalb dessen Schutz herbeigeführt wird, erstreckt sich im allgemeinen von 1 bis 10 Tage, vorzugsweise 1 bis 7 Tage nach der Behandlung der Pflanzen mit den Wirkstoffen.

Die gute Pflanzenverträglichkeit der Wirkstoffe in den zur Bekämpfung von Pflanzenkrankheiten notwendigen Konzentrationen erlaubt eine Behandlung von oberirdischen Pflanzenteilen, von Pflanz-und Saatgut, und des Bodens.

Dabei lassen sich die erfindungsgemäßen Wirkstoffe mit besonders gutem Erfolg zur Bekämpfung von Getreidekrankheiten, wie beispielsweise gegen Erysiphe-Arten, gegen Puccinia und gegen Fusarien-Arten, von Reiskrankheiten, wie beispielsweise gegen Pyricularia und Rhizoctonia und von Krankheiten im Wein-, Obst-und Gemüseanbau, wie beispielsweise gegen Botrytis-, Venturia-, Sphaerotheca-und Podosphaera-Arten, einsetzen.

Die erfindungsgemäßen Wirkstoffe eignen sich auch zur Steigerung des Ernteertrages. Sie sind außerdem mindertoxisch und weisen eine gute Pflanzenverträglichkeit auf.

Die erfindungsgemäßen Verbindungen können gegebenenfalls in bestimmten Konzentrationen bzw. Aufwandmengen auch als Herbizide, Safener, Wachstumsregulatoren oder Mittel zur Verbesserung der Pflanzeneigenschaften, oder als Mikrobizide, beispielsweise als Fungizide, Antimykotika, Bakterizide, Virizide (einschließlich Mittel gegen Viroide) oder als Mittel gegen MLO (Mycoplasma-like-organism) und RLO (Rickettsia-like-organism) verwendet werden. Sie lassen sich gegebenenfalls auch als Insektizide einsetzen. Sie lassen sich gegebenenfalls auch als Zwischen- oder Vorprodukte für die Synthese weiterer Wirkstoffe einsetzen.

Die erfindungsgemäßen Wirkstoffe können gegebenenfalls in bestimmten Konzentrationen und Aufwandmengen auch als Herbizide, zur Beeinflussung des Pflanzenwachstums, sowie zur Bekämpfung von tierischen Schädlingen verwendet werden. Sie lassen sich gegebenenfalls auch als Zwischen-und Vorprodukte für die Synthese weiterer Wirkstoffe einsetzen.

Die erfindungsgemäßen Wirkstoffe eignen sich bei guter Pflanzenverträglichkeit, günstiger Warmblütertoxizität und guter Umweltverträglichkeit zum Schutz von Pflanzen und Pflanzenorganen, zur Steigerung der Ernteerträge, Verbesserung der Qualität des Ernteguts. Sie können vorzugsweise als Pflanzenschutzmittel eingesetzt werden. Sie sind gegen normal sensible und resistente Arten sowie gegen alle oder einzelne Entwicklungsstadien wirksam.

Die erfindungsgemäße Behandlung der Pflanzen und Pflanzenteile mit den Wirkstoffen bzw. Mitteln erfolgt direkt oder durch Einwirkung auf deren Umgebung, Lebensraum oder Lagerraum nach den üblichen Behandlungsmethoden, z.B. durch Tauchen, (Ver-)Spritzen, (Ver-)Sprühen, Berieseln, Verdampfen, Zerstäuben, Vernebeln, (Ver-)Streuen, Verschäumen, Bestreichen, Verstreichen, Gießen (drenchen), Tröpfchenbewässerung und bei Vermehrungsmaterial, insbesondere bei Samen, weiterhin durch Trockenbeizen, Nassbeizen, Schlämmbeizen, Inkrustieren, ein- oder mehrschichtiges Umhüllen usw. Es ist ferner möglich, die Wirkstoffe nach dem Ultra-Low-Volume-Verfahren auszubringen oder die Wirkstoffzubereitung oder den Wirkstoff selbst in den Boden zu injizieren.

Darüber hinaus kann durch die erfindungsgemäße Behandlung der Mykotoxingehalt im Erntegut und den daraus hergestellten Nahrungs- und Futtermitteln verringert werden. Besonders, aber nicht ausschließlich sind hierbei folgende Mykotoxine zu nennen: Deoxynivalenol (DON), Nivalenol, 15-Ac-DON, 3-Ac-DON, T2- und HT2- Toxin, Fumonisine, Zearalenon, Moniliformin, Fusarin, Diaceotoxyscirpenol (DAS), Beauvericin, Enniatin, Fusaroproliferin, Fusarenol, Ochratoxine, Patulin, Mutterkornalkaloide und Aflatoxine, die beispielsweise von den folgenden Pilzen verursacht werden können: Fusarium spec., wie Fusarium acuminatum, F. avenaceum, F. crookwellense, F. culmorum, F. graminearum (Gibberella zeae), F. equiseti, F. fujikoroi, F. musarum, F. oxysporum, F. proliferatum, F. poae, F. pseudograminearum, F. sambucinum, F. scirpi, F. semitectum, F. solani, F. sporotrichoides, F. langsethiae, F. subglutinans, F. tricinctum, F. verticillioides u.a. sowie auch von Aspergillus spec., Penicillium spec., Claviceps purpurea, Stachybotrys spec. u.a.

Die erfindungsgemäßen Wirkstoffe bzw. Mittel können außerdem im Materialschutz zum Schutz von technischen Materialien gegen Befall und Zerstörung durch unerwünschten Mikroorganismen, wie z.B. Pilzen, eingesetzt werden.

Unter technischen Materialien sind im vorliegenden Zusammenhang nichtlebende Materialien zu verstehen, die für die Verwendung in der Technik zubereitet worden sind. Beispielsweise können technische Materialien, die durch erfindungsgemäße Wirkstoffe vor mikrobieller Veränderung oder Zerstörung geschützt werden sollen, Klebstoffe, Leime, Papier und Karton, Textilien, Leder, Holz, Anstrichmittel und Kunststoffartikel, Kühlschmierstoffe und andere Materialien sein, die von Mikroorganismen befallen oder zersetzt werden können. Im Rahmen der zu schützenden Materialien seien auch Teile von Produktionsanlagen, beispielsweise Kühlwasserkreisläufe, genannt, die durch Vermehrung von Mikroorganismen beeinträchtigt werden können. Im Rahmen der vorliegenden Erfindung seien als technische Materialien vorzugsweise Klebstoffe, Leime, Papiere und Kartone, Leder, Holz, Anstrichmittel, Kühlschmiermittel und Wärmeübertragungsflüssigkeiten genannt, besonders bevorzugt Holz. Die erfindungsgemäßen Wirkstoffe bzw. Mittel können nachteilige Effekte wie Vermodern, Verfall, Ver-, Entfärbung oder Verschimmeln verhindern.

Das erfindungsgemäße Verfahren zum Bekämpfen von unerwünschten Pilzen kann auch zum Schutz von so genannte Storage Goods verwendet werden. Unter "Storage Goods" werden dabei natürliche Substanzen pflanzlichen oder tierischen Ursprungs oder deren Verarbeitungsprodukte, welche der Natur entnommen wurden und für die Langzeitschutz gewünscht ist, verstanden. Storage Goods pflanzlichen Ursprungs, wie z.B. Pflanzen oder Pflanzenteile, wie Stiele, Blätter, Knollen, Samen, Früchte, Körner, können in frisch geerntetem Zustand oder nach Verarbeitung durch (Vor-)Trocknen, Befeuchten, Zerkleinern, Mahlen, Pressen oder Rösten, geschützt werden. Storage Goods umfasst auch Nutzholz, sei es unverarbeitet, wie Bauholz, Stromleitungsmasten und Schranken, oder in Form fertiger Produkte, wie Möbel. Storage Goods tierischen Ursprungs sind beispielsweise Felle, Leder, Pelze und Haare. Die erfindungsgemäßen Wirkstoffe können nachteilige Effekte wie Vermodern, Verfall, Ver-, Entfärbung oder Verschimmeln verhindern.

Als Mikroorganismen, die einen Abbau oder eine Veränderung der technischen Materialien bewirken können, seien beispielsweise Bakterien, Pilze, Hefen, Algen und Schleimorganismen genannt. Vorzugsweise wirken die erfindungsgemäßen Wirkstoffe gegen Pilze, insbesondere Schimmelpilze, Holz verfärbende und Holz zerstörende Pilze (Basidiomyceten) sowie gegen Schleimorganismen und Algen. Es seien beispielsweise Mikroorganismen der folgenden Gattungen genannt: Alternaria, wie Alternaria tenuis; Aspergillus, wie Aspergillus niger; Chaetomium, wie Chaetomium globosum; Coniophora, wie Coniophora puetana; Lentinus, wie Lentinus tigrinus; Penicillium, wie Penicillium glaucum; Polyporus, wie Polyporus versicolor; Aureobasidium, wie Aureobasidium pullulans; Sclerophoma, wie Sclerophoma pityophila; Trichoderma, wie Trichoderma viride; Escherichia, wie Escherichia coli; Pseudomonas, wie Pseudomonas aeruginosa; Staphylococcus, wie Staphylococcus aureus.

Die vorliegende Erfindung betrifft weiterhin ein Mittel zum Bekämpfen unerwünschter Mikroorganismen, umfassend wenigstens eines der erfindungsgemäßen Diaminopyrimidine. Vorzugsweise handelt es sich um fungizide Mittel, welche landwirtschaftlich verwendbare Hilfsmittel, Solventien, Trägerstoffe, oberflächenaktive Stoffe oder Streckmittel enthalten.

Erfindungsgemäß bedeutet Trägerstoff eine natürliche oder synthetische, organische oder anorganische Substanz, mit welchen die Wirkstoffe zur besseren Anwendbarkeit, v.a. zum Aufbringen auf Pflanzen oder Pflanzenteile oder Saatgut, gemischt oder verbunden sind. Der Trägerstoff, welcher fest oder flüssig sein kann, ist im Allgemeinen inert und sollte in der Landwirtschaft verwendbar sein.

Als feste Trägerstoffe kommen infrage: z.B. Ammoniumsalze und natürliche Gesteinsmehle, wie Kaoline, Tonerden, Talkum, Kreide, Quarz, Attapulgit, Montmorillonit oder Diatomeenerde und synthetische Gesteinsmehle, wie hochdisperse Kieselsäure, Aluminiumoxid und Silikate, als feste Trägerstoffe für Granulate kommen in Frage: z.B. gebrochene und fraktionierte natürliche Gesteine wie Calcit, Marmor, Bims, Sepiolith, Dolomit sowie synthetische Granulate aus anorganischen und organischen Mehlen sowie Granulate aus organischem Material wie Papier, Sägemehl, Kokosnußschalen, Maiskolben und Tabakstengeln; als Emulgier- und/oder schaumerzeugende Mittel kommen in Frage: z.B. nichtionogene und anionische Emulgatoren, wie Polyoxyethylen-FettsäureEster, Polyoxyethylen-Fettalkohol-Ether, z.B. Alkylaryl-polyglykolether, Alkylsulfonate, Alkylsulfate, Arylsulfonate sowie Eiweißhydrolysate; als Dispergiermittel kommen in Frage nicht-ionische und/oder ionische Stoffe, z.B. aus den Klassen der Alkohol-POE- und/oder POP-Ether, Säure- und/oder POP-POE-Ester, Alkyl-Aryl- und/oder POP- POE-Ether, Fett- und/oder POP- POE-Addukte, POE- und/oder POP-Polyol Derivate, POE- und/oder POP-Sorbitan- oder-Zucker-Addukte, Alky- oder Aryl-Sulfate, Sulfonate und Phosphate oder die entsprechenden PO-Ether-Addukte. Ferner geeignete Oligo- oder Polymere, z.B. ausgehend von vinylischen Monomeren, von Acrylsäure, aus EO und/oder PO allein oder in Verbindung mit z.B. (poly-) Alkoholen oder (poly-) Aminen. Ferner können Einsatz finden Lignin und seine Sulfonsäure-Derivate, einfache und modifizierte Cellulosen, aromatische und/oder aliphatische Sulfonsäuren sowie deren Addukte mit Formaldehyd.

Die Wirkstoffe können in die üblichen Formulierungen überführt werden, wie Lösungen, Emulsionen, Spritzpulver, wasser- und ölbasierte Suspensionen, Pulver, Stäubemittel, Pasten, lösliche Pulver, lösliche Granulate, Streugranulate, Suspensions-Emulsions-Konzentrate, Wirkstoff-imprägnierte Naturstoffe, Wirkstoff-imprägnierte synthetische Stoffe, Düngemittel sowie Feinstverkapselungen in polymeren Stoffen.

Die Wirkstoffe können als solche, in Form ihrer Formulierungen oder den daraus bereiteten Anwendungsformen, wie gebrauchsfertige Lösungen, Emulsionen, wasser- oder ölbasierte Suspensionen, Pulver, Spritzpulver, Pasten, lösliche Pulver, Stäubemittel, lösliche Granulate, Streugranulate, Suspensions-Emulsions-Konzentrate, Wirkstoff-imprägnierte Naturstoffe, Wirkstoff-imprägnierte synthetische Stoffe, Düngemittel sowie Feinstverkapselungen in polymeren Stoffen angewendet werden. Die Anwendung geschieht in üblicher Weise, z.B. durch Gießen, Verspritzen, Versprühen, Verstreuen, Verstäuben, Verschäumen, Bestreichen usw. Es ist ferner möglich, die Wirkstoffe nach dem Ultra-Low-Volume-Verfahren auszubringen oder die Wirkstoffzubereitung oder den Wirkstoff selbst in den Boden zu injizieren. Es kann auch das Saatgut der Pflanzen behandelt werden.

Die genannten Formulierungen können in an sich bekannter Weise hergestellt werden, z.B. durch Vermischen der Wirkstoffe mit mindestens einem üblichen Streckmittel, Lösungs- bzw. Verdünnungsmittel, Emulgator, Dispergier- und/oder Binde- oder Fixiermittels, Netzmittel, Wasser-Repellent, gegebenenfalls Sikkative und UV-Stabilisatoren und gegebenenfalls Farbstoffen und Pigmenten, Entschäumer, Konservierungsmittel, sekundäre Verdickungsmittel, Kleber, Gibberelline sowie weiteren Verarbeitungshilfsmitteln.

Die erfindungsgemäßen Mittel umfassen nicht nur Formulierungen, welche bereits anwendungsfertig sind und mit einer geeigneten Apparatur auf die Pflanze oder das Saatgut ausgebracht werden können, sondern auch kommerzielle Konzentrate, welche vor Gebrauch mit Wasser verdünnt werden müssen.

Die erfindungsgemäßen Wirkstoffe können als solche oder in ihren (handelsüblichen) Formulierungen sowie in den aus diesen Formulierungen bereiteten Anwendungsformen in Mischung mit anderen (bekannten) Wirkstoffen, wie Insektiziden, Lockstoffen, Sterilantien, Bakteriziden, Akariziden, Nematiziden, Fungiziden, Wachstumsregulatoren, Herbiziden, Düngemitteln, Safener bzw. Semiochemicals vorliegen.

Als Hilfsstoffe können solche Stoffe Verwendung finden, die geeignet sind, dem Mittel selbst oder und/oder davon abgeleitete Zubereitungen (z.B. Spritzbrühen, Saatgutbeizen) besondere Eigenschaften zu verleihen, wie bestimmte technische Eigenschaften und/oder auch besondere biologische Eigenschaften. Als typische Hilfsmittel kommen in Frage: Streckmittel, Lösemittel und Trägerstoffe.

Als Streckmittel eignen sich z.B. Wasser, polare und unpolare organische chemische Flüssigkeiten z.B. aus den Klassen der aromatischen und nicht-aromatischen Kohlenwasserstoffe (wie Paraffine, Alkylbenzole, Alkylnaphthaline, Chlorbenzole), der Alkohole und Polyole (die ggf. auch substituiert, verethert und/oder verestert sein können), der Ketone (wie Aceton, Cyclohexanon), Ester (auch Fette und Öle) und (poly-)Ether, der einfachen und substituierten Amine, Amide, Lactame (wie N-Alkylpyrrolidone) und Lactone, der Sulfone und Sulfoxide (wie Dimethylsysulfoxid).

Mit verflüssigten gasförmigen Streckmitteln oder Trägerstoffen sind solche Flüssigkeiten gemeint, welche bei normaler Temperatur und unter Normaldruck gasförmig sind, z.B. Aerosol-Treibgase, wie Halogenkohlenwasserstoffe sowie Butan, Propan, Stickstoff und Kohlendioxid.

Es können in den Formulierungen Haftmittel wie Carboxymethylcellulose, natürliche und synthetische pulverige, körnige oder latexförmige Polymere verwendet werden, wie Gummiarabicum, Polyvinylalkohol, Polyvinylacetat, sowie natürliche Phospholipide, wie Kephaline und Lecithine, und synthetische Phospholipide. Weitere Additive können mineralische und vegetabile Öle sein.

Im Falle der Benutzung von Wasser als Streckmittel können z.B. auch organische Lösungsmittel als Hilfslösungsmittel verwendet werden. Als flüssige Lösungsmittel kommen im Wesentlichen infrage: Aromaten, wie Xylol, Toluol oder Alkylnaphthaline, chlorierte Aromaten oder chlorierte aliphatische Kohlenwasserstoffe, wie Chlorbenzole, Chlorethylene oder Methylenchlorid, aliphatische Kohlenwasserstoffe, wie Cyclohexan oder Paraffine, z.B. Erdölfraktionen, Alkohole, wie Butanol oder Glycol sowie deren Ether und Ester, Ketone, wie Aceton, Methylethylketon, Methylisobutylketon oder Cyclohexanon, stark polare Lösungsmittel, wie Dimethylformamid und Dimethylsulfoxid, sowie Wasser.

Die erfindungsgemäßen Mittel können zusätzlich weitere Bestandteile enthalten, wie z.B. oberflächenaktive Stoffe. Als oberflächenaktive Stoffe kommen Emulgier- und/oder Schaum erzeugende Mittel, Dispergiermittel oder Benetzungsmittel mit ionischen oder nicht-ionischen Eigenschaften oder Mischungen dieser oberflächenaktiven Stoffe infrage. Beispiele hierfür sind Salze von Polyacrylsäure, Salze von Lignosulphonsäure, Salze von Phenolsulphonsäure oder Naphthalinsulphonsäure, Polykondensate von Ethylenoxid mit Fettalkoholen oder mit Fettsäuren oder mit Fettaminen, substituierten Phenolen (vorzugsweise Alkylphenole oder Arylphenole), Salze von Sulphobernsteinsäureestern, Taurinderivate (vorzugsweise Alkyltaurate), Phosphorsäureester von polyethoxylierten Alkoholen oder Phenole, Fettsäureester von Polyolen, und Derivate der Verbindungen enthaltend Sulphate, Sulphonate und Phosphate, z.B. Alkylarylpolyglycolether, Alkylsulfonate, Alkylsulfate, Arylsulfonate, Eiweißhydrolysate, Lignin-Sulfitablaugen und Methylcellulose. Die Anwesenheit einer oberflächenaktiven Substanz ist notwendig, wenn einer der Wirkstoff und/oder einer der inerten Trägerstoffe nicht in Wasser löslich ist und wenn die Anwendung in Wasser erfolgt. Der Anteil an oberflächenaktiven Stoffen liegt zwischen 5 und 40 Gewichtsprozent des erfindungsgemäßen Mittels.

Es können Farbstoffe wie anorganische Pigmente, z.B. Eisenoxid, Titanoxid, Ferrocyanblau und organische Farbstoffe, wie Alizarin-, Azo- und Metallphthalocyaninfarbstoffe und Spurennährstoffe, wie Salze von Eisen, Mangan, Bor, Kupfer, Kobalt, Molybdän und Zink verwendet werden.

Weitere Additive können Duftstoffe, mineralische oder vegetabile gegebenenfalls modifizierte Öle, Wachse und Nährstoffe (auch Spurennährstoffe), wie Salze von Eisen, Mangan, Bor, Kupfer, Kobalt, Molybdän und Zink sein.

Weiterhin enthalten sein können Stabilisatoren wie Kältestabilisatoren, Konservierungsmittel, Oxidationsschutzmittel, Lichtschutzmittel oder andere die chemische und / oder physikalische Stabilität verbessernde Mittel.

Gegebenenfalls können auch andere zusätzliche Komponenten enthalten sein, z.B. schützende Kolloide, Bindemittel, Klebstoffe, Verdicker, thixotrope Stoffe, Penetrationsförderer, Stabilisatoren, Sequestiermittel, Komplexbildner. Im Allgemeinen können die Wirkstoffe mit jedem festen oder flüssigen Additiv, welches für Formulierungszwecke gewöhnlich verwendet wird, kombiniert werden.

Die Formulierungen enthalten im Allgemeinen zwischen 0,05 und 99 Gew.-%, 0,01 und 98 Gew.-%, vorzugsweise zwischen 0,1 und 95 Gew.-%, besonders bevorzugt zwischen 0,5 und 90 % Wirkstoff, ganz besonders bevorzugt zwischen 10 und 70 Gewichtsprozent.

Die zuvor beschriebenen Formulierungen können in einem erfindungsgemäßen Verfahren zum Bekämpfen unerwünschter Mikroorganismen verwendet werden, bei dem die erfindungsgemäßen Diaminopyrimidine auf die Mikroorganismen und/oder in deren Lebensraum ausgebracht werden.

Die erfindungsgemäßen Wirkstoffe können als solche oder in ihren Formulierungen auch in Mischung mit bekannten Fungiziden, Bakteriziden, Akariziden, Nematiziden oder Insektiziden verwendet werden, um so z.B. das Wirkungsspektrum zu verbreitern oder Resistenzentwicklungen vorzubeugen.

Als Mischpartner kommen zum Beispiel bekannte Fungizide, Insektizide, Akarizide, Nematizide oder auch Bakterizide (siehe auch Pesticide Manual, 13th ed.) infrage.

Auch eine Mischung mit anderen bekannten Wirkstoffen, wie Herbiziden, oder mit Düngemitteln und Wachstumsregulatoren, Safenern bzw. Semiochemicals ist möglich.

Die Anwendung geschieht in einer den Anwendungsformen angepaßten üblichen Weise.

Die Bekämpfung von pflanzenpathogenen Schadpilzen erfolgt in erster Linie durch die Behandlung des Bodens und der oberirdischen Pflanzenteile mit Pflanzenschutzmitteln. Aufgrund der Bedenken hinsichtlich eines möglichen Einflusses der Pflanzenschutzmittel auf die Umwelt und die Gesundheit von Menschen und Tieren gibt es Anstrengungen, die Menge der ausgebrachten Wirkstoffe zu vermindern.

Die Wirkstoffe können als solche, in Form ihrer Formulierungen oder den daraus bereiteten Anwendungsformen, wie gebrauchsfertige Lösungen, Suspensionen, Spritzpulver, Pasten, lösliche Pulver, Stäubemittel und Granulate angewendet werden. Die Anwendung geschieht in üblicher Weise, z.B. durch Gießen, Verspritzen, Versprühen, Verstreuen, Verstäuben, Verschäumen, Bestreichen usw. Es ist ferner möglich, die Wirkstoffe nach dem Ultra-Low-Volume-Verfahren auszubringen oder die Wirkstoffzubereitung oder den Wirkstoff selbst in den Boden zu injizieren. Es kann auch das Saatgut der Pflanzen behandelt werden.

Beim Einsatz der erfindungsgemäßen Wirkstoffe als Fungizide können die Aufwandmengen je nach Applikationsart innerhalb eines größeren Bereiches variiert werden. Die Aufwandmenge der erfindungsgemäßen Wirkstoffe beträgt
- bei der Behandlung von Pflanzenteilen, z.B. Blättern: von 0,1 bis 10 000 g/ha, bevorzugt von 10 bis 1 000 g/ha, besonders bevorzugt von 50 bis 300g/ha (bei Anwendung durch Gießen oder Tropfen kann die Aufwandmenge sogar verringert werden, vor allem wenn inerte Substrate wie Steinwolle oder Perlit verwendet werden);
- bei der Saatgutbehandlung: von 2 bis 200 g pro 100 kg Saatgut, bevorzugt von 3 bis 150 g pro 100 kg Saatgut, besonders bevorzugt von 2,5 bis 25 g pro 100 kg Saatgut, ganz besonders bevorzugt von 2,5 bis 12,5 g pro 100 kg Saatgut;
- bei der Bodenbehandlung: von 0,1 bis 10 000 g/ha, bevorzugt von 1 bis 5 000 g/ha.

Diese Aufwandmengen seien nur beispielhaft und nicht limitierend im Sinne der Erfindung genannt.

Zugleich können die erfindungsgemäßen Verbindungen zum Schutz vor Bewuchs von Gegenständen, insbesondere von Schiffskörpern, Sieben, Netzen, Bauwerken, Kaianlagen und Signalanlagen, welche mit See- oder Brackwasser in Verbindung kommen, eingesetzt werden.

Weiter können die erfindungsgemäßen Verbindungen allein oder in Kombinationen mit anderen Wirkstoffen als Antifouling-Mittel eingesetzt werden.

Das erfindungsgemäße Behandlungsverfahren kann für die Behandlung von genetisch modifizierten Organismen (GMOs), z. B. Pflanzen oder Samen, verwendet werden. Genetisch modifizierte Pflanzen (oder transgene Pflanzen) sind Pflanzen, bei denen ein heterologes Gen stabil in das Genom integriert worden ist. Der Begriff "heterologes Gen" bedeutet im wesentlichen ein Gen, das außerhalb der Pflanze bereitgestellt oder assembliert wird und das bei Einführung in das Zellkerngenom, das Chloroplastengenom oder das Hypochondriengenom der transformierten Pflanze dadurch neue oder verbesserte agronomische oder sonstige Eigenschaften verleiht, daß es ein interessierendes Protein oder Polypeptid exprimiert oder daß es ein anderes Gen, das in der Pflanze vorliegt bzw. andere Gene, die in der Pflanze vorliegen, herunterreguliert oder abschaltet (zum Beispiel mittels Antisense-Technologie, Cosuppressionstechnologie oder RNAi-Technologie [RNA Interference]). Ein heterologes Gen, das im Genom vorliegt, wird ebenfalls als Transgen bezeichnet. Ein Transgen, das durch sein spezifisches Vorliegen im Pflanzengenom definiert ist, wird als Transformations- bzw. transgenes Event bezeichnet.

In Abhängigkeit von den Pflanzenarten oder Pflanzensorten, ihrem Standort und ihren Wachstumsbedingungen (Böden, Klima, Vegetationsperiode, Ernährung) kann die erfindungsgemäße Behandlung auch zu überadditiven ("synergistischen") Effekten führen. So sind zum Beispiel die folgenden Effekte möglich, die über die eigentlich zu erwartenden Effekte hinausgehen: verringerte Aufwandmengen und/oder erweitertes Wirkungsspektrum und/oder erhöhte Wirksamkeit der Wirkstoffe und Zusammensetzungen, die erfindungsgemäß eingesetzt werden können, besseres Pflanzenwachstum, erhöhte Toleranz gegenüber hohen oder niedrigen Temperaturen, erhöhte Toleranz gegenüber Trockenheit oder Wasser- oder Bodensalzgehalt, erhöhte Blühleistung, Ernteerleichterung, Reifebeschleunigung, höhere Erträge, größere Früchte, größere Pflanzenhöhe, intensiver grüne Farbe des Blatts, frühere Blüte, höhere Qualität und/oder höherer Nährwert der Ernteprodukte, höhere Zuckerkonzentration in den Früchten, bessere Lagerfähigkeit und/oder Verarbeitbarkeit der Ernteprodukte.

Im vorliegenden Fall versteht man unter unerwünschten phytopathogenen Pilzen und/oder Mikroorganismen und/oder Viren phytopathogene Pilze, Bakterien und Viren. Die erfindungsgemäßen Substanzen lassen sich daher zum Schutz von Pflanzen gegen Angriff durch die erwähnten Pathogene innerhalb eines gewissen Zeitraums nach der Behandlung einsetzen. Der Zeitraum, über den eine Schutzwirkung erzielt wird, erstreckt sich im allgemeinen von 1 bis 10 Tagen, vorzugsweise 1 bis 7 Tagen, nach der Behandlung der Pflanzen mit den Wirkstoffen.

Zu Pflanzen und Pflanzensorten, die vorzugsweise erfindungsgemäß behandelt werden, zählen alle Pflanzen, die über Erbgut verfügen, das diesen Pflanzen besonders vorteilhafte, nützliche Merkmale verleiht (egal, ob dies durch Züchtung und/oder Biotechnologie erzielt wurde).

Pflanzen und Pflanzensorten, die ebenfalls vorzugsweise erfindungsgemäß behandelt werden, sind gegen einen oder mehrere biotische Streßfaktoren resistent, d. h. diese Pflanzen weisen eine verbesserte Abwehr gegen tierische und mikrobielle Schädlinge wie Nematoden, Insekten, Milben, phytopathogene Pilze, Bakterien, Viren und/oder Viroide auf.

Pflanzen und Pflanzensorten, die ebenfalls erfindungsgemäß behandelt werden können, sind solche Pflanzen, die gegen einen oder mehrere abiotische Streßfaktoren resistent sind. Zu den abiotischen Streßbedingungen können zum Beispiel Dürre, Kälte- und Hitzebedingungen, osmotischer Streß, Staunässe, erhöhter Bodensalzgehalt, erhöhtes Ausgesetztsein an Mineralien, Ozonbedingungen, Starklichtbedingungen, beschränkte Verfügbarkeit von Stickstoffnährstoffen, beschränkte Verfügbarkeit von Phosphornährstoffen oder Vermeidung von Schatten zählen.

Pflanzen und Pflanzensorten, die ebenfalls erfindungsgemäß behandelt werden können, sind solche Pflanzen, die durch erhöhte Ertragseigenschaften gekennzeichnet sind. Ein erhöhter Ertrag kann bei diesen Pflanzen z. B. auf verbesserter Pflanzenphysiologie, verbessertem Pflanzenwuchs und verbesserter Pflanzenentwicklung, wie Wasserverwertungseffizienz, Wasserhalteeffizienz, verbesserter Stickstoffverwertung, erhöhter Kohlenstoffassimilation, verbesserter Photosynthese, verstärkter Keimkraft und beschleunigter Abreife beruhen. Der Ertrag kann weiterhin durch eine verbesserte Pflanzenarchitektur (unter Streß- und nicht-Streß-Bedingungen) beeinflußt werden, darunter frühe Blüte, Kontrolle der Blüte für die Produktion von Hybridsaatgut, Keimpflanzenwüchsigkeit, Pflanzengröße, Internodienzahl und -abstand, Wurzelwachstum, Samengröße, Fruchtgröße, Schotengröße, Schoten- oder Ährenzahl, Anzahl der Samen pro Schote oder Ähre, Samenmasse, verstärkte Samenfüllung, verringerter Samenausfall, verringertes Schotenplatzen sowie Standfestigkeit. Zu weiteren Ertragsmerkmalen zählen Samenzusammensetzung wie Kohlenhydratgehalt, Proteingehalt, Ölgehalt und Ölzusammensetzung, Nährwert, Verringerung der nährwidrigen Verbindungen, verbesserte Verarbeitbarkeit und verbesserte Lagerfähigkeit.

Pflanzen, die erfindungsgemäß behandelt werden können, sind Hybridpflanzen, die bereits die Eigenschaften der Heterosis bzw. des Hybrideffekts exprimieren, was im allgemeinen zu höherem Ertrag, höherer Wüchsigkeit, besserer Gesundheit und besserer Resistenz gegen biotische und abiotische Streßfaktoren führt. Solche Pflanzen werden typischerweise dadurch erzeugt, daß man eine ingezüchtete pollensterile Elternlinie (den weiblichen Kreuzungspartner) mit einer anderen ingezüchteten pollenfertilen Elternlinie (dem männlichen Kreuzungspartner) kreuzt. Das Hybridsaatgut wird typischerweise von den pollensterilen Pflanzen geerntet und an Vermehrer verkauft. Pollensterile Pflanzen können manchmal (z. B. beim Mais) durch Entfahnen (d. h. mechanischem Entfernen der männlichen Geschlechtsorgane bzw. der männlichen Blüten), produziert werden; es ist jedoch üblicher, daß die Pollensterilität auf genetischen Determinanten im Pflanzengenom beruht. In diesem Fall, insbesondere dann, wenn es sich bei dem gewünschten Produkt, da man von den Hybridpflanzen ernten will, um die Samen handelt, ist es üblicherweise günstig, sicherzustellen, daß die Pollenfertilität in Hybridpflanzen, die die für die Pollensterilität verantwortlichen genetischen Determinanten enthalten, völlig restoriert wird. Dies kann erreicht werden, indem sichergestellt wird, daß die männlichen Kreuzungspartner entsprechende Fertilitätsrestorergene besitzen, die in der Lage sind, die Pollenfertilität in Hybridpflanzen, die die genetischen Determinanten, die für die Pollensterilität verantwortlich sind, enthalten, zu restorieren. Genetische Determinanten für Pollensterilität können im Cytoplasma lokalisiert sein. Beispiele für cytoplasmatische Pollensterilität (CMS) wurden zum Beispiel für Brassica-Arten beschrieben. Genetische Determinanten für Pollensterilität können jedoch auch im Zellkerngenom lokalisiert sein. Pollensterile Pflanzen können auch mit Methoden der pflanzlichen Biotechnologie, wie Gentechnik, erhalten werden. Ein besonders günstiges Mittel zur Erzeugung von pollensterilen Pflanzen ist in WO 89/10396 beschrieben, wobei zum Beispiel eine Ribonuklease wie eine Barnase selektiv in den Tapetumzellen in den Staubblättern exprimiert wird. Die Fertilität kann dann durch Expression eines Ribonukleasehemmers wie Barstar in den Tapetumzellen restoriert werden.

Pflanzen oder Pflanzensorten (die mit Methoden der Pflanzenbiotechnologie, wie der Gentechnik, erhalten werden), die erfindungsgemäß behandelt werden können, sind herbizidtolerante Pflanzen, d. h. Pflanzen, die gegenüber einem oder mehreren vorgegebenen Herbiziden tolerant gemacht worden sind. Solche Pflanzen können entweder durch genetische Transformation oder durch Selektion von Pflanzen, die eine Mutation enthalten, die solch eine Herbizidtoleranz verleiht, erhalten werden.

Herbizidtolerante Pflanzen sind zum Beispiel glyphosatetolerante Pflanzen, d. h. Pflanzen, die gegenüber dem Herbizid Glyphosate oder dessen Salzen tolerant gemacht worden sind. So können zum Beispiel glyphosatetolerante Pflanzen durch Transformation der Pflanze mit einem Gen, das für das Enzym 5-Enolpyruvylshikimat-3-phosphatsynthase (EPSPS) kodiert, erhalten werden.

Beispiele für solche EPSPS-Gene sind das AroA-Gen (Mutante CT7) des Bakterium *Salmonella typhimurium*, das CP4-Gen des Bakteriums *Agrobacterium sp.,* die Gene, die für eine EPSPS aus der Petunie, für eine EPSPS aus der Tomate oder für eine EPSPS aus Eleusine kodieren. Es kann sich auch um eine mutierte EPSPS handeln. Glyphosatetolerante Pflanzen können auch dadurch erhalten werden, daß man ein Gen exprimiert, das für ein Glyphosate-Oxidoreduktase-Enzym kodiert. Glyphosatetolerante Pflanzen können auch dadurch erhalten werden, daß man ein Gen exprimiert, das für ein Glyphosate-acetyltransferase-Enzym kodiert. Glyphosatetolerante Pflanzen können auch dadurch erhalten werden, daß man Pflanzen, die natürlich vorkommende Mutationen der oben erwähnten Gene selektiert.

Sonstige herbizidresistente Pflanzen sind zum Beispiel Pflanzen, die gegenüber Herbiziden, die das Enzym Glutaminsynthase hemmen, wie Bialaphos, Phosphinotricin oder Glufosinate, tolerant gemacht worden sind. Solche Pflanzen können dadurch erhalten werden, daß man ein Enzym exprimiert, das das Herbizid oder eine Mutante des Enzyms Glutaminsynthase, das gegenüber Hemmung resistent ist, entgiftet. Solch ein wirksames entgiftendes Enzym ist zum Beispiel ein Enzym, das für ein Phosphinotricin-acetyltransferase kodiert (wie zum Beispiel das bar- oder pat-Protein aus Streptomyces-Arten). Pflanzen, die eine exogene Phosphinotricin-acetyltransferase exprimieren, sind beschrieben.

Weitere herbizidtolerante Pflanzen sind auch Pflanzen, die gegenüber den Herbiziden, die das Enzym Hydroxyphenylpyruvatdioxygenase (HPPD) hemmen, tolerant gemacht worden sind. Bei den Hydroxyphenylpyruvatdioxygenasen handelt es sich um Enzyme, die die Reaktion, in der para-Hydroxyphenylpyruvat (HPP) zu Homogentisat umgesetzt wird, katalysieren. Pflanzen, die gegenüber HPPD-Hemmern tolerant sind, können mit einem Gen, das für ein natürlich vorkommendes resistentes HPPD-Enzym kodiert, oder einem Gen, das für ein mutiertes HPPD-Enzym kodiert, transformiert werden. Eine Toleranz gegenüber HPPD-Hemmern kann auch dadurch erzielt werden, daß man Pflanzen mit Genen transformiert, die für gewisse Enzyme kodieren, die die Bildung von Homogentisat trotz Hemmung des nativen HPPD-Enzyms durch den HPPD-Hemmer ermöglichen. Die Toleranz von Pflanzen gegenüber HPPD-Hemmern kann auch dadurch verbessert werden, daß man Pflanzen zusätzlich zu einem Gen, das für ein HPPDtolerantes Enzym kodiert, mit einem Gen transformiert, das für ein Prephenatdehydrogenase-Enzym kodiert.

Weitere herbizidresistente Pflanzen sind Pflanzen, die gegenüber Acetolactatsynthase (ALS)-Hemmern tolerant gemacht worden sind. Zu bekannten ALS-Hemmern zählen zum Beispiel Sulfonylharnstoff, Imidazolinon, Triazolopyrimidine, Pyrimidinyloxy(thio)benzoate und/oder Sulfonylaminocarbonyltriazolinon-Herbizide. Es ist bekannt, daß verschiedene Mutationen im Enzym ALS (auch als Acetohydroxysäure-Synthase, AHAS, bekannt) eine Toleranz gegenüber unterschiedlichen Herbiziden bzw. Gruppen von Herbiziden verleihen. Die Herstellung von sulfonylharnstofftoleranten Pflanzen und imidazolinontoleranten Pflanzen ist in der internationalen Veröffentlichung WO 1996/033270 beschrieben. Weitere sulfonylharnstoff- und imidazolinontolerante Pflanzen sind auch in z.B. WO 2007/024782 beschrieben.

Weitere Pflanzen, die gegenüber Imidazolinon und/oder Sulfonylharnstoff tolerant sind, können durch induzierte Mutagenese, Selektion in Zellkulturen in Gegenwart des Herbizids oder durch Mutationszüchtung erhalten werden.

Pflanzen oder Pflanzensorten (die nach Methoden der pflanzlichen Biotechnologie, wie der Gentechnik, erhalten wurden), die ebenfalls erfindungsgemäß behandelt werden können, sind insektenresistente transgene Pflanzen, d.h. Pflanzen, die gegen Befall mit gewissen Zielinsekten resistent gemacht wurden. Solche Pflanzen können durch genetische Transformation oder durch Selektion von Pflanzen, die eine Mutation enthalten, die solch eine Insektenresistenz verleiht, erhalten werden.

Der Begriff "insektenresistente transgene Pflanze" umfaßt im vorliegenden Zusammenhang jegliche Pflanze, die mindestens ein Transgen enthält, das eine Kodiersequenz umfaßt, die für folgendes kodiert:
1) ein insektizides Kristallprotein aus *Bacillus thuringiensis* oder einen insektiziden Teil davon, wie die insektiziden Kristallproteine, die online bei: http://www.lifesci.sussex.ac.uk/Home/Neil Crickmore/Bt/ beschrieben sind, zusammengestellt wurden, oder insektizide Teile davon, z.B. Proteine der Cry-Proteinklassen Cry1Ab, Cry1Ac, Cry1F, Cry2Ab, Cry3Ae oder Cry3Bb oder insektizide Teile davon; oder
2) ein Kristallprotein aus *Bacillus thuringiensis* oder einen Teil davon, der in Gegenwart eines zweiten, anderen Kristallproteins als *Bacillus thuringiensis* oder eines Teils davon insektizid wirkt, wie das binäre Toxin, das aus den Kristallproteinen Cy34 und Cy35 besteht; oder
3) ein insektizides Hybridprotein, das Teile von zwei unterschiedlichen insektiziden Kristallproteinen aus *Bacillus thuringiensis* umfaßt, wie zum Beispiel ein Hybrid aus den Proteinen von 1) oben oder ein Hybrid aus den Proteinen von 2) oben, z. B. das Protein Cry1A.105, das von dem Mais-Event MON98034 produziert wird (WO 2007/027777); oder
4) ein Protein gemäß einem der Punkte 1) bis 3) oben, in dem einige, insbesondere 1 bis 10, Aminosäuren durch eine andere Aminosäure ersetzt wurden, um eine höhere insektizide Wirksamkeit gegenüber einer Zielinsektenart zu erzielen und/oder um das Spektrum der entsprechenden Zielinsektenarten zu erweitern und/oder wegen Veränderungen, die in die Kodier- DNA während der Klonierung oder Transformation induziert wurden, wie das Protein Cry3Bb1 in Mais-Events MON863 oder MON88017 oder das Protein Cry3A im Mais-Event MIR 604;
5) ein insektizides sezerniertes Protein aus *Bacillus thuringiensis* oder *Bacillus cereus* oder einen insektiziden Teil davon, wie die vegetativ wirkenden insektentoxischen Proteine (vegetative insekticidal proteins, VIP), die unter http://www.lifesci.sussex.ac.uk/Home/Neil_Crickmore/Bt/vip.html angeführt sind, z. B. Proteine der Proteinklasse VIP3Aa; oder
6) ein sezerniertes Protein aus *Bacillus thuringiensis* oder *Bacillus cereus,* das in Gegenwart eines zweiten sezernierten Proteins aus *Bacillus thuringiensis* oder *B. cereus* insektizid wirkt, wie das binäre Toxin, das aus den Proteinen VIP1A und VIP2A besteht.
7) ein insektizides Hybridprotein, das Teile von verschiedenen sezernierten Proteinen von *Bacillus thuringiensis* oder *Bacillus cereus* umfaßt, wie ein Hybrid der Proteine von 1) oder ein Hybrid der Proteine von 2) oben; oder
8) ein Protein gemäß einem der Punkte 1) bis 3) oben, in dem einige, insbesondere 1 bis 10, Aminosäuren durch eine andere Aminosäure ersetzt wurden, um eine höhere insektizide Wirksamkeit gegenüber einer Zielinsektenart zu erzielen und/oder um das Spektrum der entsprechenden Zielinsektenarten zu erweitern und/oder wegen Veränderungen, die in die Kodier- DNA während der Klonierung oder Transformation induziert wurden (wobei die Kodierung für ein insektizides Protein erhalten bleibt), wie das Protein VIP3Aa im Baumwoll-Event COT 102.

Natürlich zählt zu den insektenresistenten transgenen Pflanzen im vorliegenden Zusammenhang auch jegliche Pflanze, die eine Kombination von Genen umfaßt, die für die Proteine von einer der oben genannten Klassen 1 bis 8 kodieren. In einer Ausführungsform enthält eine insektenresistente Pflanze mehr als ein Transgen, das für ein Protein nach einer der oben genannten 1 bis 8 kodiert, um das Spektrum der entsprechenden Zielinsektenarten zu erweitern oder um die Entwicklung einer Resistenz der Insekten gegen die Pflanzen dadurch hinauszuzögern, daß man verschiedene Proteine einsetzt, die für dieselbe Zielinsektenart insektizid sind, jedoch eine unterschiedliche Wirkungsweise, wie Bindung an unterschiedliche Rezeptorbindungsstellen im Insekt, aufweisen.

Pflanzen oder Pflanzensorten (die nach Methoden der pflanzlichen Biotechnologie, wie der Gentechnik, erhalten wurden), die ebenfalls erfindungsgemäß behandelt werden können, sind gegenüber abiotischen Streßfaktoren tolerant. Solche Pflanzen können durch genetische Transformation oder durch Selektion von Pflanzen, die eine Mutation enthalten, die solch eine Streßresistenz verleiht, erhalten werden. Zu besonders nützlichen Pflanzen mit Streßtoleranz zählen folgende:
a. Pflanzen, die ein Transgen enthalten, das die Expression und/oder Aktivität des Gens für die Poly(ADP-ribose)polymerase (PARP) in den Pflanzenzellen oder Pflanzen zu reduzieren vermag.
b. Pflanzen, die ein streßtoleranzförderndes Transgen enthalten, das die Expression und/oder Aktivität der für PARG kodierenden Gene der Pflanzen oder Pflanzenzellen zu reduzieren vermag;
c. Pflanzen, die ein streßtoleranzförderndes Transgen enthalten, das für ein in Pflanzen funktionelles Enzym des Nicotinamidadenindinukleotid-Salvage-Biosynthesewegs kodiert, darunter Nicotinamidase, Nicotinatphosphoribosyltransferase, Nicotinsäuremononukleotidadenyltransferase, Nicotinamidadenindinukleotidsynthetase oder Nicotinamidphosphoribosyltransferase.

Pflanzen oder Pflanzensorten (die nach Methoden der pflanzlichen Biotechnologie, wie der Gentechnik, erhalten wurden), die ebenfalls erfindungsgemäß behandelt werden können, weisen eine veränderte Menge, Qualität und/oder Lagerfähigkeit des Ernteprodukts und/oder veränderte Eigenschaften von bestimmten Bestandteilen des Ernteprodukts auf, wie zum Beispiel:
1) Transgene Pflanzen, die eine modifizierte Stärke synthetisieren, die bezüglich ihrer chemisch-physikalischen Eigenschaften, insbesondere des Amylosegehalts oder des Amylose/Amylopektin-Verhältnisses, des Verzweigungsgrads, der durchschnittlichen Kettenlänge, der Verteilung der Seitenketten, des Viskositätsverhaltens, der Gelfestigkeit, der Stärkekorngröße und/oder Stärkekornmorphologie im Vergleich mit der synthetisierten Stärke in Wildtyppflanzenzellen oder -pflanzen verändert ist, so daß sich diese modifizierte Stärke besser für bestimmte Anwendungen eignet.
2) Transgene Pflanzen, die Nichtstärkekohlenhydratpolymere synthetisieren, oder Nichtstärkekohlenhydratpolymere, deren Eigenschaften im Vergleich zu Wildtyppflanzen ohne genetische Modifikation verändert sind. Beispiele sind Pflanzen, die Polyfructose, insbesondere des Inulin- und Levantyps, produzieren, Pflanzen, die alpha-1,4-Glucane produzieren, Pflanzen, die alpha-1,6-verzweigte alpha-1,4-Glucane produzieren und Pflanzen, die Alternan produzieren.
3) Transgene Pflanzen, die Hyaluronan produzieren.

Pflanzen oder Pflanzensorten (die nach Methoden der pflanzlichen Biotechnologie, wie der Gentechnik, erhalten wurden), die ebenfalls erfindungsgemäß behandelt werden können, sind Pflanzen wie Baumwollpflanzen mit veränderten Fasereigenschaften. Solche Pflanzen können durch genetische Transformation oder durch Selektion von Pflanzen, die eine Mutation enthalten, die solche veränderten Fasereigenschaften verleiht, erhalten werden; dazu zählen:
a) Pflanzen wie Baumwollpflanzen, die eine veränderte Form von Cellulosesynthasegenen enthalten,
b) Pflanzen wie Baumwollpflanzen, die eine veränderte Form von rsw2- oder rsw3-homologen Nukleinsäuren enthalten;
c) Pflanzen wie Baumwollpflanzen mit einer erhöhten Expression der Saccharosephosphatsynthase;
d) Pflanzen wie Baumwollpflanzen mit einer erhöhten Expression der Saccharosesynthase;
e) Pflanzen wie Baumwollpflanzen bei denen der Zeitpunkt der Durchlaßsteuerung der Plasmodesmen an der Basis der Faserzelle verändert ist, z. B. durch Herunterregulieren der faserselektiven β-1,3-Glucanase;
f) Pflanzen wie Baumwollpflanzen mit Fasern mit veränderter Reaktivität, z. B. durch Expression des N-Acetylglucosamintransferasegens, darunter auch nodC, und von Chitinsynthasegenen.

Pflanzen oder Pflanzensorten (die nach Methoden der pflanzlichen Biotechnologie, wie der Gentechnik, erhalten wurden), die ebenfalls erfindungsgemäß behandelt werden können, sind Pflanzen wie Raps oder verwandte Brassica-Pflanzen mit veränderten Eigenschaften der Ölzusammensetzung. Solche Pflanzen können durch genetische Transformation oder durch Selektion von Pflanzen, die eine Mutation enthalten, die solche veränderten Öleigenschaften verleiht, erhalten werden; dazu zählen:
a) Pflanzen wie Rapspflanzen, die Öl mit einem hohen Ölsäuregehalt produziere;
b) Pflanzen wie Rapspflanzen, die Öl mit einem niedrigen Linolensäuregehalt produzieren.
c) Pflanzen wie Rapspflanzen, die Öl mit einem niedrigen gesättigten Fettsäuregehalt produzieren.

Besonders nützliche transgene Pflanzen, die erfindungsgemäß behandelt werden können, sind Pflanzen mit einem oder mehreren Genen, die für ein oder mehrere Toxine kodieren, sind die transgenen Pflanzen, die unter den folgenden Handelsbezeichnungen angeboten werden: YIELD GARD® (zum Beispiel Mais, Baumwolle, Sojabohnen), KnockOut® (zum Beispiel Mais), BiteGard® (zum Beispiel Mais), BT-Xtra® (zum Beispiel Mais), StarLink® (zum Beispiel Mais), Bollgard® (Baumwolle), Nucotn® (Baumwolle), Nucotn 33B® (Baumwolle), NatureGard® (zum Beispiel Mais), Protecta® und NewLeaf® (Kartoffel). Herbizidtolerante Pflanzen, die zu erwähnen sind, sind zum Beispiel Maissorten, Baumwollsorten und Sojabohnensorten, die unter den folgenden Handelsbezeichnungen angeboten werden: Roundup Ready® (Glyphosatetoleranz, zum Beispiel Mais, Baumwolle, Sojabohne), Liberty Link® (Phosphinotricintoleranz, zum Beispiel Raps), IMI® (Imidazolinontoleranz) und SCS® (Sylfonylharnstofftoleranz), zum Beispiel Mais. Zu den herbizidresistenten Pflanzen (traditionell auf Herbizidtoleranz gezüchtete Pflanzen), die zu erwähnen sind, zählen die unter der Bezeichnung Clearfield® angebotenen Sorten (zum Beispiel Mais).

Besonders nützliche transgene Pflanzen, die erfindungsgemäß behandelt werden können, sind Pflanzen, die Transformations-Events, oder eine Kombination von Transformations-Events, enthalten und die zum Beispiel in den Dateien von verschiedenen nationalen oder regionalen Behörden angeführt sind (siehe zum Beispiel http://gmoinfojrc.it/gmp_browse.aspx und http://www.agbios.com/dbase.php).

Die aufgeführten Pflanzen können besonders vorteilhaft erfindungsgemäß mit den Verbindungen der allgemeinen Formel (I) bzw. den erfindungsgemäßen Wirkstoffmischungen behandelt werden. Die bei den Wirkstoffen bzw. Mischungen oben angegebenen Vorzugsbereiche gelten auch für die Behandlung dieser Pflanzen. Besonders hervorgehoben sei die Pflanzenbehandlung mit den im vorliegenden Text speziell aufgeführten Verbindungen bzw. Mischungen.

Die erfindungsgemäßen Wirkstoffe bzw. Mittel können also eingesetzt werden, um Pflanzen innerhalb eines gewissen Zeitraumes nach der Behandlung gegen den Befall durch die genannten Schaderreger zu schützen. Der Zeitraum, innerhalb dessen Schutz herbeigeführt wird, erstreckt sich im Allgemeinen auf 1 bis 28 Tage, bevorzugt auf 1 bis 14 Tage, besonders bevorzugt auf 1 bis 10 Tage, ganz besonders bevorzugt auf 1 bis 7 Tage nach der Behandlung der Pflanzen mit den Wirkstoffen bzw. auf bis zu 200 Tage nach einer Saatgutbehandlung.

Die Herstellung und die Verwendung der erfindungsgemäßen Wirkstoffe der Formel (I) geht aus den folgenden Beispielen hervor. Die Erfindung ist jedoch nicht auf diese Beispiele beschränkt.

### Herstellung von Ausgangsstoffen der Formel (V):

### 2,5-Dichlor-N-(2,2-difluorethyl)pyrimidin-4-amin(V-1)

Zu einer Lösung von 5.43 g (29.6 mmol) 2,4,5-Trichlorpyrimidin in 40 ml Acetonitril wird bei -10 °C 6.14 g (44.4 mmol) Kaliumcarbonat zugesetzt. Anschließend tropft man 2.40 g (29.6 mmol) 2,2-Difluorethanamin als 30 %ige Acetonitril-Lösung zu. Das Reaktionsgemisch lässt man unter Rühren über Nacht auf Raumtemperatur erwärmen. Das Reaktionsgemisch wird in 250 ml Eiswasser/verdünnter Salzsäure (1:1) eingerührt. Man extrahiert mit Dichlormethan (2x 200 ml), wäscht die vereinten organischen Phasen im Anschluss mit Wasser (100 ml), trocknet über MgSO₄ und entfernt das Lösungsmittel unter vermindertem Druck. Man erhält 6.10 g (90 %) 2,5-Dichlor-N-(2,2-difluorethyl)pyrimidin-4-amin (V-1) (logP (pH2.3): 1.96).

### 2,5-Dichlor-N-(2,2,2-trifluorethyl)pyrimidin-4-amin(V-2)

Zu einer Lösung von 16.0 g (87.2 mmol) 2,4,5-Trichlorpyrimidin in 100 ml Acetonitril wird bei 50 °C 18.1 g (130 mmol) Kaliumcarbonat zugesetzt. Anschließend tropft man 9.07 g (91.6 mmol) 2,2,2-Trifluorethanamin als 30 %ige Acetonitril-Lösung zu. Das Reaktionsgemisch rührt 16 h bei 50 °C nach. Nach dem Erkalten wird das Reaktionsgemisch in 250 ml Eiswasser eingerührt. Man extrahiert mit Ethylacetat (2x 200 ml), wäscht die vereinten organischen Phasen im Anschluss mit Wasser (2x 100 ml), trocknet über MgSO₄ und entfernt das Lösungsmittel unter vermindertem Druck. Das Rohprodukt wird mit Cyclohexan verrührt und der ausgefallene Feststoff nach 2 h abfiltriert und getrocknet. Man erhält 13.9 g (64 %) 2,5-Dichlor-N-(2,2,2-trifluorethyl)pyrimidin-4-amin (V-2) (logP (pH2.3): 2.26).

### 2,5-Dichlor-N-(1,1,1-trifluorpropan-2-yl)pyrimidin-4-amin (V-3)

Zu einer Lösung von 4.00 g (21.8 mmol) 2,4,5-Trichlorpyrimidin in 40 ml Acetonitril wird bei 50 °C 4.5 g (33.0 mmol) Kaliumcarbonat zugesetzt. Anschließend gibt man 3.5 g (31 mmol) 1,1,1-Trifluorpropan-2-amin als 30 %ige Acetonitril-Lösung zu. Das Reaktionsgemisch rührt 16 h bei 70 °C nach. Nach dem Erkalten wird das Reaktionsgemisch unter vermindertem Druck eingeengt und in 100 ml Eiswasser eingerührt. Man extrahiert mit Dichlormethan (2x 100 ml), wäscht die vereinten organischen Phasen im Anschluss mit Wasser (2x 100 ml), trocknet über MgSO₄ und entfernt das Lösungsmittel unter vermindertem Druck. Das Rohprodukt wird mit Cyclohexan verrührt und der ausgefallene Feststoff nach 2 h abfiltriert. Das Filtrat wird eingeengt und man erhält 1.90 g (32 %) 2,5-Dichlor-N-(1,1,1-trifluorpropan-2-yl)pyrimidin-4-amin (V-3) (logP (pH2.3): 2.66).

### Herstellung von Verbindungen der Formel (I)

### Methode A:

### 5-Chlor-N⁴-(2,2-difluorethyl)-N²-phenylpyrimidin-2,4-diamin (Verbindung 1)

Zu einer Lösung von 300 mg (1.32 mmol) 2,5-Dichlor-N-(2,2-difluorethyl)pyrimidin-4-amin in 10 ml Acetonitril setzt man bei Raumtemperatur 270 mg (2.89 mmol) Anilin und dann 510 µl einer 4 M HCl in Dioxan zu und erhitzt auf 85 °C. Nach 16 h filtriert man das Reaktionsgemisch heiß und lässt das Filtrat unter Rühren abkühlen. Das aus dem Filtrat ausgefallene Produkt wird abfiltriert und in 10 ml Wasser aufgeschlämmt, mit Wasser (2x 10 ml) gewaschen und getrocknet. Man erhält 184 mg (44%) des gewünschten Produktes (logP (pH2.3): 1.89).

### Methode B:

### 5-Chlor-N²-phenyl-N⁴-(2,2,2-trifluorethyl)pyrimidin-2,4-diamin (Verbindung 3)

Ein Gemisch aus 246 mg (1.00 mmol) 2,5-Dichlor-N-(2,2,2-trifluorethyl)pyrimidin-4-amin, 116 mg (1.25 mmol) Anilin und 137 mg (0.80 mmol) 4-Toluolsulfonsäure in 12 ml Dioxan wird 16 h bei 105 °C gerührt. Nach dem Erkalten rührt wird der ausgefallene Niederschlag abfiltriert, in 10 ml Wasser aufgeschlämmt, mit gesät. NaHCO₃-Lösung neutralisiert, mit Wasser gewaschen und getrocknet. Man erhält 200 mg des gewünschten Produktes (logP (pH2.3): 2.56).

### Herstellung von Ausgangsverbindungen der Formel (IX):

### 2-Anilino-5-brompyrimidin-4(3H)-on (IX-1)

Zu einer Lösung von 55.0 g (241 mmol) 5-Brom-2,4-dichlorpyrimidin in 400 ml Dioxan gibt man eine Lösung bestehend aus 25.7 g (459 mmol) KOH in 110 ml Wasser und rührt 16 h bei raumtemperatur und 6 h bei 50 °C nach. Das Reaktionsgemisch wurde unter vermindertem Druck eingeengt. Den Rückstand nimmt man in einem Gemisch aus 250 ml Ethylacetet und 250 ml Wasser auf und neutralisiert mit wäßriger Salzsäure auf pH 7. Nach dem Abtrennen der organischen Phase wird die wäßrige Phase auf pH 3 angesäuert und mit Ethylacetat (3x 250 ml) extrahiert. Die vereinigten organischen Phasen werden mit gesät. NaCl-Lösung gewaschen, über MgSO₄ getrocknet und unter vermindertem Druck vom Lösungsmittel befreit. Das Rohprodukt wird zusammen mit 12.3 g (131 mmol) Anilin in 455 ml Dioxan aufgenommen und unter Rühren auf 92 °C erhitzt. Nach 18 h engt man das Reaktionsgemisch unter vermindertem Druck ein und nimmt den Rückstand in 350 ml Propan-2-ol auf und gibt portionsweise 300 ml einer gesät. NaHCO₃-Lösung zu. Der ausgefallene Feststoff wird abfiltriert, mit einem kalten Gemisch aus Propan-2-ol und Wasser (1:1) gewaschen und getrocknet. Man erhält 17.7 g 2-Anilino-5-brompyrimidin-4(3H)-on (IX-1), das ohne weitere Aufreinigung direkt weiter umgesetzt wird. logP (pH2.3): 1.62.

### Herstellung von Ausgangsverbindungen der Formel (X):

### 5-Brom-4-chlor-N-phenylpyrimidin-2-amin (X-1)

Eine Lösung von 28.0 g 2-Anilino-5-brompyrimidin-4(3H)-on in 140 ml Phophorylchlorid wird 18 h auf 85 °C erhitzt. Nach dem Erkalten wird das Reaktionsgemisch unter vermindertem Druck eingeengt, und in 150 ml Dichlormethan wiederaufgenommen. Man gibt langsam 150 ml Eiswasser zu und neutralisiert das Gemisch mittels wäßriger Ammoniak-Lösung (28%ig). Die wäßrige Phase wird abgetrennt und mit Dichlormethan (2x 150 ml) extrahiert. Die vereinigten organischen Phasen werden mit Wasser gewaschen, über MgSO₄ getrocknet und unter vermindertem Druck vom Lösungsmittel befreit. Das Rohprodukt wurde aus Propan-2-ol/Wasser umkristallisiert. Man erhält 15.6 g 5-Brom-4-chlor-N-phenylpyrimidin-2-amin (X-1) (logP (pH2.3): 3.64).

### Herstellung von Verbindungen der Formel (I)

### 5-Brom-N²-phenyl-N⁴-(4,4,4-trifluorbutan-2-yl)pyrimidin-2,4-diamin (Verbindung 42)

Zu einer Lösung von 193 mg (0.68 mmol) 5-Brom-4-chlor-N-phenylpyrimidin-2-amin in 8 ml Acetonitril gibt man bei Raumtemperatur 261 mg (2.6 mmol) Triethylamin und 133 mg (0.82 mmol) 4,4,4-Trifluorbutan-2-amin Hydrochlorid. Das Reaktionsgemisch wird für 48 h auf 70 °C erwärmt. Im Anschluß entfernt man das Lösungsmittel unter vermindertem Druck und nimmt den erhaltenden Rückstand in 10 ml Ethylacetat und 10 ml ges. NaHCO₃-Lösung wieder auf. Die wäßrige Phase wird erneut mit Ethylacetat (10 ml) extrahiert. Die vereinigten organischen Phasen werden über MgSO₄ getrocknet und unter vermindertem Druck vom Lösungsmittel befreit. Das Rohprodukt wird im Anschluss säulenchromatographisch über RP18 (Wasser/Acetonitril) gereinigt. Man erhält 19 mg (7 %) des gewünschten Produktes (logP (pH2.7): 2.40).

### Beispiele

Nach den zuvor angegebenen Methoden werden auch die in der nachstehenden Tabelle I genannten Verbindungen der Formel (I) erhalten.

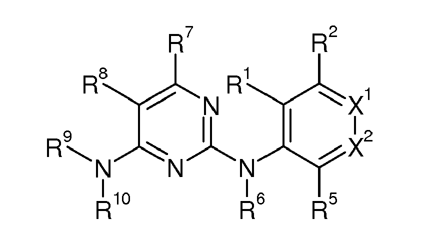

**Tabelle I**

| Bsp | R⁵ | X² = CR⁴ | X¹ = CR³ | R² | R¹ | R⁶ | R⁷ | R⁸ | R⁹ | R¹⁰ | Logp |
|---|---|---|---|---|---|---|---|---|---|---|---|
| 1 | H | H | H | H | H | H | H | Cl | H | 2,2-Difluorethyl | 1,89[a] |
| 2 | H | H | H | H | H | H | H | Cl | H | 1,1,1-Trifluorpropan-2-yl | 2,87[a] |
| 3 | H | H | H | H | H | H | H | Cl | H | 2,2,2-Trifluorethyl | 2,56[a]; 3,09[c] |
| 4 | H | Cyan | H | H | H | H | H | Cl | H | 2,2,2-Trifluorethyl | 2,67[b] |
| 5 | H | Methoxy | Acetylamino | H | H | H | H | Cl | H | 2,2,2-Trifluorethyl | 1,75[b] |
| 6 | H | Methoxy | H | H | H | H | H | Cl | H | 2,2,2-Trifluorethyl | 2,55[b] |
| 7 | H | H | Methoxy | H | H | H | H | Cl | H | 2,2,2-Trifluorethyl | 2,06[b] |
| 8 | H | H | Cyan | H | H | H | H | Cl | H | 2,2,2-Trifluorethyl | 2,77[b] |
| 9 | H | Trifluormethoxy | H | H | H | H | H | Cl | H | 2,2,2-Trifluorethyl | 3,72[b] |
| 10 | H | H | Methylsulfonyl | H | H | H | H | Cl | H | 2,2,2-Trifluorethyl | 2,21[b] |
| 11 | H | H | Dimethylsulfamoyl | H | H | H | H | Cl | H | 2,2,2-Trifluorethyl | 2,64[b] |
| 12 | H | Chlor | Methylsulfanyl | H | H | H | H | Cl | H | 2,2,2-Trifluorethyl | 3,55[b] |
| 13 | H | H | Acetylamino | H | H | H | H | Cl | H | 2,2,2-Trifluorethyl | 1,54[b] |
| 14 | H | Chlor | Acetylamino | H | H | H | H | Cl | H | 2,2,2-Trifluorethyl | 2,04[b] |
| 15 | H | H | Acetyl(ethyl)amino | H | H | H | H | Cl | H | 2,2,2-Trifluorethyl | 2,13[b] |
| 16 | H | H | Acetyl(methyl)amino | H | H | H | H | Cl | H | 2,2,2-Trifluorethyl | 1,92[b] |
| 17 | H | H | (Trifluoracetyl)amino | H | H | H | H | Cl | H | 2,2,2-Trifluorethyl | 2,55[b] |
| 18 | H | H | [(1-Methylcyclopropyl)carb onyl]amino | H | H | H | H | Cl | H | 2,2,2-Trifluorethyl | 2,16[b] |
| 19 | H | Acetylamino | H | H | H | H | H | Cl | H | 2,2,2-Trifluorethyl | 1,66[b] |
| 20 | H | (2-Methylacryloyl)amino | H | H | H | H | H | Cl | H | 2,2,2-Trifluorethyl | 2,16[b] |
| 21 | H | (2,2-Dimethylpropanoyl)ami no | H | H | H | H | H | Cl | H | 2,2,2-Trifluorethyl | 2,51[b] |
| 22 | H | H | Methylcarbamoyl | H | H | H | H | Cl | H | 2,2,2-Trifluorethyl | 1,66[b] |
| 23 | H | Methylcarbamoyl | H | H | H | H | H | Cl | H | 2,2,2-Trifluorethyl | 1,66[b] |
| 24 | H | Methylsulfanyl | H | H | H | H | H | Cl | H | 2,2,2-Trifluorethyl | 3,04[b] |
| 25 | H | H | Methylsulfanyl | H | H | H | H | Cl | H | 2,2,2-Trifluorethyl | 2,92[b] |
| 26 | H | Piperidin-1-ylsulfonyl | H | H | H | H | H | Cl | H | 2,2,2-Trifluorethyl | 2,58[b] |
| 27 | H | H | 3,3-Dimethyl-2-oxobutyl | H | H | H | H | Cl | H | 2,2,2-Trifluorethyl | 3,17[b] |
| 28 | H | H | Acetyl | H | H | H | H | Cl | H | 2,2,2-Trifluorethyl | 2,51 [b] |
| 29 | H | Acetyl | H | H | H | H | H | Cl | H | 2,2,2-Trifluorethyl | 2,39[b] |
| 30 | H | H | 2,5-Dioxoimidazolidin-4-yl | H | H | H | H | Cl | H | 2,2,2-Trifluorethyl | 1,47[b] |
| 31 | H | H | (Methoxycarbonyl)amin o | H | H | H | H | Cl | H | 2,2,2-Trifluorethyl | 1,85[b] |
| 32 | H | H | (Ethoxycarbonyl)amino | H | H | H | H | Cl | H | 2,2,2-Trifluorethyl | 2,11[b] |
| 33 | H | (Ethoxycarbonyl)amino | H | H | H | H | H | Cl | H | 2,2,2-Trifluorethyl | 2,36[b] |
| 34 | H | (Methylcarbamoyl)oxy | H | H | H | H | H | Cl | H | 2,2,2-Trifluorethyl | 1,94[b] |
| 35 | H | (Methoxycarbonyl)ami no | H | H | H | H | H | Cl | H | 2,2,2-Trifluorethyl | 2,06[b] |
| 36 | H | -OCONH- | | H | H | H | H | Cl | H | 2,2,2-Trifluorethyl | 1,71[b] |
| 37 | H | (3-Fluor-2,2-dimethylpropanoyl)ami no | H | H | H | H | H | Cl | H | 2,2,2-Trifluorethyl | 2,42[b] |
| 38 | H | Acetylamino | CH₃ | H | H | H | H | Cl | H | 2,2,2-Trifluorethyl | 1,61[b] |
| 39 | H | Methoxy | H | H | H | H | H | Cl | H | 2,2-Difluorethyl | 1,96[b] |
| 40 | H | H | Dimethylcarbamoyl | H | H | H | H | Cl | H | 2,2-Difluorethyl | 1,55[b] |
| 41 | H | H | Prop-2-en-1-ylcarbamoyl | H | H | H | H | Cl | H | 2,2-Difluorethyl | 1,84[b] |
| 42 | H | H | H | H | H | H | H | Bro m | H | 4,4,4-Trifluorbutan-2-yl | 2,4[b] |
| 43 | H | Cyanmethyl | H | H | H | H | H | Cl | H | 2,2-Difluorethyl | 1,89[b] |
| 44 | H | CH₃ | H | H | H | H | H | Cl | H | 2,2,2-Trifluorethyl | 2,88[b] |
| 45 | H | CH₃ | H | H | H | H | H | Cl | H | 2,2-Difluorethyl | 2,27[b] |
| 46 | H | Formyl | H | H | H | H | H | Cl | H | 2,2,2-Trifluorethyl | 2,67[c]; 2,46[b] |
| 47 | H | Formyl | H | H | H | H | H | Cl | H | 2,2-Difluorethyl | 2,49[c]; 2,01[b] |
| 48 | H | H | Dimethylcarbamoyl | H | H | H | H | Cl | H | 2,2,2-Trifluorethyl | 1,93[b] |

Die Messung der logP Werte erfolgte gemäß EEC Directive 79/831 Annex V.A8 durch HPLC (High Performance Liquid Chromatography) an reversed-phase Säulen (C 18), mit nachfolgenden Methoden:
^{[a]} Die Bestimmung erfolgt im sauren Bereich bei pH 2.3 mit 0,1% wässriger Phosphorsäure und Acetonitril als Eluenten
   linearer Gradient von 10% Acetonitril bis 95% Acetonitril.
^{[b]} Die Bestimmung mit der LC-MS im sauren Bereich erfolgt bei pH 2,7 mit 0,1 % wässriger Ameisensäure und Acetonitril (enthält 0,1% Ameisensäure) als Eluenten
   linearer Gradient von 10% Acetonitril bis 95% Acetonitril
^{[c]} Die Bestimmung mit der LC-MS im neutralen Bereich erfolgt bei pH 7.8 mit 0,001 molarer wässriger Ammoniumhydrogencarbonat-Lösung und Acetonitril als Eluenten
   linearer Gradient von 10 % Acetonitril bis 95 % Acetonitril.

Die Eichung erfolgt mit unverzweigten Alkan-2-onen (mit 3 bis 16 Kohlenstoffatomen), deren logP-Werte bekannt sind (Bestimmung der logP-Werte anhand der Retentionszeiten durch lineare Interpolation zwischen zwei aufeinander folgenden Alkanonen).

Die lambda-maX-Werte wurden an Hand der UV-Spektren von 200 nm bis 400 nm in den Maxima der chromatographischen Signale ermittelt.

### Verwendungsbeispiele

### Beispiel A

### Venturia - Test (Apfel) / protektiv

- Lösungsmittel:: 24,5 Gewichtsteile Aceton
24,5 Gewichtsteile Dimethylacetamid
- Emulgator :: 1 Gewichtsteil Alkyl-Aryl-Polyglykolether

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit Wasser auf die gewünschte Konzentration.

Zur Prüfung auf protektive Wirksamkeit werden junge Pflanzen mit der Wirkstoffzubereitung in der angegebenen Aufwandmenge besprüht. Nach Antrocknen des Spritzbelages werden die Pflanzen mit einer wäßrigen Konidiensuspension des Apfelschorferregers ***Venturia inaequalis*** inokuliert und verbleiben dann 1 Tag bei ca. 20°C und 100% relativer Luftfeuchtigkeit in einer Inkubations-kabine.

Die Pflanzen werden dann im Gewächshaus bei ca. 21°C und einer relativen Luftfeuchtigkeit von ca. 90% aufgestellt.

10 Tage nach der Inokulation erfolgt die Auswertung. Dabei bedeutet 0% ein Wirkungsgrad, der demjenigen der Kontrolle entspricht, während ein Wirkungsgrad von 100% bedeutet, daß kein Befall beobachtet wird.

In diesem Test zeigen die erfindungsgemäßen Verbindungen 1 und 3 aus Tabelle 1 bei einer Konzentration an Wirkstoff von 100ppm einen Wirkungsgrad von 70% oder mehr.

### Beispiel B

### Botrytis - Test (Bohne) / protektiv

- Lösungsmittel:: 24,5 Gewichtsteile Aceton
24,5 Gewichtsteile Dimethylacetamid
- Emulgator :: 1 Gewichtsteil Alkyl-Aryl-Polyglykolether

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit Wasser auf die gewünschte Konzentration.

Zur Prüfung auf protektive Wirksamkeit werden junge Pflanzen mit der Wirkstoffzubereitung in der angegebenen Aufwandmenge besprüht. Nach Antrocknen des Spritzbelages werden auf jedes Blatt 2 kleine mit Botrytis cinerea bewachsene Agarstückchen aufgelegt. Die inokulierten Pflanzen werden in einer abgedunkelten Kammer bei ca. 20°C und 100% relativer Luftfeuchtigkeit aufgestellt.

2 Tage nach der Inokulation wird die Größe der Befallsflecken auf den Blättern ausgewertet. Dabei bedeutet 0% ein Wirkungsgrad, der demjenigen der Kontrolle entspricht, während ein Wirkungsgrad von 100% bedeutet, daß kein Befall beobachtet wird.

In diesem Test zeigen die erfindungsgemäßen Verbindungen 43 aus Tabelle 1 bei einer Konzentration an Wirkstoff von 250ppm einen Wirkungsgrad von 70% oder mehr.

### Beispiel C

### Alternaria-Test (Tomate) / protektiv

- Lösungsmittel:: 49 Gewichtsteile N, N - Dimethylformamid
- Emulgator:: 1 Gewichtsteil Alkylarylpolyglykolether

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit Wasser auf die gewünschte Konzentration.

Zur Prüfung auf protektive Wirksamkeit bespritzt man junge Tomatenpflanzen mit der Wirkstoffzubereitung in der angegebenen Aufwandmenge. 1 Tag nach der Behandlung werden die Pflanzen mit einer Sporensuspension von ***Alternaria solani*** inokuliert und stehen dann 24h bei 100% rel. Feuchte und 22°C. Anschließend stehen die Pflanzen bei 96% rel. Luftfeuchtigkeit und einer Temperatur von 20°C.

7 Tage nach der Inokulation erfolgt die Auswertung. Dabei bedeutet 0 % ein Wirkungsgrad, der demjenigen der Kontrolle entspricht, während ein Wirkungsgrad von 100 % bedeutet, dass kein Befall beobachtet wird.

In diesem Test zeigen die erfindungsgemäßen Verbindungen 1, 4, 15, 16, 27, 34, 43, 46, 48 aus Tabelle 1 bei einer Konzentration an Wirkstoff von 500ppm einen Wirkungsgrad von 70% oder mehr.

### Beispiel D

### Puccinia-Test (Weizen) / protektiv

- Lösungsmittel:: 49 Gewichtsteile N, N - Dimethylformamid
- Emulgator:: 1 Gewichtsteil Alkylarylpolyglykolether

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit Wasser auf die gewünschte Konzentration.

Zur Prüfung auf protektive Wirksamkeit bespritzt man junge Weizenpflanzen mit der Wirkstoffzubereitung in der angegebenen Aufwandmenge. 1 Tag nach der Behandlung werden die Pflanzen mit einer Sporensuspension von ***Puccinia recondita*** inokuliert und stehen dann 48h bei 100% rel. Feuchte und 22°C. Anschließend stehen die Pflanzen bei 80% rel. Luftfeuchtigkeit und einer Temperatur von 20°C.

7-9 Tage nach der Inokulation erfolgt die Auswertung. Dabei bedeutet 0 % ein Wirkungsgrad, der demjenigen der Kontrolle entspricht, während ein Wirkungsgrad von 100 % bedeutet, dass kein Befall beobachtet wird.

In diesem Test zeigen die erfindungsgemäßen Verbindungen 3, 5, 6, 13, 15, 16, 19, 21, 23, 24, 26, 29, 34, 35, 36, 37, 38, 40, 41, 43, 45, 46, 47 aus Tabelle 1 bei einer Konzentration an Wirkstoff von 500ppm einen Wirkungsgrad von 70% oder mehr.

### Beispiel E

### Fusarium nivale (var.majus) -Test (Weizen) / protektiv

- Lösungsmittel:: 50 Gewichtsteile N,N-Dimethylacetamid
- Emulgator:: 1 Gewichtsteil Alkylarylpolyglykolether

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit Wasser auf die gewünschte Konzentration.

Zur Prüfung auf protektive Wirksamkeit besprüht man junge Pflanzen mit der Wirkstoffzubereitung in der angegebenen Aufwandmenge. Nach Antrocknen des Spritzbelages werden die Pflanzen mit Sporen mit einer Sporensuspension von ***Fusarium nivale (var.majus)*** besprüht. Die Pflanzen werden in einer Gewächshauskammer unter eine lichtdurchlässige Inkubationshaube bei 10°C und 100 % relativer Luftfeuchtigkeit gestellt.

5 Tage nach der Inokulation erfolgt die Auswertung. Dabei bedeutet 0 % ein Wirkungsgrad, der demjenigen der Kontrolle entspricht, während ein Wirkungsgrad von 100 % bedeutet, dass kein Befall beobachtet wird.

In diesem Test zeigen die erfindungsgemäßen Verbindungen 1, 3, 24, 29 aus Tabelle 1 bei einer Konzentration an Wirkstoff von 1000 ppm einen Wirkungsgrad von 70% oder mehr.

### Beispiel F

### Produktion von Fumonisin FB1 durch Fusarium proliferatum

Die verwendete Methode wurde für Mikrotiter-Platten adaptiert ausgehend von der durch Lopez-Errasquin et al beschriebenen Methode: Journal of Microbiological Methods 68 (2007) 312-317.

Fumonisin-induzierendes flüssiges Medium (Jiménez et al., Int. J. Food Microbiol. (2003), 89, 185-193) wird mit einer konzentrierten Sporen-Suspension von *Fusarium proliferatum* (350000 Sporen/ml, gelagert bei -160°C) bis zu einer endgültigen Konzentration von 2000 Sporen/ml inokuliert.

Die Verbindungen werden gelöst (10 mM in 100 % DMSO) und auf 100 µM in H2O verdünnt. Die Verbindungen werden in 7 Konzentrationen in einem Bereich von 50 µM bis 0,01 µM getestet (verdünnt ausgehend von der 100 µM Stock-Lösung in 10 % DMSO).

5 µl von jeder verdünnten Lösung werden mit 95µl inokuliertem Medium in einer Vertiefung einer 96-well-mikroarray-Platte gemischt. Die Platte wird abgedeckt und für 6 Tage bei 20°C inkubiert.

Zu Beginn und nach 6 Tagen wird eine OD-Messung (OD620 multiple read per well (square: 3 x 3)) durchgeführt, um das "pI50" Wachstum zu berechnen.

Nach 6 Tagen wird eine Probe des flüssigen Mediums genommen und in 10% Acetonitril verdünnt. Die Konzentration von FB1 der verdünnten Proben wird per HPLC-MS/MS analysiert und die Ergebnisse verwendet um die "p150 FB1" Werte zu berechnen.

HPLC-MS/MS wird mit den folgenden Parametern durchgeführt:
Instrument Massenspektrometrie: Applied Biosystems API4000 QTrap
HPLC: Agilent 1100
Autosampler: CTC HTS PAL
Chromatographiesäule: Waters Atlantis T3 (50x2mm)

### Beispiele der gemessenen pI50-Werte

### Produktion von Fumonisin FB1 durch Fusarium proliferatum

| **Verbindung** | **pI50 FB1** | **pI50 Wachstum** |
|---|---|---|
| 43 | 5.9 | 5.2 |
| 45 | 5.8 | 5.3 |
| 44 | 5.9 | 4.7 |
| 39 | 5.5 | 5.0 |
| 1 | 5.5 | 5.1 |
| 3 | 5.7 | 4.9 |
| 6 | 5.8 | 5.5 |
| 24 | 4.5 | <4.3 |
| 29 | 5.0 | <4.3 |
| 34 | 5.0 | <4.3 |
| 35 | 4.5 | 4.4 |

### Beispiel G

### Pyricularia-Test (Reis) / protektiv

- Lösungsmittel:: 28,5 Gew.-Teile Aceton
- Emulgator:: 1,5 Gew.-Teil Alkylarylpolyglykolether

Zur Herstellung einer zweckmäßigen Wirkstoffzubereitung vermischt man 1 Gewichtsteil Wirkstoff mit der angegebenen Menge Lösungsmittel und verdünnt das Konzentrat mit Wasser und der angegebenen Menge Emulgator auf die gewünschte Konzentration.

Zur Prüfung auf protektive Wirksamkeit bespritzt man junge Reispflanzen mit der Wirkstoffzubereitung in der angegebenen Aufwandmenge. 1 Tag nach der Behandlung werden die Pflanzen mit einer wässrigen Sporensuspension von *Pyricularia oryzae* inokuliert. Anschließend werden die Pflanzen in einem Gewächshaus bei 100 % relativer Luftfeuchtigkeit und 25°C aufgestellt.

7 Tage nach der Inokulation erfolgt die Auswertung. Dabei bedeutet 0 % ein Wirkungsgrad, der demjenigen der Kontrolle entspricht, während ein Wirkungsgrad von 100 % bedeutet, dass kein Befall beobachtet wird.

In diesem Test zeigen die erfindungsgemäßen Verbindungen 43 aus Tabelle 1 bei einer Konzentration an Wirkstoff von 250ppm einen Wirkungsgrad von 80% oder mehr.

### Beispiel H

### Rhizoctonia Test (Reis) / protektiv

- Lösungsmittel:: 28,5 Gew.-Teile Aceton
- Emulgator:: 1,5 Gew.-Teil Alkylarylpolyglykolether

Zur Herstellung einer zweckmäßigen Wirkstoffzubereitung vermischt man 1 Gewichtsteil Wirkstoff mit der angegebenen Menge Lösungsmittel und verdünnt das Konzentrat mit Wasser und der angegebenen Menge Emulgator auf die gewünschte Konzentration.

Zur Prüfung auf protektive Wirksamkeit bespritzt man junge Reispflanzen mit der Wirkstoffzubereitung in der angegebenen Aufwandmenge. 1 Tag nach der Behandlung werden die Pflanzen mit Hyphen von *Rhizoctonia solani* inokuliert. Anschließend werden die Pflanzen in einem Gewächshaus bei 100 % relativer Luftfeuchtigkeit und 25°C aufgestellt.

4 Tage nach der Inokulation erfolgt die Auswertung. Dabei bedeutet 0 % ein Wirkungsgrad, der demjenigen der Kontrolle entspricht, während ein Wirkungsgrad von 100 % bedeutet, dass kein Befall beobachtet wird.

In diesem Test zeigen die erfindungsgemäßen Verbindungen 34, 36 aus Tabelle 1 bei einer Konzentration an Wirkstoff von 250ppm einen Wirkungsgrad von 80% oder mehr.

### Beispiel I

### Cochliobolus Test (Reis) / protektiv

- Lösungsmittel:: 28,5 Gew.-Teile Aceton
- Emulgator:: 1,5 Gew.-Teil Alkylarylpolyglykolether

Zur Herstellung einer zweckmäßigen Wirkstoffzubereitung vermischt man 1 Gewichtsteil Wirkstoff mit der angegebenen Menge Lösungsmittel und verdünnt das Konzentrat mit Wasser und der angegebenen Menge Emulgator auf die gewünschte Konzentration.

Zur Prüfung auf protektive Wirksamkeit bespritzt man junge Reispflanzen mit der Wirkstoffzubereitung in der angegebenen Aufwandmenge. 1 Tag nach der Behandlung werden die Pflanzen mit einer wässrigen Sporensuspension von *Cochliobolus miyabeanus* inokuliert. Anschließend werden die Pflanzen in einem Gewächshaus bei 100 % relativer Luftfeuchtigkeit und 25°C aufgestellt.

4 Tage nach der Inokulation erfolgt die Auswertung. Dabei bedeutet 0 % ein Wirkungsgrad, der demjenigen der Kontrolle entspricht, während ein Wirkungsgrad von 100 % bedeutet, dass kein Befall beobachtet wird.

In diesem Test zeigen die erfindungsgemäßen Verbindungen 43 aus Tabelle 1 bei einer Konzentration an Wirkstoff von 250ppm einen Wirkungsgrad von 80% oder mehr.

### Beispiel J

### Gibberella test (Reis) / protektiv

- Lösungsmittel:: 28,5 Gew.-Teile Aceton
- Emulgator:: 1,5 Gew.-Teil Alkylarylpolyglykolether

Zur Herstellung einer zweckmäßigen Wirkstoffzubereitung vermischt man 1 Gewichtsteil Wirkstoff mit der angegebenen Menge Lösungsmittel und verdünnt das Konzentrat mit Wasser und der angegebenen Menge Emulgator auf die gewünschte Konzentration.

Zur Prüfung auf protektive Wirksamkeit bespritzt man junge Reispflanzen mit der Wirkstoffzubereitung in der angegebenen Aufwandmenge. 1 Tag nach der Behandlung werden die Pflanzen mit einer wässrigen Sporensuspension von *Gibberella zeae* inokuliert. Anschließend werden die Pflanzen in einem Gewächshaus bei 100 % relativer Luftfeuchtigkeit und 25°C aufgestellt.

4 Tage nach der Inokulation erfolgt die Auswertung. Dabei bedeutet 0 % ein Wirkungsgrad, der demjenigen der Kontrolle entspricht, während ein Wirkungsgrad von 100 % bedeutet, dass kein Befall beobachtet wird.

In diesem Test zeigen die erfindungsgemäßen Verbindungen 34 aus Tabelle 1 bei einer Konzentration an Wirkstoff von 250ppm einen Wirkungsgrad von 80% oder mehr.

## Patentansprüche

**1.** Verbindungen der Formel (I), 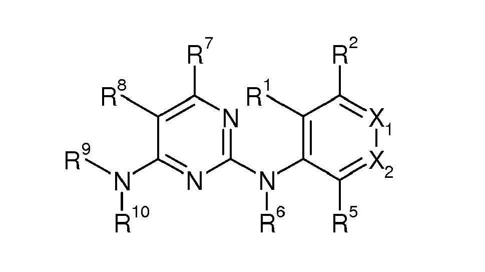 in welcher die Symbole folgende Bedeutungen haben:
X¹ ist Stickstoff oder CR³,
X² ist Stickstoff oder CR⁴,
wobei X¹ und X² nicht gleichzeitig für Stickstoff stehen,
R¹ und R⁵ sind unabhängig voneinander Wasserstoff, C₁-C₄-Alkyl, C₁-C₄-Alkoxy, OH, C₁-C₄-Halogenalkyl oder Halogen,
R² ist Wasserstoff, Halogen, Nitro, Cyano, C₁-C₄-Alkyl, C₁-C₂-Haloalkyl, Hydroxy, OR¹², OCON(R¹²)₂, C₁-C₃-Haloalkoxy, COR¹², CR¹²=NOR¹², SF₅, SR¹², SOR¹², SO₂R¹², CO₂R¹², N(R¹²)₂, NR¹²COR¹² oder NR¹²CO₂R¹³,
R³ und R⁴ sind unabhängig voneinander Wasserstoff, Halogen, Cyano, Nitro, ein 3- bis 8-gliedriger, unsubstituierter oder substituierter, gesättigter oder ungesättigter Zyklus, der keine oder bis zu vier Heteroatome, ausgewählt aus N, O und S enthalten kann, wobei zwei Sauerstoffatome nicht benachbart sind, OR¹², OSO₂N(R¹²)₂, OCON(R¹²)₂, OCOR¹³, SF₅, SR¹², SOR¹², SO₂R¹², SON(R¹²)₂, SO₂N(R¹³)₂, SO₂NHR¹⁴, SO₂NHCOR¹², C=OR¹², CH=NOR¹², CR¹³=NOR¹², COCl, CON(R¹²)₂, COOR¹², COO(CH₂)ₘOR¹², CO(CH₂)ₘCN, CO(CH₂)ₘN(R¹²)₂, NR¹²COR¹², NR¹²COOR¹³, NR¹²(C=S)R¹³, NR¹²(C=S)OR¹³, N(R¹²)₂, NR¹²(CH₂)ₘN(R¹²)₂, NR¹²SO₂R¹³, NR¹²SOR¹³, [C(R¹²)₂]ₘCN, (CH₂)ₘSON(R¹²)₂, (CH₂)ₘSO₂N(R¹²)₂, (CH₂)ₘCOOR¹², (CH₂)ₘCOR¹², [C(R¹²)₂]ₘCOR¹², [C(R¹²)₂]ₘCON(R¹²)₂, (CH₂)ₘN(R¹²)₂, (CH₂)ₘNR¹²COR¹², (CH₂)ₘNR¹²COOR¹³, [C(R¹²)₂]ₘNR¹²COR¹², [C(R¹²)₂]ₘNR¹²COOR¹³, unsubstituiertes oder substituiertes C₁-C₈-Alkyl, unsubstituiertes oder substituiertes C₂-C₆-Alkenyl, unsubstituiertes oder substituiertes C₁-C₈-Haloalkyl, unsubstituiertes oder substituiertes C₂-C₆-Alkinyl; mit m = 1 - 4,
wobei zusätzlich oder unabhängig davon R³ und R⁴, ggf. über R¹² oder R¹³, gemeinsam einen 3- bis 7-gliedrigen, unsubstituierten oder substituierten, gesättigten oder ungesättigten Zyklus bilden können, der kein oder bis zu vier Heteroatome, ausgewählt aus N, O und S enthalten kann, wobei zwei Sauerstoffatome nicht benachbart sind,
wobei die Substituenten unabhängig voneinander ausgewählt sind aus Wasserstoff, Fluor, Chlor, oder Brom, C₁-C₄-Alkyl, C₁-C₄-Alkoxy, Hydroxy, Oxo, C₁-C₄-Halogenalkyl, (C₁-C₄-Alkyl)carbonyl oder Cyano,
R⁶ ist Wasserstoff, C₁-C₄-Alkyl, C₁-C₄-Alkoxy(C₁-C₄)alkyl, Formyl, (C₁-C₄-Alkyl)carbonyl, (C₁-C₄-Alkoxy-C₁-C₄-alkyl)carbonyl, (C₃-C₆-Alkenyloxy)carbonyl, (C₃-C₆-Cycloalkyl)-carbonyl, (Halogen-C₁-C₄-alkoxy-C₁-C₄-alkyl)carbonyl, (C₁-C₄-Haloalkyl)carbonyl, (C₁-C₄-Alkoxy)carbonyl, (C₁-C₄-Haloalkoxy)carbonyl, Benzyloxycarbonyl, unsubstituiertes oder substituiertes Benzyl, unsubstituiertes oder substituiertes Benzoyl, unsubstituiertes oder substituiertes C₂-C₆-Alkenyl, unsubstituiertes oder substituiertes C₂-C₆-Alkinyl, C₁-C₂-Alkylsulfinyl oder C₁-C₂-Alkylsulfonyl,
wobei die Substituenten unabhängig voneinander ausgewählt sind aus Wasserstoff, Fluor, Chlor oder Brom, C₁-C₄-Alkyl, C₁-C₄-Alkoxy, Hydroxy, C₁-C₄-Halogenalkyl, oder Cyano,
R⁷ ist Wasserstoff, C₁-C₃-Alkyl, Cyano oder C₁-C₃-Haloalkyl,
R⁸ ist Fluor, Chlor, Brom, Iod, Cyano, Methyl, SMe, SOMe, SO₂Me, CF₃, CCl₃, CFH₂ oder CF₂H,
R⁹ ist Wasserstoff, C₁-C₃-Alkyl, C₂-C₃-Haloalkyl, C₁-C₄-Alkoxy(C₁-C₄)alkyl, (C₁-C₄-Alkyl)carbonyl, (C₁-C₄-Haloalkyl)carbonyl, (C₁-C₄-Alkoxy)carbonyl, unsubstituiertes oder substituiertes Benzyl, unsubstituiertes oder substituiertes C₂-C₄-Alkenyl, unsubstituiertes oder substituiertes C₂-C₄-Alkinyl, C₁-C₆-Alkylsulfinyl, C₁-C₆-Alkylsulfonyl, C₁-C₆-Haloalkylsulfinyl oder C₁-C₆-Haloalkylsulfonyl,
wobei die Substituenten unabhängig voneinander ausgewählt sind aus Fluor-, Chlor- und/oder Bromatomen, Cyano, Hydroxy, Methyl, Methoxy, CF₃
R¹⁰ ist eine Gruppe der Formel E1 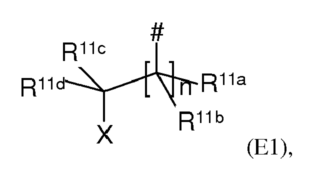
in welcher die Symbole die folgenden Bedeutungen haben und # für die Anknüpfungsstelle an das Stickstoffatom steht:
R^{11a} ist Wasserstoff oder C₁-C₄-Alkyl,
R^{11b} ist Wasserstoff, C₁-C₄-Alkyl, C₁-C₃-Halogenalkyl, (CH₂)ₘOH, (CH₂)ₘO(C₁-C₂-Alkyl), COOR¹², CON(R¹²)₂, (CH₂)ₘCOOR¹², COR¹², unsubstituiertes oder substituiertes Benzyl, unsubstituiertes oder substituiertes C₂-C₆-Alkenyl oder unsubstituiertes oder substituiertes C₂-C₆-Alkinyl,
wobei die Substituenten unabhängig voneinander ausgewählt sind aus Wasserstoff, Fluor, Chlor oder Brom, C₁-C₄-Alkyl, C₁-C₄-Alkoxy, Hydroxy, C₁-C₄-Halogenalkyl oder Cyano,
R^{11c} ist Wasserstoff, Halogen oder C₁-C₄-Alkyl,
R^{11a} ist Wasserstoff, Halogen, C₁-C₆-Alkyl, OR¹², COOR¹², (CH₂)ₘCOOR¹² oder COR¹²,
X ist Halogen oder C₁-C₆-Halogenalkyl,
mit m = 1 - 4 und n = 1 - 4
R¹² ist unabhängig voneinander gleich oder verschieden Wasserstoff, C₁-C₆-Alkyl, C₁-C₆-Haloalkyl, unsubstituiertes oder substituiertes C₃-C₆-Cycloalkyl, C₁-C₄-Trialkyl-Silyl, unsubstituiertes oder substituiertes C₂-C₄-Alkenyl, unsubstituiertes oder substituiertes C₂-C₄-Alkinyl, unsubstituiertes oder substituiertes Aryl, C₁-C₄-Alkoxy(C₁-C₄)alkyl, unsubstituiertes oder substituiertes Benzyl oder ein 3- bis 7-gliedriger, unsubstituierter oder substituierter, gesättigter oder ungesättigter Zyklus, der keine oder bis zu vier Heteroatome, ausgewählt aus N, O und S enthalten kann, wobei zwei Sauerstoffatome nicht benachbart sind
oder
für den Fall, dass zwei Reste R¹² an ein Stickstoffatom gebunden sind, können zwei Reste R¹² einen 3 bis 7-gliedrigen, unsubstituierten oder substituierten, gesättigten oder ungesättigten Zyklus bilden, der bis zu vier weitere Heteroatome, ausgewählt aus N, O und S enthalten kann, wobei zwei Sauerstoffatome nicht benachbart sind, oder
für den Fall, dass zwei Reste R¹² benachbart in der Gruppierung NR¹²COR¹² NR¹²SOR¹² NR¹²SO₂R¹² NR¹²SONR¹² NR¹²SO₂NR¹² vorliegen, können zwei Reste R¹² einen 3 bis 7-gliedrigen, unsubstituierten oder substituierten, gesättigten oder ungesättigten Zyklus, der bis zu vier weitere Heteroatome, ausgewählt aus N, O und S enthalten kann, wobei zwei Sauerstoffatome nicht benachbart sind, bilden,
R¹³ ist unabhängig voneinander gleich oder verschieden C₁-C₈-Alkyl, C₁-C₈-Haloalkyl, C₁-C₄-Trialkyl-Silyl, unsubstituiertes oder substituiertes C₂-C₆-Alkenyl, unsubstituiertes oder substituiertes C₂-C₆-Alkinyl, unsubstituiertes oder substituiertes C₃-C₆-Cycloalkyl, unsubstituiertes oder substituiertes Aryl, C₁-C₄-Alkoxy(C₁-C₄)alkyl, unsubstituiertes oder substituiertes Benzyl oder ein 3- bis 7-gliedriger, unsubstituierter oder substituierter, gesättigter oder ungesättigter Zyklus, der keine oder bis zu vier Heteroatome, ausgewählt aus N, O und S enthalten kann, wobei zwei Sauerstoffatome nicht benachbart sind,
oder
für den Fall, dass zwei Reste R¹³ an ein Stickstoffatom gebunden sind, können zwei Reste R¹³ einen einen 3 bis 7-gliedrigen, unsubstituierten oder substituierten, gesättigten oder ungesättigten Zyklus, der bis zu vier weitere Heteroatome, ausgewählt aus N, O und S enthalten kann, wobei zwei Sauerstoffatome nicht benachbart sind, bilden können,
R¹⁴ ist gleich oder verschieden C₁-C₆-Alkyl, C₁-C₈-Haloalkyl, C₁-C₄-Trialkyl-Silyl, unsubstituiertes oder substituiertes C₂-C₆-Alkenyl, unsubstituiertes oder substituiertes C₂-C₆-Alkinyl, unsubstituiertes oder substituiertes C₃-C₆-Cycloalkyl, unsubstituiertes oder substituiertes Aryl, unsubstituiertes oder substituiertes Benzyl oder ein 3- bis 7-gliedriger, unsubstituierter oder substituierter, gesättigter oder ungesättigter Zyklus, der keine oder bis zu vier Heteroatome, ausgewählt aus N, O und S enthalten kann, wobei zwei Sauerstoffatome oder zwei Stickstoffatome nicht benachbart sind,
und wobei mögliche Substituenten ausgewählt sind aus folgender Liste:
Fluor Chlor, Brom, Iod, Cyano, Nitro, CF₃, CFH₂, CF₂H, C₂F₅, CCl₃, Hydroxy, OMe, OEt, OPr, O*iso*Pr, OBu, O*sec*Bu, O*iso*Bu, O*tert*Bu, O(CH₂)₂OCH₃, O(CH₂)₃OCH₃, O-cylopentyl, O-Phenyl, OCF₃, OCF₂H, OCF₂CF₃, OCF₂CF₂H, SH, SMe, SEt, SCF₃, SCF₂H, SPh, SCF₅, SO₂Me, SO₂CF₃, SOMe, SOEt, CO₂H, CO₂CH₃, CO₂Et, CO₂Pr, CO₂*iso*Pr, CO₂*tert*Bu, COMe, COCF₃, NH₂, NHMe, NMe₂, NHEt, NEt₂, NHPr, NH*iso*Pr, NH*n*Bu, NH*tert*Bu, NH*iso*Bu, NHsecBu, cyclopropylamino, Formyl, CH₂CN, CHMeCN, CH₂COCH₃, CH₂OMe, (CH₂)₂OMe, (CH₂)₃OMe, CH₂OH, CH₂SMe, (CH₂)₂SMe, Methyl, Ethyl, Propyl, 1-Methylethyl, Butyl, 1-Methyl-propyl, 2-Methylpropyl, 1,1-Dimethylethyl, Cyclopropyl, 1-methoxycyclopropyl, 1-chlorcyclopropyl, Cyclobutyl, 3-dimethylbutyl, Cyclopentyl, Cyclohexyl, Cyclohexylmethyl, Neopentyl, prop-2-en-1-yl, 1-methylprop-2-en-1-yl, but-3-en-1-yl, trimethylsilyl)methyl, Cyclopropyl, cyclobutyl, cyclopentyl, cyclohexyl, Phenyl, Benzyl,-CH₂CH=CH₂, -CH(CH₃)CH=CH₂, -CH₂C≡CH,
sowie die agrochemisch wirksamen Salze davon.

**2.** Verbindungen der Formel (I) nach Anspruch 1,
in denen ein oder mehrere der Symbole eine der folgenden Bedeutungen haben:
X¹ ist Stickstoff oder CR³,
X² ist Stickstoff oder CR⁴,
wobei X¹ und X² nicht gleichzeitig für Stickstoff stehen
R¹ und R⁵ sind unabhängig voneinander H, C₁-C₂-Alkyl, C₁-C₂-Alkoxy, OH, CF₃, F, Cl oder Br
R² ist Wasserstoff, Halogen, Nitro, Cyano, C₁-C₄-Alkyl, C₁-C₂-Haloalkyl, Hydroxy, C₁-C₄-Alkoxy, O(C₂-C₄-Alkenyl), O(C₂-C₄-Alkinyl), OCONH(C₁-C₄-Alkyl), OCON(C₁-C₄-Alkyl)₂, C₁-C₃-Haloalkoxy, COH, CO(C₁-C₄-Alkyl), C(=NOH)Me, C(=NO(C₁-C₄-Alkyl)Me, CH(=NO(C₁-C₄-Alkyl), SF₅, S(C₁-C₃)-Alkyl, S(C₁-C₂)Haloalkyl, SO(C₁-C₄-Alkyl), SO₂(C₁-C₄-Alkyl), CO₂(C₁-C₃-Alkyl), NH₂, NH(C₁-C₄-Alkyl), N(C₁-C₄-Alkyl)₂, NHCO(C₁-C₄-Alkyl), NHCOH, NMeCO(C₁-C₄-Alkyl), NHCO₂(C₁-C₄-Alkyl),
R³ und R⁴ sind unabhängig voneinander Wasserstoff, Halogen, Cyano, Nitro, Hydroxy, O-C₁-C₄-Alkyl, O-(C₁-C₃-Haloalkyl), O-(C₃-C₆-Cycloalkyl), O-C₂-C₄-Alkenyl, O-C₂-C₄-Alkinyl, OPh, OSO₂N(C₁-C₄-Alkyl)₂, OCONH(C₁-C₄-Alkyl), OCON(C₁-C₄-Alkyl)₂, OCO(C₁-C₄-Alkyl), SF₅, SH, S-C₁-C₄-Alkyl, S-C₁-C₃-Haloalkyl, SPh, SO(C₁-C₄-Alkyl), SO₂(C₁-C₄-Alkyl), SO₂(C₁-C₃-Haloalkyl), SO₂(C₂-C₄-Alkenyl), SO₂(C₂-C₄-Alkinyl), SONH(C₁-C₄-Alkyl), SON(C₁-C₄-Alkyl)₂, SO₂N(C₁-C₄-Alkyl)₂, SO₂NHCO(C₁-C₄-Alkyl), SO₂NHPh, SO₂NH(C₂-C₄-Alkenyl), Formyl, (C₁-C₄-Alkyl)carbonyl, (C₁-C₃-Haloalkyl)carbonyl, CH=NO(C₁-C₄-Alkyl), C(C₁-C₄-Alkyl)=NO(C₁-C₄-Alkyl), CO(CH₂)ₘCN, CONH(C₁-C₄-Alkyl), CON(C₁-C₄-Alkyl)₂, CONH(C₁-C₃-Haloalkyl), CONH(C₃-C₄-Alkenyl), CONH(C₃-C₄-Alkinyl), CONH(C₃-C₆-Cycloalkyl), CONHCH(CH₃)CH₂O(C₁-C₄-Alkyl), CONH(CH₂)ₘO(C₁-C₄-Alkyl), CONHPh, COCH₂N(C₁-C₄-Alkyl)₂, Piperidin-1-ylcarbonyl, Morpholin-4-ylcarbonyl, (4-Methylpiperazin-1-yl)carbonyl, Pyrrolidin-1-ylcarbonyl, Azetidin-1-yl-carbonyl, Aziridin-1-yl-carbonyl, Hexamethylenimin-1-yl-carbonyl, Morpholin-1-ylcarbonyl, Thiomorpholin-1-ylcarbonyl, COOH, (C₁-C₄-Alkoxy)carbonyl, CO₂(CH₂)ₘO(C₁-C₄-Alkyl), NHCO(C₁-C₄-Alkyl), NHCO(C₁-C₄-Haloalkyl), N(C₁-C₂-Alkyl)CO(C₁-C₄-Alkyl), NHCO(C₂-C₄-Alkenyl), NHCOPh, NHCOC((C₁-C₄-Alkyl)₂CH₂Hal, NHCO(C=CH₂)CH₃, NHCO(CH₂)ₘO(C₁-C₄-Alkyl), NHCHO, N(C₁-C₄-Alkyl)CHO, NHCO₂(C₁-C₄-Alkyl), NHCO₂Ph, NHCO₂CH₂CH₂Hal, N(C₁-C₄-Alkyl)CO₂(C₁-C₄-Alkyl), NH(C=S)O(C₁-C₄-Alkyl), NH₂, NH(C₁-C₄-Alkyl), N(C₁-C₄-Alkyl)₂, cyclopropylamino, NHCH(C₁-C₄-Alkyl)CH₂O(C₁-C₄-Alkyl), acetyl(cyclopropyl)amino, [(1-methylcyclopropyl)carbonyl]-amino, morpholin-1-yl, morpholin-4-yl-methyl, NHSO₂(C₁-C₄-Alkyl), NHSO₂(C₁-C₃-Haloalkyl), CH₂CN, CH(C₁-C₄-Alkyl)CN, (CH₂)ₘSO₂NH(C₁-C₄-Alkyl), (CH₂)ₘSO₂N(C₁-C₄-Alkyl)₂, (CH₂)ₘCO(C₁-C₄-Alkyl), CH(C₁-C₄-Alkyl)CO(C₁-C₄-Alkyl), (CH₂)ₘCOcyclopropyl, (CH₂)ₘCO₂(C₁-C₄-Alkyl), (CH₂)ₘCONH(C₁-C₄-Alkyl), (CH₂)ₘCON(C₁-C₃-Alkyl)₂, (CH₂)ₘNHCOO(C₁-C₄-Alkyl), CH₂NHCOOBn, (CH₂)ₘNHCO(C₁-C₄-Alkyl), (CH₂)ₘNHCO(C₁-C₃-Haloalkyl), C₁-C₅-Alkyl, C₃-C₆-Cycloalkyl, 1-methoxycyclopropyl, 1-chlorcyclopropyl, Cyclopenten(1)yl, 2-Oxocyclopentyl C₂-C₆-Alkenyl, C₁-C₃-Haloalkyl, morpholin-4-ylsulfonyl, [(4,6-dimethylpyrimidin-2-yl)amino]sulfonyl, 1H-tetrazol-5-yl, (cyclo-propylcarbonyl)amino, (2-furoyl-amino), (3-methyl-2,5-dioxoimidazolidin-1-yl), (piperidin-1-ylethyl)amino, cyclopropyl(trifluor-acetyl)amino, (1-methylcyclopropyl)carbonylamino, 4,4-dimethyl-2,5-dioxoimidazolidin-1-yl, 2,3-dimethyl-5-oxo-2,5-dihydro-1H-pyrazol-1-yl, 5-thioxo-4,5-dihydro-1H-tetrazol-1-yl, 3-methyl-2-oxoimidazol-idin-1-yl, 3-(1-methylethyl)-2-oxoimidazolidin-1-yl, 3-(2-methylpropyl)-2-oxoimidazol-idin-1-yl, 2-oxo-3-prop-2-en-1-ylimidazolidin-1-yl, 3-tert-butyl-2-oxoimidazolidin-1-yl, pyrrolidin-1-ylsulfonyl, 2,5-dioxoimidazolidin-4-yl, piperidin-1-ylsulfonyl, 1,3-thiazol-2-yl, 1,3-thiazol-4-yl, (morpholin-4-ylsulfonyl)methyl, (Dimethylsulfamoyl)methyl, (piperidin-1-ylsulfonyl)methyl, [(4-methyl-phenyl)amino]sulfonyl, (pyrrolidin-1-ylsulfonyl)methyl, 2-oxoimidazolidin-1-yl, 3-methyl-5-oxo-4,5-dihydro-1H-pyrazol-1-yl, (3,4-dimethyl-5-oxo-4,5-dihydro-1H-pyrazol-1-yl, (1-methylcyclopentyl), pyrrolidin-1-yl, piperidin-1-yl, 2-oxo-2,5-dihydro-1H-pyrrol- 1-yl, 3,3-dimethyl-2-oxocyclopentyl, 3-Oxo-4,5-dimethyl-2,4-dihydro-pyrazol-2-yl-, 3-Oxo-4-ethyl-5-methyl-2,4-dihydro-pyrazol-2-yl-, 3-Oxo-5-trifluormethyl-2,4-dihydro-pyrazol-2-yl-, 3-Oxo-5-isopropyl-2,4-dihydro-pyrazol-2-yl-, 3,5-Dioxo-4,4-dimethyl-pyrazolidin-1-yl, 3,5-Dioxo-4-ethyl-pyrazolidin-1-yl, 3-Oxo-4,4-dimethyl-pyrazolidin-1-yl, 2,5-dioxo-2,5-dihydro-1H-pyrrol-1-yl, 3,5-Dimethylpiperidin-1-yl, 4-(tert-butoxy-carbonyl)piperazin-1-yl, 5-Ethoxy-3,4-dimethyl-1H-pyrazol-1-yl, Acetyl(cyclo-hexyl)amino, 2-Furoylamino, 2,2,2-trifluor-ethyl)carbamoyl, 5-Ethoxy-3-(trifluormethyl)-1H-pyrazol-1-yl 3-(2-Chlorethyl)-2-oxoimidazolidin-1-yl, 3-oxobutyl, 1,1-dioxidoisothiazolidin-2-yl, 1,1-dioxido-tetrahydrothiophen-2-yl, 5-methyl-1,1-dioxido-1,2,5-thiadiazolidin-2-yl, 4-methoxy-2-oxo-2,5-dihydro-1H-pyrrol-1-yl, 2-oxo-2,5-dihydro-1H-pyrrol-1-yl, 5-oxo-4,5-dihydro-1H-imidazol-1-yl, 4-methyl-5-oxo-4,5-dihydro-1H-1,2,4-triazol-1-yl, 3-methyl-5-oxo-2,5-dihydro-1H-pyrazol-1-yl, 2-(methoxymethyl)pyrrolidin-1-yl, 2-oxocyclopentyl, 2-oxotetrahydrofuran-3-yl, 1-methyl-3-oxo-2,3-dihydro-1H-pyrazol-4-yl, 1-methyl-3-oxopyrazolidin-4-yl, tetrahydro-furan-2-yl, furan-2-yl, 1,1-dioxido-1,2-thiazinan-2-yl, 5-methyl-1,1-dioxido-1,2,5-thiadiazoli-din-2-yl, 6-methyl-1,1-dioxido-1,2,6-thiadiazinan-2-yl,
mit m = 1 - 3
und für den Fall, dass R³ und R⁴, ggf. über R¹² oder R¹³ einen Zyklus bilden, kann folgende Untereinheit aus der allgemeinen Formel (I): 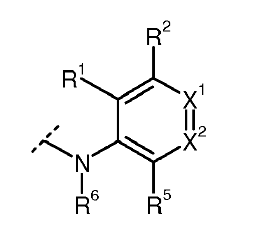 (2-oxo-2,3-dihydro-1H-indol-5-yl)amino, 1H-indol-6-ylamino, 1H-indol-5-ylamino, 2-(trifluormethyl)-1H-benzimidazol-6-yl]amino, (3-methyl-1,1-dioxido-2H-1,2,4-benzothiadiazin-7-yl)amino, (1,1-dioxido-2H-1,2,4-benzothiadiazin-6-yl)amino, (4-methyl-3-oxo-3,4-dihydro-2H-1,4-benzoxazin-6-yl)amino, (4-methyl-3-oxo-3,4-dihydro-2H-1,4-benzoxazin-7-yl)amino, (1-acetyl-2,3-dihydro-1H-indol-6-yl)amino, (4H-1,3-benzodioxin-7-ylamino, (2-oxo-2,3,4,5-tetra-hydro-1H-1-benzazepin-8-yl)amino, (2,2-dioxido-1,3-dihydro-2-benzothien-5-yl)amino, (1-oxo-2,3-dihydro-1H-inden-5-yl)amino, [2-(ethylsulfonyl)-2,3-dihydro-1,3-benzothiazol-6-yl]ami-no, (2,2,3,3-tetrafluor-2,3-dihydro-1,4-benzodioxin-6-yl)amino, 1,3-benzodioxol-5-ylamino, (1,3-dioxo-2,3-dihydro-1H-isoindol-5-yl)amino, 2-methyl-1,3-benzothiazol-6-yl)amino, (2-oxo-2,3-dihydro-1H-benzimidazol-5-yl)amino, (2-oxo-1,3-benzoxathiol-5-yl)amino, 2-oxo-2,3-dihydro-1,3-benzoxazol-5-yl)amino, (2-ethyl-1,3-benzoxazol-5-yl)amino, 2-oxo-1,2,3,4-tetrahy-droqui-nolin-6-yl)amino, (3-oxo-3,4-dihydro-2H-1,4-benzoxazin-6-yl)amino, (2-oxo-2,3-dihydro-1,3-benzoxazol-6-yl)amino, 3-oxo-1,3-dihydro-2-benzofuran-5-yl)amino, [2-(ethylsulfonyl)-1,3-benzo-thiazol-6-yl]amino, (2-methyl-1,3-benzothiazol-5-yl)amino, (1-acetyl-2,3-dihydro-1H-indol-5-yl)amino, (2,2,3,3-tetrafluoro-2,3-dihydro-1,4-benzodioxin-6-yl)amino, (2,2-dioxido-1,3-dihydro-2-benzothiophen-5-yl)amino, (2-oxo-2,3-dihydro-1H-indol-6-yl)amino, (2-oxo-1,2,3,4-tetrahydro-quinolin-7-yl)amino, 1H-indazol-6-ylamino sein,
R⁶ ist Wasserstoff, C₁-C₃-Alkyl, C₁-C₂-Alkoxy(C₁-C₂)alkyl, Formyl, (C₁-C₄-Alkyl)carbonyl, (C₁-C₄-Haloalkyl)carbonyl, (C₁-C₄-Alkoxy)carbonyl, Benzyloxycarbonyl, Benzyl, 4-Methoxybenzyl, 2,4-Dimethoxybenzyl, 2-Hydroxybenzyl, unsubstituiertes oder substituiertes Benzoyl, unsubstituiertes oder substituiertes C₂-C₄-Alkenyl, unsubstituiertes oder substituiertes C₂-C₄-Alkinyl, C₁-C₂-Alkylsulfinyl oder C₁-C₂-Alkylsulfonyl,
wobei die Substituenten unabhängig voneinander ausgewählt sind aus Wasserstoff, Fluor, Chlor oder Brom, C₁-C₂-Alkyl, C₁-C₂-Halogenalkyl oder Cyano,
R⁷ ist Wasserstoff, Methyl, Cyano, CF₃, CFH₂ oder CF₂H,
R⁸ ist Fluor, Chlor, Brom, Iod, Cyano, Methyl, SMe, SOMe, SO₂Me, CF₃, CFH₂ oder CF₂H,
R⁹ ist Wasserstoff, C₁-C₃-Alkyl, C₂-C₃-Haloalkyl, C₁-C₂-Alkoxy(C₁-C₂)alkyl, COMe, COCF₃, COOMe, COOEt, COO*tert*Bu, Benzyl, 4-Methoxybenzyl, CH₂CH=CH₂ oder CH₂C≡CH,
R¹⁰ ist eine Gruppe der Formel E1 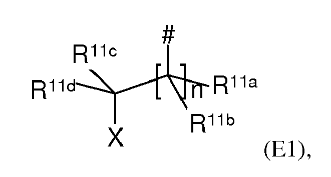
in welcher die Symbole die folgenden Bedeutungen haben und # für die Anknüpfungsstelle an das Stickstoffatom steht:
R^{11a} ist Wasserstoff oder Methyl,
R^{11b} ist Wasserstoff, C₁-C₄-Alkyl, C₁-Haloalkyl, (CH₂)ₘOH, (CH₂)ₘO(C₁-C₂-Alkyl), COO(C₁-C₂-Alkyl), CH₂COO(C₁-C₂-Alkyl) CO(C₁-C₄-Alkyl), Benzyl oder C₂-C₄-Alkenyl,
mit m = 1 - 2
R^{11c} ist Wasserstoff, Halogen oder Methyl,
R^{11d} ist Wasserstoff, Halogen, Methyl, Ethyl, Propyl, Hydroxy, OCH₃, OCH₂CH₃, OCF₃, COOMe oder COOEt,
X ist Halogen oder C₁-C₄-Halogenalkyl,
mit n = 1
oder für R^{11a} und R^{11b} = Wasserstoff kann n = 1-4 sein;
sowie die agrochemisch wirksamen Salze davon.

**3.** Verbindungen der Formel (I) nach einem oder mehreren der Ansprüche 1 bis 2,
in denen ein oder mehrere der Symbole eine der folgenden Bedeutungen haben:
X¹ ist Stickstoff oder CR³,
X² ist Stickstoff oder CR⁴,
wobei X¹ und X² nicht gleichzeitig für Stickstoff stehen,
R¹ und R⁵ sind unabhängig voneinander H, C₁-C₂-Alkyl, C₁-C₂-Alkoxy, F, Cl oder Br,
R² ist Wasserstoff, Fluor, Chlor, Brom, Iod, Nitro, Cyano, C₁-C₃-Alkyl, C₁-C₂-Haloalkyl, Hydroxy, C₁-C₃-Alkoxy, OCH₂CH=CH₂, OCH₂C≡CH, OCONHMe, C₁-C₂-Haloalkoxy, COMe, COH, COEt, CMe(=NOH), CMe(=NOMe), SF₅, S(C₁-C₃)-Alkyl, SCF₃, SCF₂H, SOMe, SO₂Me, CO₂(C₁-C₃-Alkyl), NH₂, NH(C₁-C₂-Alkyl), N(C₁-C₂-Alkyl)₂, NHCO(C₁-C₃-Alkyl), NHCOH, NMeCO(C₁-C₃-Alkyl) oder NHCO₂(C₁-C₃-Alkyl),
R³ und R⁴ sind unabhängig voneinander Wasserstoff, Halogen, Cyano, Nitro, Hydroxy, O-C₁-C₄-Alkyl, O-cylopentyl, OCF₃, OCF₂H, OCF₂CF₃, OCF₂CF₂H, OCONH(C₁-C₃-Alkyl), OCON(C₁-C₃-Alkyl)₂, OCO(C₁-C₄-Alkyl), OSO₂N(CH₃)₂, SH, SF₅, S-C₁-C₃-Alkyl, SCF₃, SCF₂H, SPh, SOMe, SONHMe, SONMe₂, SO₂Me, SO₂CF₃, SO₂CH₂CH=CH₂, SO₂CH₂C≡CH, SO₂N(C₁-C₄-Alkyl)₂, SO₂NHAc, SO₂NHPh, CO(C₁-C₄-Alkyl), COCHF₂, COCF₃, COCH₂CN, CONH(C₁-C₄-Alkyl), CON(C₁-C₄-Alkyl)₂, CONHCH₂CF₃, CONHCH₂CH=CH_{2,} CONHCH₂C≡CH_{,} CONHCH₂C(=CH₂)CH_{3,} CONHCH(CH₃)CH₂OCH₃, CONH(CH₂)₂OCH₃, CONHPh, COCH₂NMe₂, CONHcyclo-propyl, Piperidin-1-ylcarbonyl, Morpholin-4-ylcarbonyl, (4-Methylpiperazin-1-yl)carbonyl, Formyl, COOH, (C₁-C₃-Alkoxy)carbonyl, CO₂(CH₂)₂OCH₃, NHCO(C₁-C₄-Alkyl), NHCOCH=CH₂, NHCOPh, NHCOCF₃, NHCOC(CH₃)₂CH₂F, NHCOC(CH₃)₂CH₂Cl, NHCO(C=CH₂)CH₃, NHCOCH₂OCH₃, NHCO(CH₂)₂OCH₃, N(C₁-C₂-Alkyl)CO(C₁-C₄-Alkyl), NHCHO, NMeCHO, NHCO₂(C₁-C₄-Alkyl), NHCO₂Ph, NHCO₂CH₂CH₂Cl, N(C₁-C₂-Alkyl)CO₂(C₁-C₂-Alkyl), NH(C=S)OMe, NH₂, NH(C₁-C₄-Alkyl), N(C₁-C₂-Alkyl)₂, cyclopropylamino, acetyl(cyclopropyl)amino, [(1-methylcyclopropyl)carbonyl]amino, morpholin-1-yl, morpholin-4-yl-methyl, NHSO₂Me, NHSO₂CF₃, CH₂CN, CHMeCN, CH₂SO₂NH(C₁-C₄-Alkyl), CH₂SO₂H(C₁-C₄-Alkyl)₂, CH₂COCH₃, CH₂CO*tert*Bu, CH(CH₃)COCH₃, CH₂COCH(CH₃)₂, CH₂COcyclopropyl, CH₂CONH*tert*Bu, CH₂NHCOO(C₁-C₄-Alkyl), CH₂NHCOOBn, CH=NOMe, C(CH₃)=NOMe, CH=NOEt, C(CH₃)=NOEt, CH₂NHAc, CH₂NHCOCF₃, CH₂COOCH₃, CH₂COOEt, C₁-C₄-Alkyl, C₃-C₆-Cycloalkyl, 1-methoxycyclopropyl, 1-chlorcyclopropyl, 3-dimethylbutyl, C₂-C₆-Alkenyl, C₁-C₂-Haloalkyl, morpholin-4-ylsulfonyl, [(4,6-dimethylpyrimidin-2-yl)amino]sulfonyl, 1H-tetrazol-5-yl, (cyclo-propyl-carbonyl)amino, (2-furoylamino), (3-methyl-2,5-dioxoimidazolidin-1-yl), (piperidin-1-ylethyl)amino, cyclopropyl(trifluor-acetyl)amino, (1-methylcyclo-propyl)carbonylamino, 4,4-dimethyl-2,5-dioxoimidazolidin-1-yl, 2,3-dimethyl-5-oxo-2,5-dihydro-1H-pyrazol-1-yl, 5-thioxo-4,5-dihydro-1H-tetrazol-1-yl, 3-methyl-2-oxoimida-zolidin-1-yl, 3-(1-methylethyl)-2-oxoimidazolidin-1-yl, 3-(2-methylpropyl)-2-oxoimidazo-lidin-1-yl, 2-oxo-3-prop-2-en-1-ylimidazolidin-1-yl, 3-tert-butyl-2-oxoimidazolidin-1-yl, pyrrolidin-1-ylsulfonyl, 2,5-dioxoimidazolidin-4-yl, piperidin-1-ylsulfonyl, 1,3-thiazol-2-yl, 1,3-thiazol-4-yl, (Dimethylsulfamoyl)methyl, (morpholin-4-ylsulfonyl)methyl, (piperidin-1-ylsulfonyl)methyl, [(4-methylphenyl)amino]sulfonyl, (pyrrolidin-1-ylsulfonyl)methyl, 2-oxoimidazolidin-1-yl, 3-methyl-5-oxo-4,5-dihydro-1H-pyrazol-1-yl, (3,4-dimethyl-5-oxo-4,5-dihydro-1H-pyrazol-1-yl, (1-methylcyclopentyl), pyrrolidin-1-yl, piperidin-1-yl, 2-oxo-2,5-dihydro-1H-pyrrol-1-yl, 3,3-dimethyl-2-oxocyclopentyl, 3-Oxo-4,5-dimethyl-2,4-dihydro-pyrazol-2-yl-, 3-Oxo-4-ethyl-5-methyl-2,4-dihydro-pyrazol-2-yl-, 3-Oxo-5-trifluormethyl-2,4-dihydro-pyrazol-2-yl-, 3-Oxo-5-isopropyl-2,4-dihydro-pyrazol-2-yl-, 3,5-Dioxo-4,4-dimethyl-pyrazolidin-1-yl, 3,5-Dioxo-4-ethyl-pyrazolidin-1-yl, , 3-Oxo-4,4-dimethyl-pyrazolidin-1-yl, 2,5-dioxo-2,5-dihydro-1H-pyrrol-1-yl, 3,5-Dimethylpiperidin-1-yl, 4-(tert-butoxycarbonyl)piperazin-1-yl, 5-Ethoxy-3,4-dimethyl-1H-pyrazol-1-yl, Acetyl(cyclo-hexyl)amino, 2-Furoylamino, 2,2,2-trifluorethyl)carbamoyl, 5-Ethoxy-3-(trifluormethyl)-1H-pyrazol-1-yl, 3-(2-Chlorethyl)-2-oxoimidazolidin-1-yl, 3-oxobutyl, 1,1-dioxido-isothiazolidin-2-yl, 1,1-dioxidotetrahydrothiophen-2-yl, 5-methyl-1,1-dioxido-1,2,5-thiadiazolidin-2-yl, 4-methoxy-2-oxo-2,5-dihydro-1H-pyrrol-1-yl, 2-oxo-2,5-dihydro-1H-pyrrol-1-yl, 5-oxo-4,5-dihydro-1H-imidazol-1-yl, 4-methyl-5-oxo-4,5-dihydro-1H-1,2,4-triazol-1-yl, 3-methyl-5-oxo-2,5-dihydro-1H-pyrazol-1-yl, 2-(methoxymethyl-)pyrrolidin-1-yl, 2-oxocyclopentyl, 2-oxo-tetrahydrofuran-3-yl, 1-methyl-3-oxo-2,3-dihydro-1H-pyrazol-4-yl, 1-methyl-3-oxo-pyrazolidin-4-yl, tetrahydrofuran-2-yl, furan-2-yl, 1,1-dioxido-1,2-thiazinan-2-yl, 5-methyl-1,1-dioxido-1,2,5-thiadiazol-idin-2-yl, 6-methyl-1,1-dioxido-1,2,6-thiadiazinan-2-yl.
Und für den Fall, dass je R³ und R⁴, ggf. über R¹² oder R¹³ einen Zyklus bilden, kann folgende Untereinheit aus der allgemeinen Formel (I): 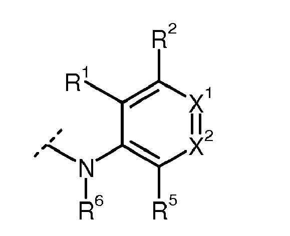 (2-oxo-2,3-dihydro-1H-indol-5-yl)amino, 1H-indol-6-ylamino, 1H-indol-5-ylamino, 2-(trifluormethyl)-1H-benzimidazol-6-yl]amino, (3-methyl-1,1-dioxido-2H-1,2,4-benzothiadiazin-7-yl)amino, (1,1-dioxido-2H-1,2,4-benzothiadiazin-6-yl)amino, (4-methyl-3-oxo-3,4-dihydro-2H-1,4-benzoxazin-6-yl)amino, (4-methyl-3-oxo-3,4-dihydro-2H-1,4-benzoxazin-7-yl)amino, (1-acetyl-2,3-dihydro-1H-indol-6-yl)amino, (4H-1,3-benzodioxin-7-ylamino, (2-oxo-2,3,4,5-tetra-hydro-1H-1-benzazepin-8-yl)amino, (2,2-dioxido-1,3-dihydro-2-benzothien-5-yl)amino, (1-oxo-2,3-dihydro-1H-inden-5-yl)amino, [2-(ethylsulfonyl)-2,3-dihydro-1,3-benzothiazol-6-yl]ami-no, (2,2,3,3-tetrafluor-2,3-dihydro-1,4-benzodioxin-6-yl)amino, 1,3-benzodioxol-5-ylamino, (1,3-dioxo-2,3-dihydro-1H-isoindol-5-yl)amino, 2-methyl-1,3-benzothiazol-6-yl)amino, (2-oxo-2,3-dihydro-1H-benzimidazol-5-yl)amino, (2-oxo-1,3-benzoxathiol-5-yl)amino, 2-oxo-2,3-dihydro-1,3-benzoxazol-5-yl)amino, (2-ethyl-1,3-benzoxazol-5-yl)amino, 2-oxo-1,2,3,4-tetrahydro-quino-lin-6-yl)amino, (3-oxo-3,4-dihydro-2H-1,4-benzoxazin-6-yl)amino, (2-oxo-2,3-dihydro-1,3-benz-oxazol-6-yl)amino, 3-oxo-1,3-dihydro-2-benzofuran-5-yl)amino, [2-(ethylsulfonyl)-1,3-benzothia-zol-6-yl]amino, (2-methyl-1,3-benzothiazol-5-yl)amino, (1-acetyl-2,3-dihydro-1H-indol-5-yl)ami-no, (2,2,3,3-tetrafluoro-2,3-dihydro-1,4-benzodioxin-6-yl)amino, (2,2-dioxido-1,3-dihydro-2-ben-zothiophen-5-yl)amino, (2-oxo-2,3-dihydro-1H-indol-6-yl)amino, (2-oxo-1,2,3,4-tetrahydroquino-lin-7-yl)amino, 1H-indazol-6-ylamino sein,
R⁶ ist Wasserstoff, C₁-C₃-Alkyl, CH₂OCH₃, CH₂CH₂OCH₃, Formyl, (C₁-C₄-Alkyl)carbonyl, (C₁-C₄-Haloalkyl)carbonyl, (C₁-C₄-Alkoxy)carbonyl, Benzyloxycarbonyl, Benzyl, 4-Methoxybenzyl, 2,4-Dimethoxybenzyl, 2-Hydroxybenzyl, unsubstituiertes oder substituiertes Benzoyl, unsubstituiertes oder substituiertes C₂-C₄-Alkenyl, unsubstituiertes oder substituiertes C₂-C₄-Alkinyl, C₁-C₂-Alkylsulfinyl oder C₁-C₂-Alkylsulfonyl,
wobei die Substituenten unabhängig voneinander ausgewählt sind aus Wasserstoff, Fluor, Chlor oder Brom, C₁-C₂-Alkyl, C₁-C₂-Halogenalkyl oder Cyano,
R⁷ ist Wasserstoff, Methyl, Cyano, CF₃ oder CFH₂,
R⁸ ist Fluor, Chlor, Brom, Iod, Cyano, Methyl, SMe, SOMe, SO₂Me, CF₃, CFH₂ oder CF₂H,
R⁹ ist Wasserstoff, C₁-C₃-Alkyl, CH₂CH₂OCH₃, CH₂OCH₃, COMe, COCF₃, COOMe, COOEt, Benzyl, 4-Methoxybenzyl, CH₂CH=CH₂ oder CH₂C≡CH,
R¹⁰ ist eine Gruppe der Formel E1 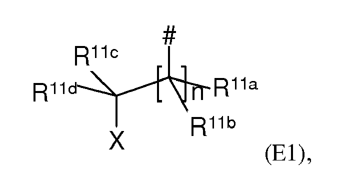
in welcher die Symbole die folgenden Bedeutungen haben und # für die Anknüpfungsstelle an das Stickstoffatom steht:
R^{11a} ist Wasserstoff oder Methyl,
R^{11b} ist Wasserstoff, Methyl, Ethyl, Propyl, 1-Methylethyl, Butyl, 1-Methylpropyl, 2-Methylpropyl, CH₂F, CH₂OH, CH₂CH₂OH, CH₂OEt, COOMe, COOEt, CH₂COOMe, CH₂COOEt, COMe, Benzyl oder Allyl,
R^{11c} ist Wasserstoff, Halogen oder Methyl,
R^{11d} ist Wasserstoff, Halogen, Methyl, Hydroxy oder COOMe,
X ist Halogen oder C₁-C₃-Halogenalkyl,
mit n = 1
oder für R^{11a} und R^{11b} = Wasserstoff kann n = 1-3 sein;
sowie die agrochemisch wirksamen Salze davon.

**4.** Verbindungen der Formel (I) nach einem oder mehreren der Ansprüche 1 bis 3,
in denen ein oder mehrere der Symbole eine der folgenden Bedeutungen haben:
X¹ CR³,
X² CR⁴,
R¹ und R⁵ sind unabhängig voneinander Wasserstoff, Methyl, Methoxy oder F,
R² ist Wasserstoff, Fluor, Chlor, Brom, Cyano, Methyl, Ethyl, Propanyl, Propan-2-yl, CF₃, Hydroxy, OMe, OEt, OCF₃, COMe, COEt, SMe, SEt, CO₂CH₃, CO₂Et, NHMe, NMe₂, NHEt, NEt₂ oder NHCOMe,
R³ und R⁴ sind unabhängig voneinander Wasserstoff, Halogen, Cyano, Nitro, Hydroxy, O-C₁-C₄-Alkyl, OCF₃, OCF₂H, OCF₂CF₃, OCF₂CF₂H, OCONH(C₁-C₃-Alkyl), OCO(C₁-C₄-Alkyl), SH, SF₅, S-C₁-C₃-Alkyl, SCF₃, SCF₂H, SPh, SOMe, SO₂Me, SO₂CF₃, SO₂CH₂CH=CH₂, SO₂CH₂C≡CH, SO₂N(C₁-C₄-Alkyl)₂, SO₂NHAc, SO₂NHPh, Formyl, CO(C₁-C₄-Alkyl), COCHF₂, COCF₃, COCH₂CN, CONH(C₁-C₄-Alkyl), CON(C₁-C₄-Alkyl)₂, CONHCH₂CF₃, CONHCH₂CH=CH₂, CONHCH₂C≡CH, CONHCH₂C(=CH₂)CH₃, CONHPh, CONHcyclopropyl, piperidin-1-ylcarbonyl, morpholin-4-ylcarbonyl, (4-methylpiperazin-1-yl)carbonyl, (C₁-C₃-Alkoxy)carbonyl, CO₂(CH₂)₂OCH₃, NHCO(C₁-C₄-Alkyl), NHCOCF₃, NHCOCH=CH₂, NHCOPh, NHCOC(CH₃)₂CH₂F, NHCO(C=CH₂)CH₃, NHCOCH₂OCH₃, NHCO(CH₂)₂OCH₃, N(CH₃)COCH₃, N(C₂H₅)COCH₃, N(CH₃)COC(CH₃)₃, NHCHO, NMeCHO, NHCO₂(C₁-C₄-Alkyl), NHCO₂Ph, NEtCO₂Me, NMeCO₂Me, NH(C=S)OMe, NH(C₁-C₄-Alkyl), N(C₁-C₂-Alkyl)₂, cyclopropylamino, acetyl(cyclopropyl) amino, [(1-methylcyclopropyl)carbonyl] amino, morpholin-1-yl, morpholin-4-yl-methyl, NHSO₂Me, NHSO₂CF₃, CH₂CN, CHMeCN, CH₂SO₂NH(C₁-C₄-Alkyl), CH₂SO₂N(C₁-C₄-Alkyl)₂, CH₂COCH₃, CH₂CO*tert*Bu, CH(CH₃)COCH₃, CH₂COCH(CH₃)₂, CH₂COcyclopropyl, CH₂CONH*tert*Bu, CH₂NHCOO(C₁-C₄-Alkyl), CH=NOMe, C(CH₃)=NOMe, CH=NOEt, C(CH₃)=NOEt, CH₂NHCOCF₃, CH₂COOCH₃, CH₂COOEt, C₁-C₄-Alkyl, C₃-C₆-Cycloalkyl, prop-2-en-1-yl, 1-methylprop-2-en-1-yl, but-3-en-1-yl, CF₃, CF₂H, C₂F₅, morpholin-4-ylsulfonyl, [(4,6-dimethylpyrimidin-2-yl)amino]sulfonyl, 1H-tetrazol-5-yl, (cyclo-propylcarbonyl)amino, (2-furoyl-amino), (3-methyl-2,5-dioxoimidazolidin-1-yl), cyclopropyl(trifluor-acetyl)amino, (1-methylcyclo-propyl)carbonylamino, 4,4-dimethyl-2,5-dioxoimidazolidin-1-yl, 2,3-dimethyl-5-oxo-2,5-dihydro-1H-pyrazol-1-yl, 3-methyl-2-oxoimidazolidin-1-yl, 3-(1-methylethyl)-2-oxoimidazolidin-1-yl, 3-(2-methylpropyl)-2-oxoimidazolidin-1-yl, 3-tert-butyl-2-oxoimidazolidin-1-yl, pyrrolidin-1-ylsulfonyl, 2,5-dioxoimidazolidin-4-yl, piperidin-1-ylsulfonyl, (Dimethylsulfamoyl)methyl, 1,3-thiazol-2-yl, 1,3-thiazol-4-yl, (morpholin-4-ylsulfonyl)methyl, (piperidin-1-ylsulfonyl)methyl, [(4-methylphenyl)amino]sulfonyl,-(pyrrolidin-1-ylsulfonyl)methyl, 2-oxoimidazolidin-1-yl, 3-methyl-5-oxo-4,5-dihydro-1H-pyrazol-1-yl, (3,4-dimethyl-5-oxo-4,5-dihydro-1H-pyrazol-1-yl, pyrrolidin-1-yl, piperidin-1-yl, 3-Oxo-4,5-dimethyl-2,4-dihy-dropyrazol-2-yl-, 3-Oxo-4-ethyl-5-methyl-2,4-dihydro-pyrazol-2-yl-, 3-Oxo-5-trifluor-methyl-2,4-dihydro-pyrazol-2-yl-, 3-Oxo-5-isopropyl-2,4-dihydro-pyrazol-2-yl-, 3,5-Dioxo-4,4-dimethyl-pyrazolidin-1-yl, 3,5-Dioxo-4-ethyl-pyrazolidin-1-yl, 3-Oxo-4,4-dimethyl-pyrazolidin-1-yl, 2,5-dioxo-2,5-dihydro-1H-pyrrol-1-yl, 3,5-Dimethylpiperidin-1-yl, 4-(tert-butoxycarbo-nyl)piperazin-1-yl, Acetyl(cyclohexyl)amino, 2-Furoylamino, 3-(2-Chlorethyl)-2-oxoimidazolidin-1-yl, 1,1-dioxidoisothiazolidin-2-yl, 1,1-dioxidotetrahydrothiophen-2-yl, 5-methyl-1,1-dioxido-1,2,5-thiadiazolidin-2-yl, 4-methoxy-2-oxo-2,5-dihydro-1H-pyrrol-1-yl, 5-oxo-4,5-dihydro-1H-imidazol-1-yl, 3-methyl-5-oxo-2,5-dihydro-1H-pyrazol-1-yl, 2-(methoxymethyl)pyrrolidin-1-yl, 2-oxotetrahydrofuran-3-yl, 1-me-thyl-3-oxo-2,3-dihydro-1H-pyrazol-4-yl, tetrahydro-fura-n-2-yl, furan-2-yl., 1,1-dioxido-1,2-thiazinan-2-yl, 5-methyl-1,1-dioxido-1,2,5-thiadiazolidin-2-yl, 6-methyl-1,1-dioxido-1,2,6-thiadiazinan-2-yl.
und für den Fall, dass R³ und R⁴, ggf. über R¹² oder R¹³ einen Zyklus bilden, kann folgende Untereinheit aus der allgemeinen Formel (I): 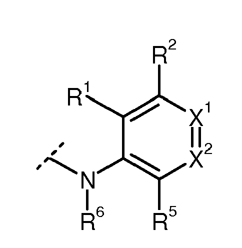 (2-oxo-2,3-dihydro-1H-indol-5-yl)amino, 1H-indol-6-ylamino, 1H-indol-5-ylamino, (4-methyl-3-oxo-3,4-dihydro-2H-1,4-benzoxazin-6-yl)amino, (4-methyl-3-oxo-3,4-dihydro-2H-1,4-benzoxazin-7-yl)amino, (1-acetyl-2,3-dihydro-1H-indol-6-yl)amino, (2-oxo-2,3,4,5-tetrahydro-1H-1-benzaze-pin-8-yl)amino, 1,3-benzodioxol-5-ylamino, (1,3-dioxo-2,3-dihydro-1H-isoindol-5-yl)amino, (2-oxo-1,3-benzoxathiol-5-yl)amino, 2-oxo-2,3-dihydro-1,3-benzoxazol-5-yl)amino, (2-ethyl-1,3-benz oxazol-5-yl)amino, 2-oxo-1,2,3,4-tetrahydroquinolin-6-yl)amino, (3-oxo-3,4-dihydro-2H-1,4-benz-oxazin-6-yl)amino, (2-oxo-2,3-dihydro-1,3-benzoxazol-6-yl)amino, [2-(ethylsulfonyl)-1,3-benzo-thiazol-6-yl]amino, (2-methyl-1,3-benzothiazol-5-yl)amino, (1-acetyl-2,3-dihydro-1H-indol-5-yl)amino, (2,2,3,3-tetrafluoro-2,3-dihydro-1,4-benzodioxin-6-yl)amino, (2,2-dioxido-1,3-dihydro-2-benzothiophen-5-yl)amino sein,
R⁶ ist Wasserstoff, Methyl, Formyl oder COMe,
R⁷ ist Wasserstoff,
R⁸ ist Fluor, Chlor, Brom, Iod, Cyano, Methyl, SMe, SOMe, SO₂Me oder CF₃,
R⁹ ist Wasserstoff" Methyl, Ethyl, Propanyl, CH₂CH=CH₂ oder CH₂C≡CH,
R¹⁰ ist 2,2-Difluorethyl, 2,2-Dichlorethyl, 1,1,1-Trifluorpropan-2-yl, 2,2,2-Trifluorethyl, 2,2,2-Trichlorethyl, 4,4,4-Trifluorbutan-2-yl, 2-Fluorethyl, 2-Chlorethyl, 2,2,3,3-Tetrafluorpropan-1-yl, 3,3,3-Trifluorpropan-1yl, 2,2-Difluorpropan-1-yl, 1,1,1-Trifluor-4-methyl-pentan-2-yl, 1,1,1-Trifluorpentan-2-yl, 1,1,1-Trifluorbutan-2-yl, 1-Fluorpropan-2-yl, 2-Fluorpropan-1-yl, 2,3,3,3-Tetrafluorpropan-1-yl, 2,2,3,3,4,4,5,5,5-Nonafluorpentan-1-yl, 2,2,3,3,4,4,5,5-Octafluorpentan-1-yl, 3-Fluorpropan-1-yl, 3,3-Difluorpropan-1-yl, 3,3,3-Trifluorpropan-1-yl, 3-Chlorpropan-1-yl, 3,3-Dichlorpropan-1-yl, 3,3,3-Trichlorpropan-1-yl, 2-Brom-2,2-difluorethyl, 2-Chlor-2,2-difluorethyl, 3-Brom-3,3-difluorpropan-1 yl, 2,2,3,3,3-Pentafluorpropan-1-yl, 2,2,3,3,4,4,4-Heptafluorbutan-1-yl, 3,3,4,4,4-Pentafluorbutan-1-yl, 3,3,4,4,5,5-Heptafluorpentan-1-yl, 4,4,4-Trifluorbutan-1-yl, 4,4-Difluorbutan-1-yl, 1,3-Difluorpropan-2-yl, 1,1-Difluorpropan-2-yl, 1,1,1-Trifluor-3-hydroxypropan-2-yl, 1,1,1 -Trifluor-3 -ethoxypropan-2-yl, 1,1,1-Trifluor-4-hydroxybutan-2-yl, 1,1,1-Trifluorpent-4-en-2-yl, 3-Chlor-2-methylpropyl, 2-Hydroxy-3,3,3-trifluorpropan-1-yl oder 2,5-Dichlorpentan-1-yl,
sowie die agrochemisch wirksamen Salze davon.

**5.** Mittel zur Bekämpfung von pflanzenpathogenen Schadpilzen, **gekennzeichnet durch** einen Gehalt an mindestens einem Diaminopyrimidin der Formel (**I**) gemäß einem oder mehreren der Ansprüche 1 bis 4 neben Streckmitteln und/oder oberflächenaktiven Stoffen.

**6.** Verwendung von Diaminopyrimidine der Formel (**I**) gemäß einem oder mehreren der Ansprüche 1 bis 4 zur Bekämpfung von pflanzenpathogenen Schadpilzen.

**7.** Verfahren zur Bekämpfung von pflanzenpathogenen Schadpilzen, **dadurch gekennzeichnet, dass** man Diaminopyrimidine der Formel (**I**) gemäß einem oder mehreren der Ansprüche 1 bis 4 auf pflanzenpathogenen Schadpilze und/oder deren Lebensraum ausbringt.

**8.** Verfahren zur Herstellung von Mitteln zur Bekämpfung von pflanzenpathogenen Schadpilzen, **dadurch gekennzeichnet, dass** man Diaminopyrimidine der Formel (**I**) gemäß einem oder mehreren der Ansprüche 1 bis 4 mit Streckmitteln und/oder oberflächenaktiven Stoffen vermischt.

**9.** Verfahren zum Verfahren zum Herstellen der erfindungsgemäßen Diaminopyrimidine der Formel (**I**) umfassend wenigstens einen der folgenden Schritte (a) bis (e):
(a) Umsetzung von 2,4-Dihalopyrimidinen der Formel (**III**) mit Halogen-substituierten Aminen der Formel (**II**) zu Verbindungen der Formel (**V**) in Gegenwart einer Base gegebenenfalls in Gegenwart eines Lösungsmittels, gegebenenfalls in Gegenwart eines Katalysators gemäß dem nachfolgenden Reaktionsschema: 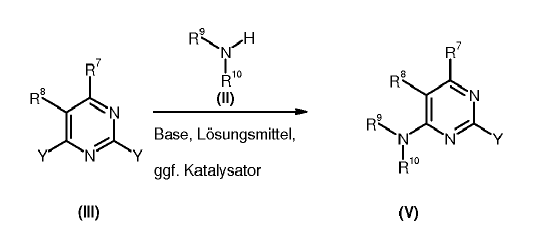 Mit Y = F, Cl, Br, I
(b) Umsetzung von Verbindungen der Formel (**V**) mit (het-)aromatischen Aminen der Formel (**IV**) gegebenenfalls in Gegenwart einer Säure, gegebenenfalls in Gegenwart eines Lösungsmittels gemäß dem nachfolgenden Reaktionsschema: 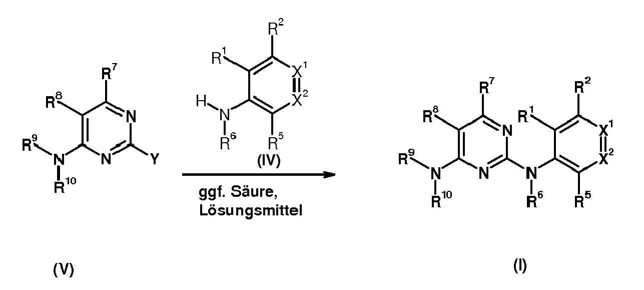 Mit Y = F, Cl, Br, I
(c) Umsetzung von Verbindungen der Formel (**VIb**) mit einem (het-)aromatischen Amin der Formel (**IV**), gegebenenfalls in Gegenwart einer Säure und in Gegenwart eines Lösungsmittels gemäß dem nachfolgenden Reaktionsschema: 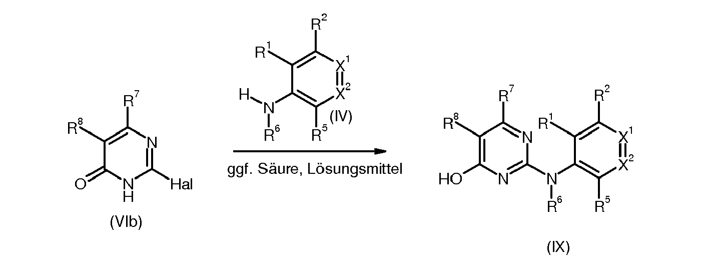 Mit Hal = F, Cl, Br, I
(d) Umsetzung von Verbindungen der Formel (**IX**) mit einem Halogenierungsmittel zu Verbindungen der Formel (**X**), gegebenenfalls in Gegenwart eines Lösungsmittels gemäß dem nachfolgenden Reaktionsschema: 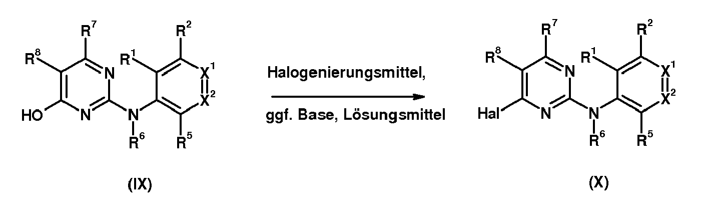
(e) Umsetzung von Verbindungen der Formel (**X**) mit Halogen-substituierten Aminen der Formel (**II**) zu Verbindungen der Formel (**I**) in Gegenwart einer Base gegebenenfalls in Gegenwart eines Lösungsmittels, gegebenenfalls in Gegenwart eines Katalysators gemäß dem nachfolgenden Reaktionsschema: 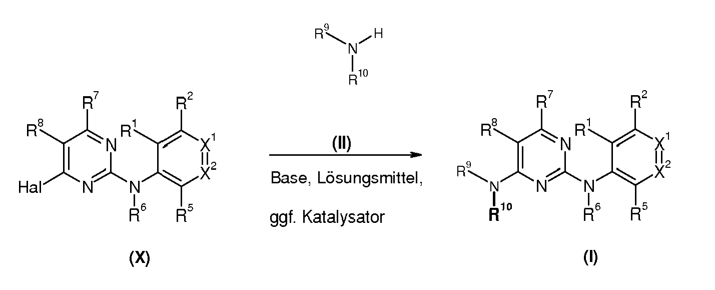
wobei die Definitionen der Reste R¹ bis R¹⁰ und X¹ und X² in den obigen Schemata die Bedeutungen anch Anspruch 1 haben, und Y und Hal für F, Cl, Br, I steht.

**9.** Verbindungen der Formel (**IXa**), 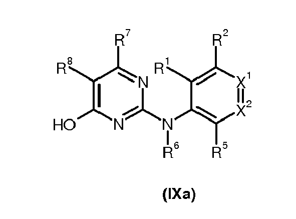 in welcher die Symbole folgende Bedeutungen haben:
X¹, X², R¹ bis R⁶, R¹², R¹³ und R¹⁴ haben die in Anspruch 1 angegebenen Bedeutungen,
R⁷ ist Wasserstoff,
R⁸ steht für Fluor, Chlor, Brom, Iod, CFH₂, CF₂H, CF₃, CCl₃, SMe, SOMe oder SO₂Me,
mit der Massgabe, dass wenn
R¹ = R² = R⁵ = Wasserstoff sind und X² = CH ist,
R³ nicht gleich Wasserstoff, CO₂H, (CH₂)₂OH, SMe, SOMe, oder Cyano sein darf
oder
mit der Massgabe, dass wenn
R¹ = R⁵ = Wasserstoff sind und X¹ = CH ist,
weder R² noch R⁴ = OH oder CONH₂ sein darf.

**10.** Verbindungen der Formel (**Xa**), 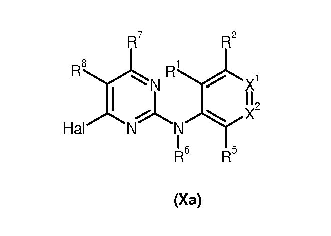 in welcher die Symbole folgende Bedeutungen haben:
X¹, X², R² bis R⁴, R¹² R¹³ und R¹⁴ entsprechen den Defintionen nach Anspruch 1
und
Hal steht für Fluor, Chlor, Brom oder Iod,
R⁸ steht für Fluor, Chlor, Brom, Iod, Me, CF₃, CFH₂, CF₂H, CCl₃ und Cyano,
R¹, R⁵, R⁶ und R⁷ stehen für Wasserstoff
mit der Massgabe, dass wenn
X² = CH und X¹ = CR³ ist
R³ nicht gleich CON(Me)-4-(N-Methyl-piperidinyl), N-Piperazinyl, CO-1-(4-Methyl-pipe-razinyl), N-Morpholinyl, SO₂Me, CONH₂, Me, OMe, COO-Benzyl, COOH, COCl, CN, NO₂, NMe₂ oder Cl ist,
oder
mit der Massgabe, dass wenn
X¹ = CH und X² = CR⁴ ist
R² oder R⁴ nicht für CN, Cl oder 5-Oxazolyl steht,
oder
mit der Massgabe, dass wenn
X¹ = CR³ und X² = CR⁴ ist
R² und R³ bzw. R³ und R⁴ nicht Chlor sind,
oder
mit der Massgabe, dass wenn
R⁸ = F oder CF₃ ist
R³ und R⁴ nicht gemeinsam einen gesättigten oder teilweise ungesättigten Heterocyclus
bilden.

**11.** Verbindungen der Formel (Va) 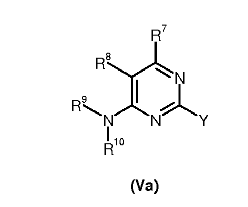 in welcher die Symbole folgende Bedeutungen haben:
R⁷ steht für Wasserstoff, C₂-C₃-Alkyl, Cyano oder C₁-C₃-Haloalkyl,
R⁸ steht für Chlor, Brom, Iod, Cyano, SMe, SOMe, SO₂Me, CF₃, CCl₃, CFH₂ oder CF₂H,
Y ist F, Cl, Br oder I
und
R⁹, R¹⁰, definiert nach Anspruch 1,
mit der Massgabe, dass wenn
R⁸ = Brom ist,
R¹⁰ nicht 2,2,3,3,3-Pentafluorpropan-1-yl oder 2,2,3,3,4,4,4-Heptafluorbutan-1-yl sein darf
oder
mit der Massgabe, dass wenn
R⁸ = CN ist,
R¹⁰ nicht 2,2,2 Trifluorethyl oder 2-Fluorethyl sein darf.
